# EUROPEAN PATENT SPECIFICATION

(11) **EP 4 265 275 B1**
(45) Date of publication and mention of the grant of the patent: **01.07.2026**
(21) Application number: 20965609.9
(22) Date of filing: 18.12.2020
(51) Int. Cl.: A61K 47/68, C07K 16/30, A61P 35/00, A61K 39/00

(54) **TROP2 TARGETING ANTIBODY-DRUG CONJUGATE, AND PREPARATION METHOD AND USE THEREFOR**
GEGEN TROP2 GERICHTETES ANTIKÖRPER-WIRKSTOFF-KONJUGAT SOWIE HERSTELLUNGSVERFAHREN UND VERWENDUNG DAVON
CONJUGUÉ ANTICORPS-MÉDICAMENT CIBLANT TROP2, PROCÉDÉ DE PRÉPARATION ET UTILISATION S'Y RAPPORTANT

(43) Date of publication of application: 25.10.2023
(73) Proprietor: Shanghai Fudan-Zhangjiang Bio-Pharmaceutical Co., Ltd., Shanghai 201210 (CN)
(72) Inventor: GUO, Qingsong, Shanghai 201210 (CN); SHEN, Yijun, Shanghai 201210 (CN); YANG, Tong, Shanghai 201210 (CN); BAO, Bin, Shanghai 201210 (CN); GAO, Bei, Shanghai 201210 (CN); WU, Fang, Shanghai 201210 (CN); XU, Jun, Shanghai 201210 (CN)
(74) Representative: Dehns
(86) International application number: PCT/CN2020/137596
(87) International publication number: WO 2022/126593

(56) References cited:
- EP-A1- 3 991 754
- WO-A1-2014/092804
- WO-A1-2020/196712
- WO-A1-2020/240467
- WO-A1-2021/225892
- WO-A2-2016/070089
- CN-A- 104 755 494
- CN-A- 105 849 126
- CN-A- 109 078 181
- POUDEL YAM B. ET AL: "Chemical Modification of Linkers Provides Stable Linker-Payloads for the Generation of Antibody-Drug Conjugates", ACS MEDICINAL CHEMISTRY LETTERS, vol. 11, no. 11, 12 November 2020 (2020-11-12), US, pages 2190 - 2194, XP093125915, ISSN: 1948-5875, DOI: 10.1021/acsmedchemlett.0c00325

## Description

### TECHNICAL FIELD

The present disclosure relates to the field of biomedicine, and in particularly relates to a TROP2-targeting antibody-drug conjugate, a preparation method therefor, a pharmaceutical composition thereof, and a use thereof.

### BACKGROUND

Tumor-associated calcium signal transducer 2 (Trop2, TROP2) is a type I transmembrane glycoprotein encoded by the *TACSTD2* gene, is a calcium signal transducer related to the regulation of intracellular calcium signals. The expression of Trop2 can activate ERK. Under normal circumstances, Trop2 is mainly expressed in trophoblast cells, and plays an important role in the development of embryonic organs. Trop2 is overexpressed in many human epithelial tumor tissues, including breast cancer, lung cancer, gastric cancer, colorectal cancer, pancreatic cancer, prostate cancer, cervical cancer, ovarian cancer, etc., and the expression level is related to tumor deterioration and prognosis. Genome analysis of breast cancer patients and current clinical research results show that Trop2 is a clinically confirmed therapeutic target. Trop2 has attracted widespread attention due to its high expression on the surface of various tumor cells. The level of Trop2 on the surface of a single cell is tens of thousands to hundreds of thousands. Epidemiology has proved that Trop2 is related to the development and prognosis of various tumors, and Trop2 has become a target for antibody drug development. After the Trop2 antibody binds to the Trop2 protein, its endocytosis efficiency is very high, which is very suitable for the development of antibody-drug conjugates.

Antibody-drug conjugate (ADC) is a new generation of targeted therapeutic drugs, mainly used for the treatment of cancer tumors. ADC drugs consist of three parts: a small molecule cytotoxic drug (Drug), an antibody (Antibody), and a linker (Linker) that links the antibody with the cytotoxic drug. The small molecule cytotoxic drug is bound to the antibody protein by chemical coupling. ADC drugs utilize antibodies to specifically recognize and guide small molecule drugs to cancer cell targets expressing cancer-specific antigens, and enter the cancer cells through endocytosis. The linker part is broken under the action of intracellular low pH value environment or lysosomal protease, releasing small molecular cytotoxic drugs, so as to achieve the effect of specifically killing cancer cells without damaging normal tissue cells. Therefore, ADC drugs have the characteristics of the targeted specificity of antibodies and the high toxicity of small molecule toxins to cancer cells at the same time, greatly expanding the effective therapeutic window of the drug. Clinical studies have proven that ADC drugs have high efficacy and are relatively stable in the blood, and can effectively reduce the toxicity of small molecule cytotoxic drugs (chemotherapy drugs) to the circulatory system and healthy tissues, and are currently a hot spot in the development of anti-cancer drugs internationally.

The following documents report on the Trop2 antibody and the payload for the antibody-drug conjugates: CN 105849126A and WO 2020/240467A1.

### CONTENT OF THE PRESENT INVENTION

The present invention is set out in the appended set of claims. Specifically, the present invention provides an antibody-drug conjugate, a pharmaceutically acceptable salt thereof, a solvate thereof, or a solvate of the pharmaceutically acceptable salt thereof as defined in claim 1, a method for preparing the antibody-drug conjugate as defined in claim 8, a pharmaceutical composition comprising the antibody-drug conjugate as defined in claim 9, the antibody-drug conjugate for use in inhibiting TROP2 protein as defined in claim 10, and the antibody-drug conjugate for use in treating and/or preventing a tumor as defined in claim 11.

The technical problem to be solved by the present disclosure is to overcome the current deficiency of limited types of antibody-drug conjugates, and the present disclosure provides a TROP2-targeting antibody-drug conjugate, a preparation method therefor, a pharmaceutical composition thereof, and a use thereof.

The present disclosure has developed an antibody-drug conjugate with good targeting ability, strong inhibition effect on tumor cells that are positive for TROP2 expression, and good druggability and high safety. The antibody-drug conjugate has an inhibitory effect on TROP2, and also has a good inhibitory effect on at least one of NCI-N87, MDA-MB-468, and BXPC3 cells.

The present disclosure solves the above technical problem through the following technical solution.

The present disclosure provides an antibody-drug conjugate of formula I, a pharmaceutically acceptable salt thereof, a solvate thereof, or a solvate of the pharmaceutically acceptable salt thereof; 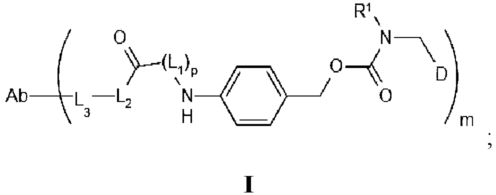
wherein Ab is a TROP2 antibody or a variant of the TROP2 antibody;
the amino acid sequence of the light chain in the TROP2 antibody is shown in SEQ ID NO: 1, and the amino acid sequence of the heavy chain is shown in SEQ ID NO: 2;
the variant of the TROP2 antibody is at least 70%, 75%, 80%, 85%, 90%, 95%, 98%, or 99% identical to the TROP2 antibody;
m is 3.5 to 4.5;
D is a cytotoxic drug topoisomerase inhibitor, wherein the cytotoxic drug topoisomerase inhibitor is 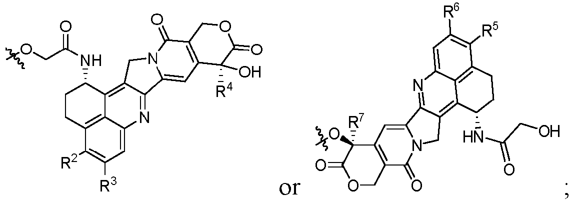
R² and R⁵ are each independently H, C₁-C₆ alkyl, or halogen; R³ and R⁶ are each independently H, C₁-C₆ alkyl, or halogen; R⁴ and R⁷ are each independently C₁-C₆ alkyl;
R¹ is C₁-C₆ alkyl substituted by one or more than one -NR¹⁻¹R¹⁻², C₁-C₆ alkyl substituted by one or more than one R¹⁻³S(O)₂-, C₁-C₆ alkyl, C₃-C₁₀ cycloalkyl, C₆-C₁₄ aryl, or 5- to 14-membered heteroaryl; the heteroatom in the 5- to 14-membered heteroaryl is selected from one or more than one of N, O, and S, and the number of heteroatoms is 1, 2, 3, or 4; the R¹⁻¹, R¹⁻², and R¹⁻³ are each independently C₁-C₆ alkyl;
L₁ is independently one or more than one of a phenylalanine residue, alanine residue, glycine residue, glutamic acid residue, aspartic acid residue, cysteine residue, histidine residue, isoleucine residue, leucine residue, lysine residue, methionine residue, proline residue, serine residue, threonine residue, tryptophan residue, tyrosine residue, and valine residue; p is 2 to 4;
L₂ is 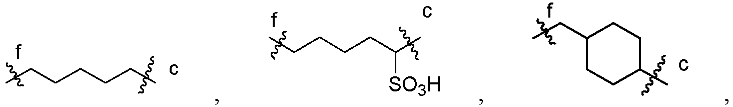 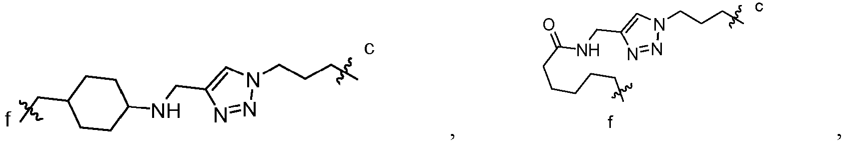 or 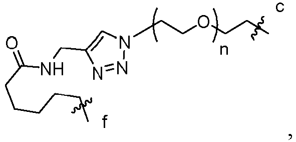 wherein n is independently 1 to 12, the c-terminal is connected to L₁ through a carbonyl group, and the f-terminal is connected to the d-terminal of L₃;
L₃ is 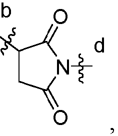 wherein the b-terminal is connected to the Ab, and the d-terminal is connected to the f-terminal of L₂.

In a preferred embodiment of the present disclosure, certain groups of the antibody-drug conjugate are defined as follows, and the definition of any unmentioned groups is as described in any of the above embodiments (this paragraph is hereinafter referred to as "in a preferred embodiment of the present disclosure"):
the b-terminal of L₃ is preferably connected to the sulfhydryl group on the antibody in the form of a thioether. Taking 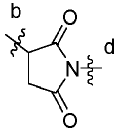 as an example, the connecting form of 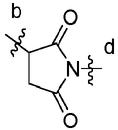 with the cysteine residue in the antibody is

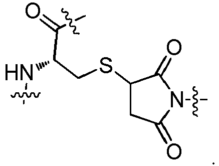

In a preferred embodiment of the present disclosure, when R¹ is C₁-C₆ alkyl substituted by one or more than one -NR¹⁻¹R¹⁻², the C₁-C₆ alkyl is methyl, ethyl, n-propyl, isopropyl, n-butyl, isobutyl, or tert-butyl, preferably ethyl.

In a preferred embodiment of the present disclosure, when R¹ is C₁-C₆ alkyl substituted by more than one -NR¹⁻¹R¹⁻², the "more than one" is two or three.

In a preferred embodiment of the present disclosure, when R¹⁻¹ and R¹⁻² are each independently C₁-C₆ alkyl, the C₁-C₆ alkyl is methyl, ethyl, n-propyl, isopropyl, n-butyl, isobutyl, or tert-butyl, preferably methyl.

In a preferred embodiment of the present disclosure, when R¹ is C₁-C₆ alkyl substituted by one or more than one R¹⁻³S(O)₂-, the C₁-C₆ alkyl is C₁-C₄ alkyl, preferably methyl, ethyl, n-propyl, isopropyl, n-butyl, isobutyl, or tert-butyl, most preferably ethyl.

In a preferred embodiment of the present disclosure, when R¹ is C₁-C₆ alkyl substituted by more than one R¹⁻³S(O)₂-, the "more than one" is two or three.

In a preferred embodiment of the present disclosure, when R¹⁻³ is C₁-C₆ alkyl, the C₁-C₆ alkyl is methyl, ethyl, n-propyl, isopropyl, n-butyl, isobutyl, or tert-butyl, preferably methyl.

In a preferred embodiment of the present disclosure, when R¹ is C₁-C₆ alkyl, the C₁-C₆ alkyl is methyl, ethyl, n-propyl, isopropyl, n-butyl, isobutyl, or tert-butyl, preferably methyl.

In a preferred embodiment of the present disclosure, n is 8 to 12.

In a preferred embodiment of the present disclosure, p is 2.

In a preferred embodiment of the present disclosure, when R¹⁻¹, R¹⁻², and R¹⁻³ are independently C₁-C₆ alkyl, the C₁-C₆ alkyl is C₁-C₄ alkyl, preferably methyl, ethyl, n-propyl, isopropyl, n-butyl, isobutyl, or tert-butyl, most preferably methyl.

In a preferred embodiment of the present disclosure, when R² and R⁵ are each independently C₁-C₆ alkyl, the C₁-C₆ alkyl is preferably C₁-C₄ alkyl, further preferably methyl, ethyl, n-propyl, isopropyl, n-butyl, isobutyl, or tert-butyl, most preferably methyl.

In a preferred embodiment of the present disclosure, when R² and R⁵ are each independently halogen, the halogen is preferably fluorine, chlorine, bromine, or iodine, further preferably fluorine.

In a preferred embodiment of the present disclosure, when R³ and R⁶ are each independently C₁-C₆ alkyl, the C₁-C₆ alkyl is preferably C₁-C₄ alkyl, further preferably methyl, ethyl, n-propyl, isopropyl, n-butyl, isobutyl, or tert-butyl, most preferably methyl.

In a preferred embodiment of the present disclosure, when R³ and R⁶ are each independently halogen, the halogen is preferably fluorine, chlorine, bromine, or iodine, further preferably fluorine.

In a preferred embodiment of the present disclosure, when R⁴ and R⁷ are each independently C₁-C₆ alkyl, the C₁-C₆ alkyl is preferably C₁-C₄ alkyl, further preferably methyl, ethyl, n-propyl, isopropyl, n-butyl, isobutyl, or tert-butyl, most preferably ethyl.

In a preferred embodiment of the present disclosure, when R¹ is C₁-C₆ alkyl substituted by one or more than one -NR¹⁻¹R¹⁻², the -NR¹⁻¹R¹⁻² is preferably -N(CH₃)₂.

In a preferred embodiment of the present disclosure, when R¹ is C₁-C₆ alkyl substituted by one -NR¹⁻¹R¹⁻², the C₁-C₆ alkyl substituted by one -NR¹⁻¹R¹⁻² is

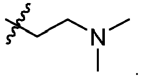

In a preferred embodiment of the present disclosure, when R¹ is C₁-C₆ alkyl substituted by one R¹⁻³S(O)₂-, the C₁-C₆ alkyl substituted by one R¹⁻³S(O)₂- is

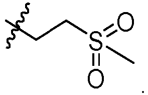

In a preferred embodiment of the present disclosure, m is 3.8 to 4.2, such as 3.88, 3.90, 3.96, 3.97, 3.98, 4.00, 4.03, 4.05, 4.10, 4.12, or 4.13.

In a preferred embodiment of the present disclosure, (L₁)ₚ is further preferably 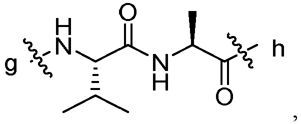 wherein the g-terminal is connected to the c-terminal of L₂ through a carbonyl group.

In a preferred embodiment of the present disclosure, n is 8 to 12, such as 8, 9, 10, 11, or 12, further such as 8 or 12.

In a preferred embodiment of the present disclosure, R² and R⁵ are each independently C₁-C₆ alkyl.

In a preferred embodiment of the present disclosure, R³ and R⁶ are each independently halogen.

In a preferred embodiment of the present disclosure, R⁴ and R⁷ are ethyl.

In a preferred embodiment of the present disclosure, when D is 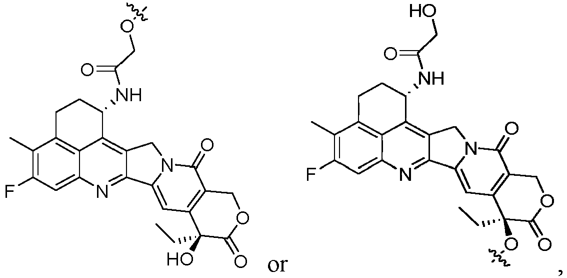 the antibody-drug conjugate can be 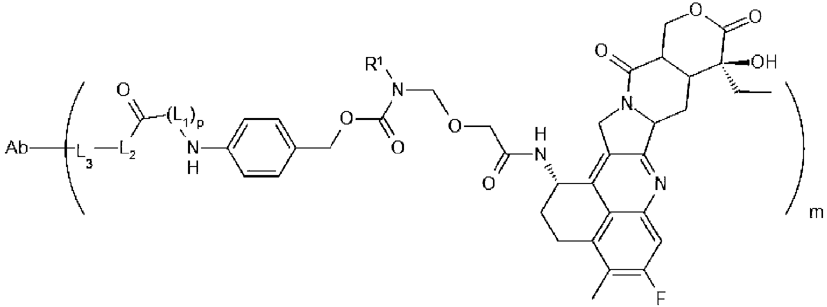 or

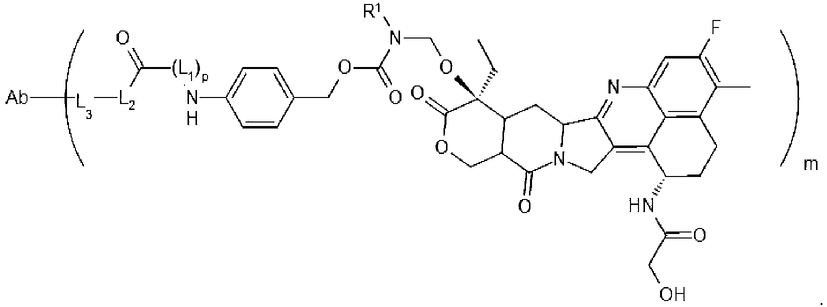

In a preferred embodiment of the present disclosure, L₁ is preferably one or more than one of the phenylalanine residue, alanine residue, glycine residue, isoleucine residue, leucine residue, proline residue, and valine residue; more preferably one or more than one of the phenylalanine residue, alanine residue, glycine residue, and valine residue; further preferably the valine residue and/or the alanine residue; the "more than one" is preferably two or three; p is preferably 2.

In a preferred embodiment of the present disclosure, R¹ is preferably C₁-C₆ alkyl substituted by one or more than one -NR¹⁻¹R¹⁻², C₁-C₆ alkyl substituted by one or more than one R¹⁻³S(O)₂-, C₁-C₆ alkyl, or C₃-C₁₀ cycloalkyl; more preferably C₁-C₆ alkyl substituted by one or more than one -NR¹⁻¹R¹⁻², C₁-C₆ alkyl substituted by one or more than one R¹⁻³S(O)₂-, or C₁-C₆ alkyl; further preferably C₁-C₆ alkyl substituted by one or more than one -NR¹⁻¹R¹⁻², or C₁-C₆ alkyl substituted by one or more than one R¹⁻³S(O)²-; most preferably C₁-C₆ alkyl substituted by one or more than one R¹⁻³S(O)²-.

Preferably, D is

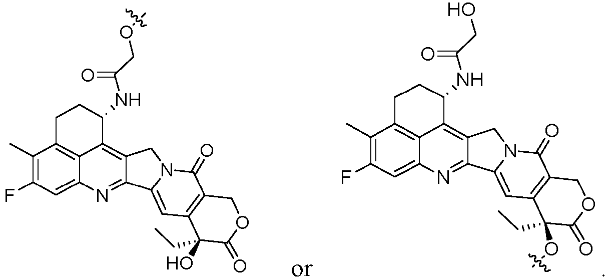

In the present disclosure, when Ab is the TROP2 antibody or the variant of the TROP2 antibody, the TROP2 antibody or the variant of the TROP2 antibody is a residue of the TROP2 antibody (a group formed by replacing a hydrogen on one of the sulfhydryl groups in TROP2 antibody) or a residue of a variant of the TROP2 antibody (a group formed by replacing a hydrogen on one of the sulfhydryl groups in the variant of the TROP2 antibody).

In a preferred embodiment of the present disclosure, the compound of formula I is any one of the following schemes:
scheme I:
   Ab is the TROP2 antibody or the variant of the TROP2 antibody;
   D is 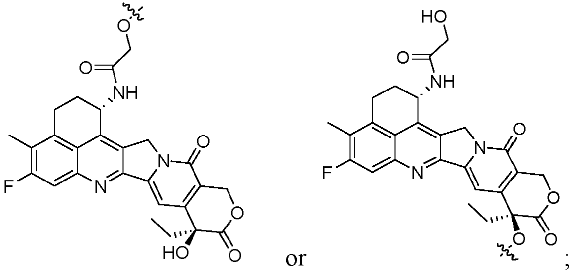
   L₁ is independently one or more than one of the phenylalanine residue, alanine residue, glycine residue, isoleucine residue, leucine residue, proline residue, and valine residue.
scheme II:
   Ab is the TROP2 antibody or the variant of the TROP2 antibody;
   D is 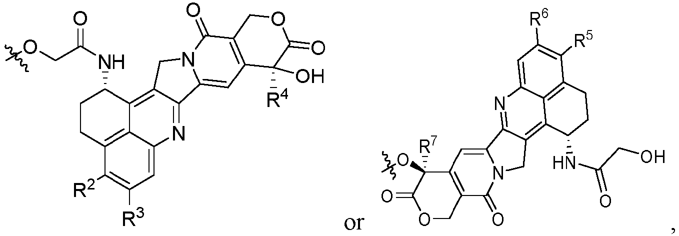 R² and R⁵ are each independently C₁-C₆ alkyl; R³ and R⁶ are each independently halogen, D is preferably 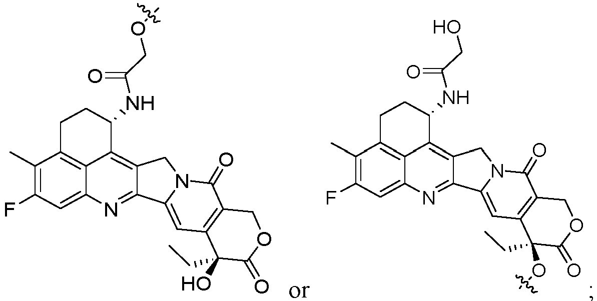
   R¹ is C₁-C₆ alkyl substituted by one or more than one -NR¹⁻¹R¹⁻², C₁-C₆ alkyl substituted by one or more than one R¹⁻³S(O)₂-, or C₁-C₆ alkyl;
   L₁ is independently one or more than one of the phenylalanine residue, alanine residue, glycine residue, and valine residue.
scheme III:
   Ab is the TROP2 antibody;
   D is 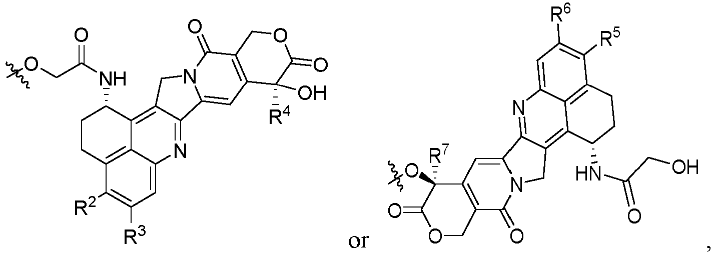 R² and R⁵ are each independently C₁-C₆ alkyl; R³ and R⁶ are each independently halogen, D is preferably 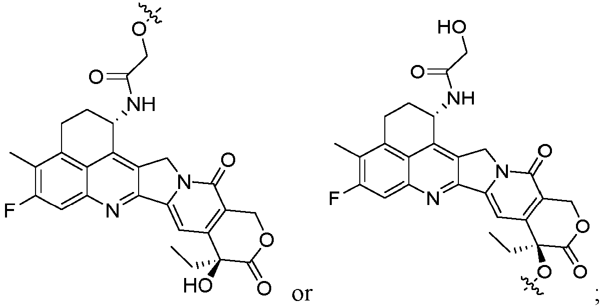
   R¹ is C₁-C₆ alkyl substituted by one or more than one -NR¹⁻¹R¹⁻², C₁-C₆ alkyl substituted by one or more than one R¹⁻³S(O)₂-, or C₁-C₆ alkyl;
   L₁ is independently the valine residue and/or the alanine residue.
scheme IV:
   Ab is the TROP2 antibody;
   D is 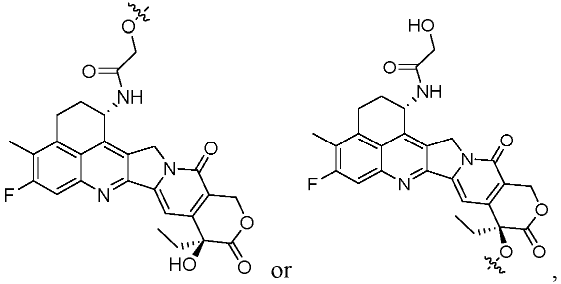
   R¹ is C₁-C₆ alkyl substituted by one or more than one -NR¹⁻¹R¹⁻², C₁-C₆ alkyl substituted by one or more than one R¹⁻³S(O)₂-, or C₁-C₆ alkyl;
   L₁ is independently the valine residue and/or the alanine residue.
scheme V:
   Ab is the TROP2 antibody;
   D is 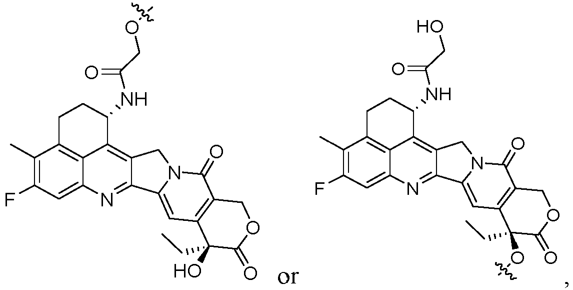
   R¹ is C₁-C₆ alkyl substituted by one or more than one -NR¹⁻¹R¹⁻², C₁-C₆ alkyl substituted by one or more than one R¹⁻³S(O)₂-, or C₁-C₆ alkyl;
   L₁ is independently the valine residue and/or the alanine residue.

In a preferred embodiment of the present disclosure, the antibody-drug conjugate is preferably 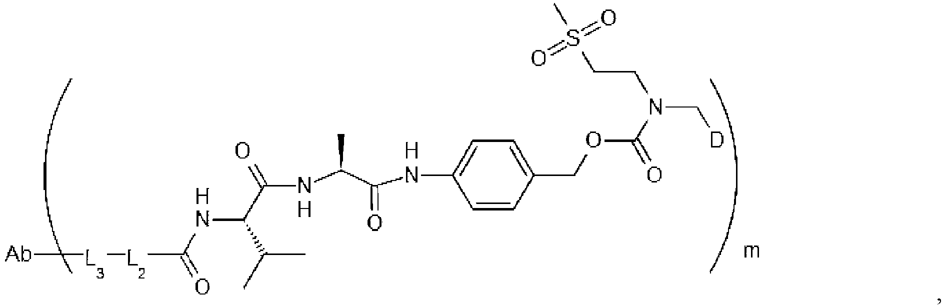 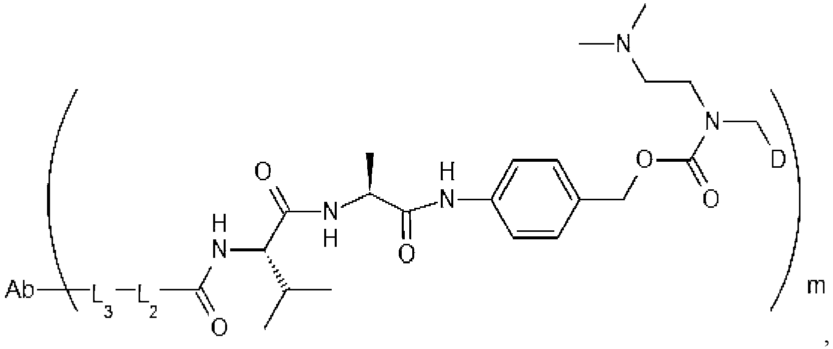 or

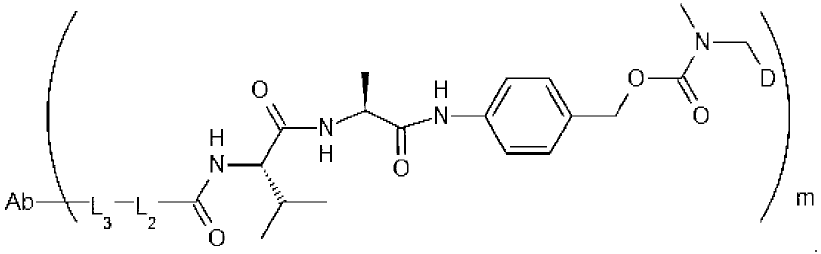

In a preferred embodiment of the present disclosure, L₂ is preferably

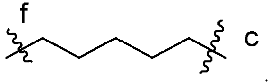

In a preferred embodiment of the present disclosure, Ab is the TROP2 antibody or the variant of the TROP2 antibody, wherein the amino acid sequence of the light chain in the TROP2 antibody is shown in SEQ ID NO: 1; the amino acid sequence of the heavy chain in the TROP2 antibody is shown in SEQ ID NO: 2; D is 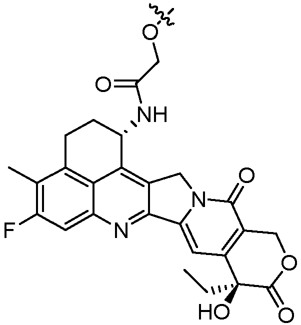 or 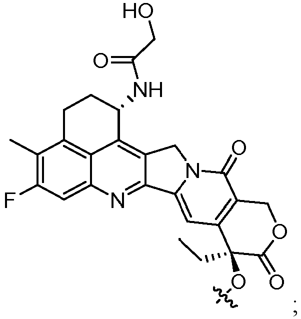 L₁ is the valine residue and/or the alanine residue, p is 2, (L₁)p is preferably 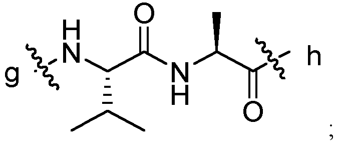 R¹ is C₁-C₆ alkyl substituted by one or more than one -NR¹⁻¹R¹⁻², C₁-C₆ alkyl substituted by one or more than one R¹⁻³S(O)₂-, or C₁-C₆ alkyl, preferably C₁-C₆ alkyl substituted by one or more than one -NR¹⁻¹R¹⁻² or C₁-C₆ alkyl substituted by one or more than one R¹⁻³S(O)₂-, further preferably C₁-C₆ alkyl substituted by one or more than one R¹⁻³S(O)₂-; R¹⁻¹, R¹⁻², and R¹⁻³ are independently C₁-C₄ alkyl, preferably methyl; the C₁-C₆ alkyl substituted by one or more than one -NR¹⁻¹R¹⁻² is preferably 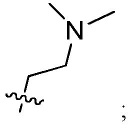 the C₁-C₆ alkyl substituted by one or more than one R¹⁻³S(O)₂- is preferably 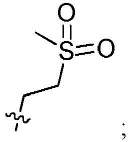 L₂ is 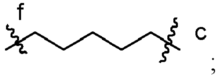 L₃ is

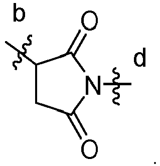

In a preferred embodiment of the present disclosure, the antibody-drug conjugate is preferably any one of the following compounds: 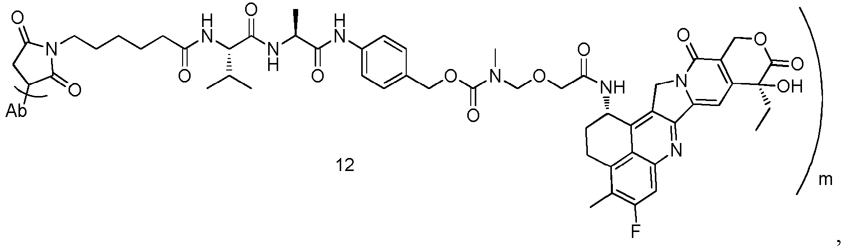 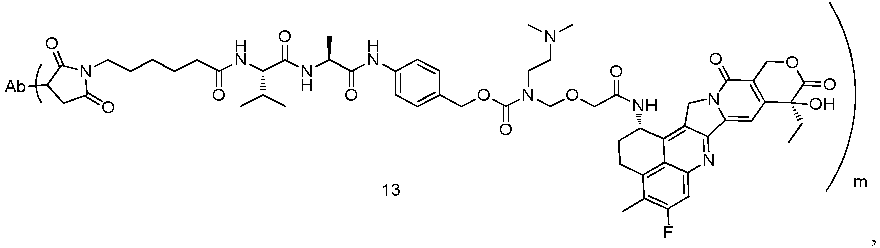 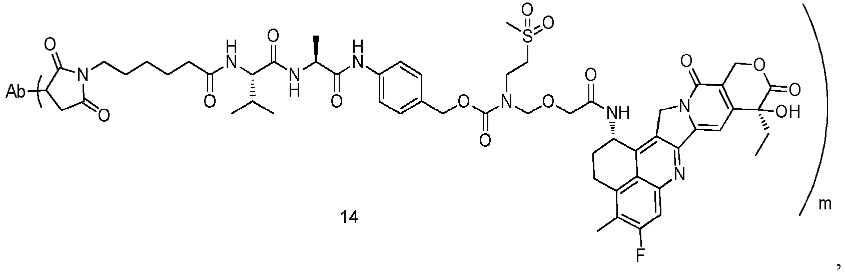 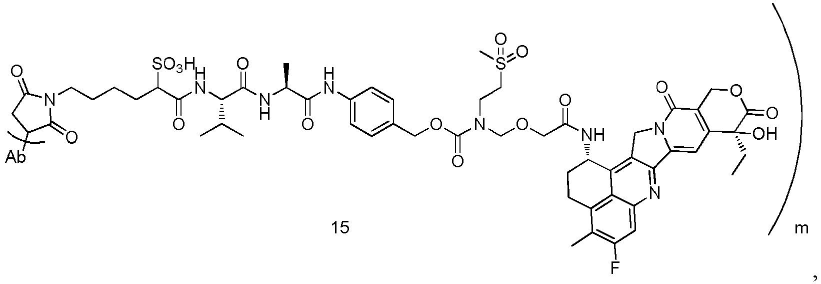 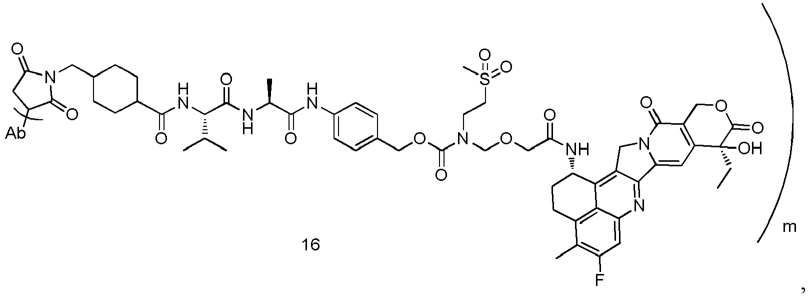 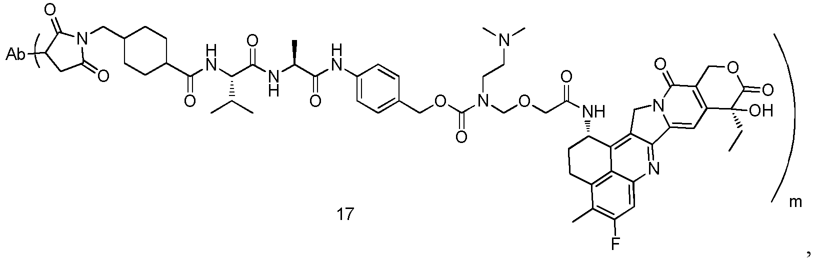 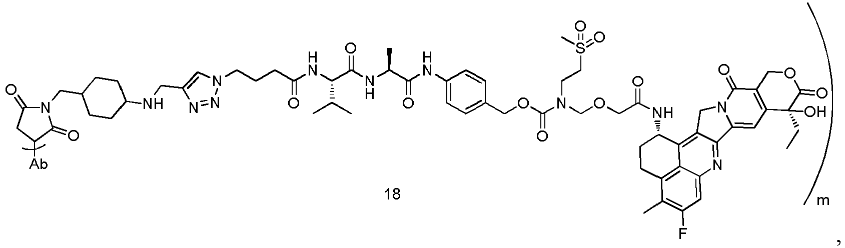 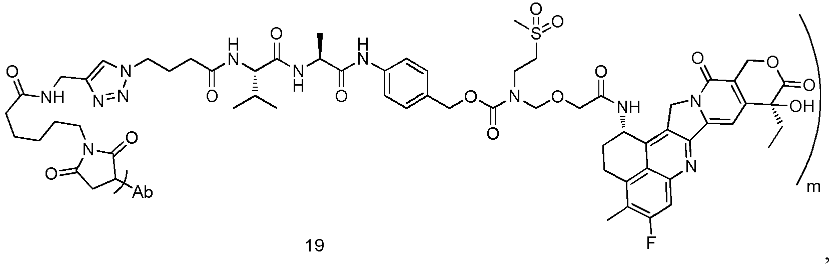 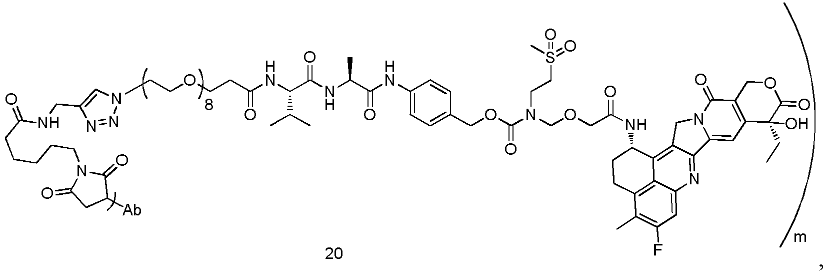 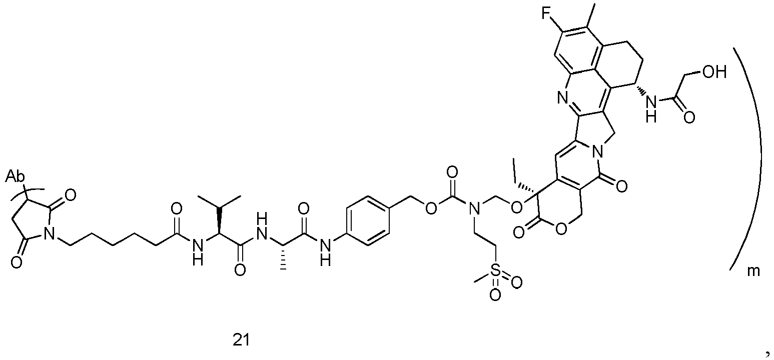 or 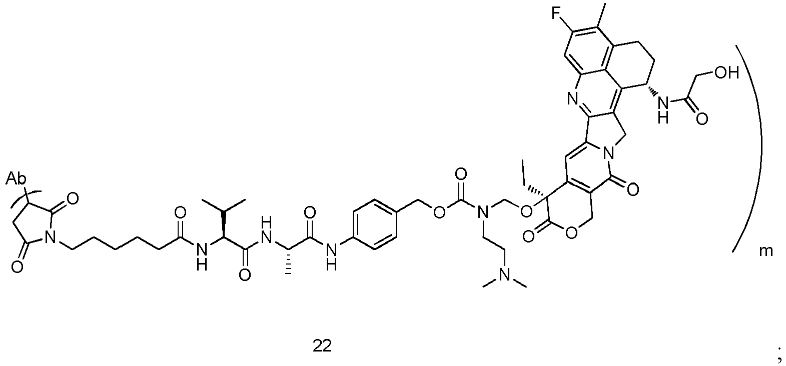 wherein Ab is the TROP2 antibody or the variant of the TROP2 antibody, and m is 3.8 to 4.2, preferably 3.88, 3.90, 3.96, 3.97, 3.98, 4.00, 4.03, 4.05, 4.10, 4.12, or 4.13; the amino acid sequence of the light chain in the variant of the TROP2 antibody is preferably shown in SEQ ID NO: 1, the amino acid sequence of the heavy chain is preferably shown in SEQ ID NO: 2.

In a preferred embodiment of the present disclosure, the antibody-drug conjugate is preferably any one of the following compounds: 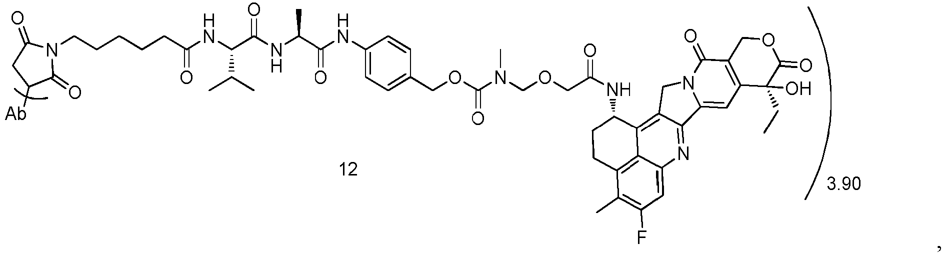 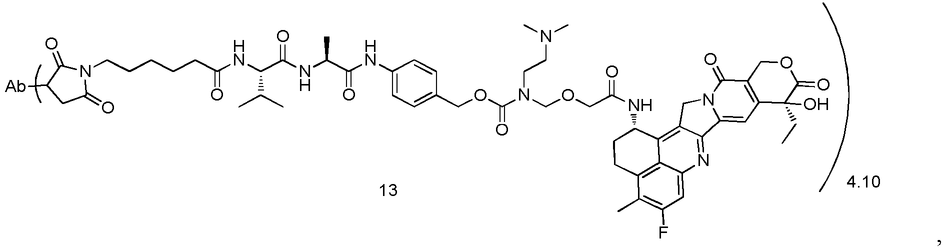 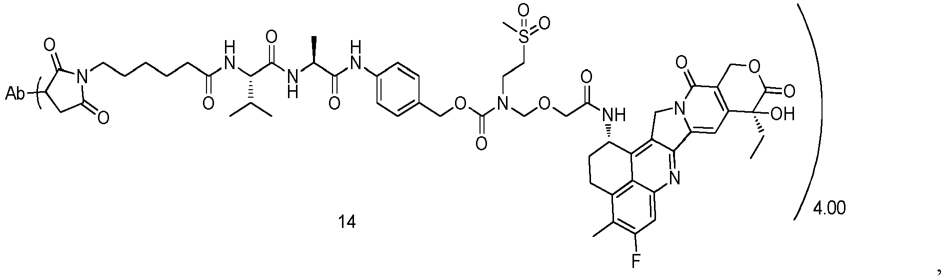 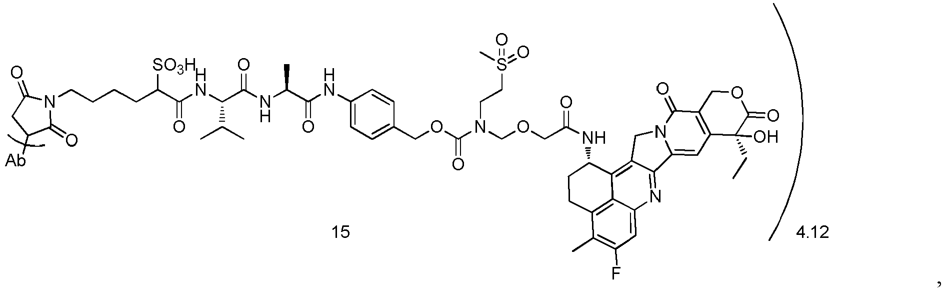 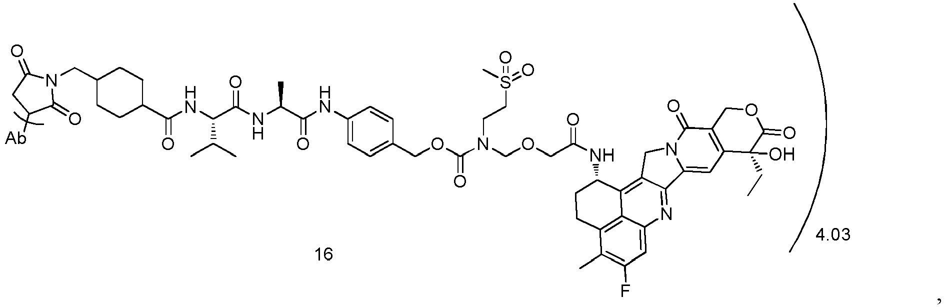 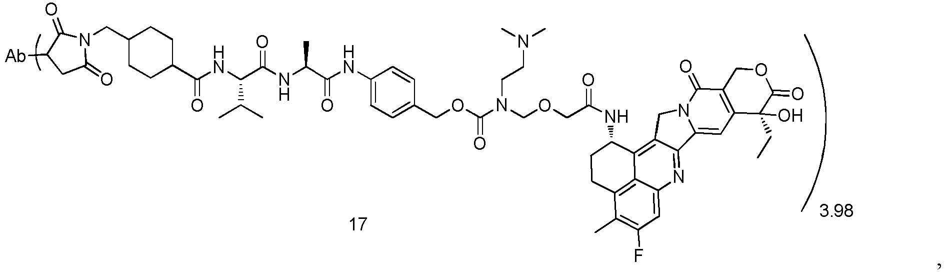 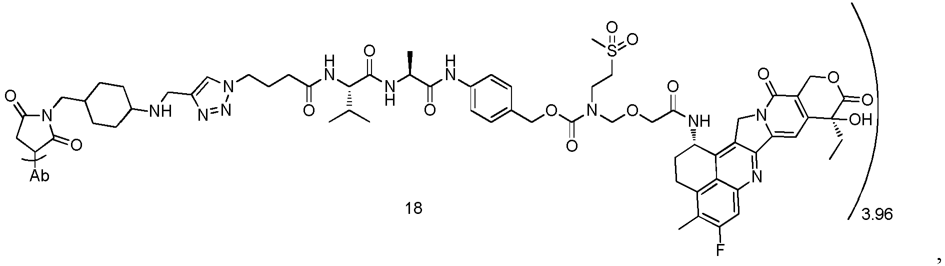 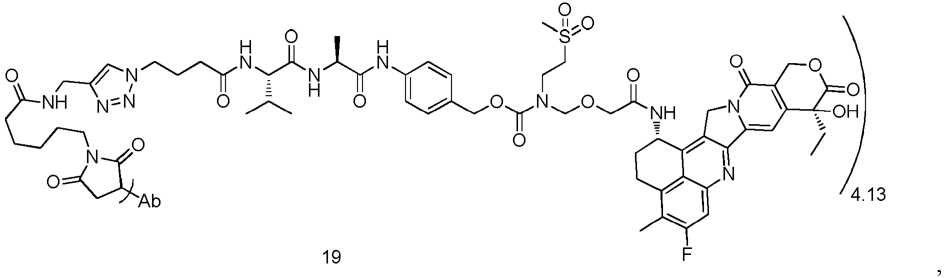 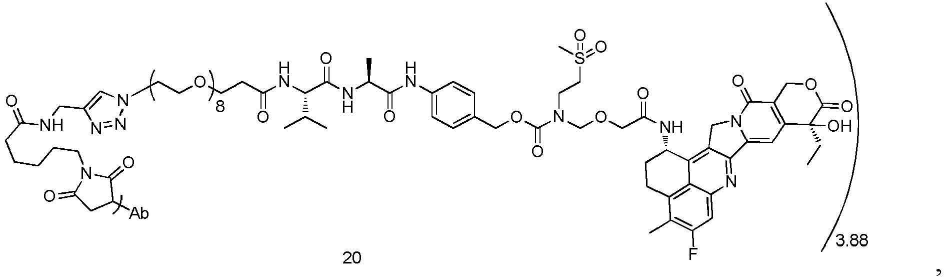 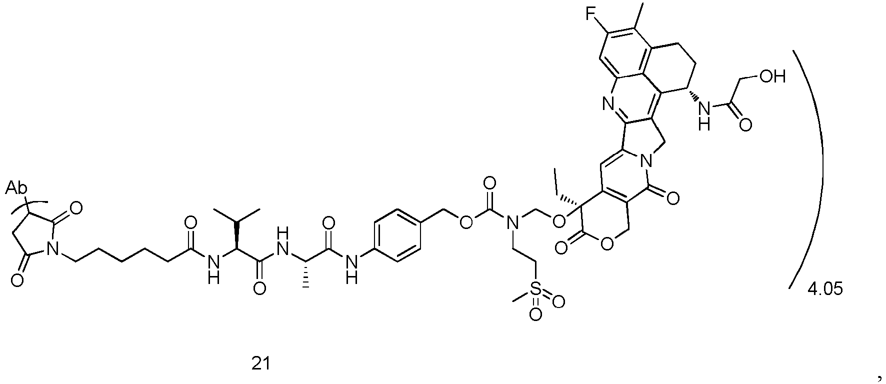 or 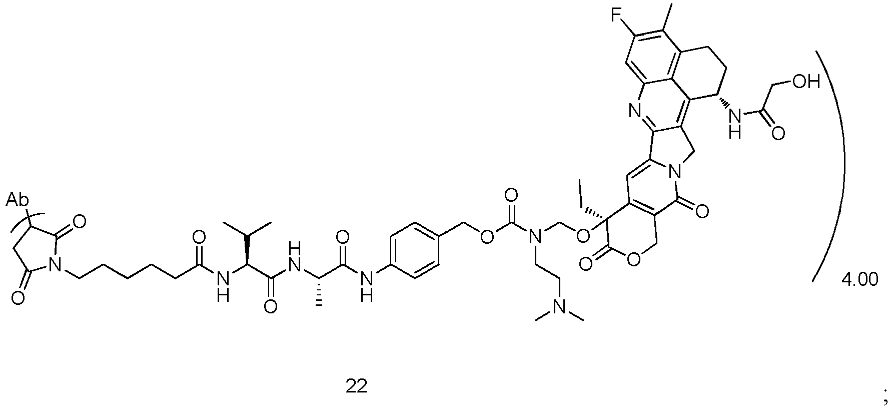 wherein Ab is the TROP2 antibody or the variant of the TROP2 antibody, preferably the TROP2 antibody; the amino acid sequence of the light chain in the variant of the TROP2 antibody is shown in SEQ ID NO: 1, and the amino acid sequence of the heavy chain is shown in SEQ ID NO: 2.

In a preferred embodiment of the present disclosure, the antibody-drug conjugate is preferably any one of the following compounds: 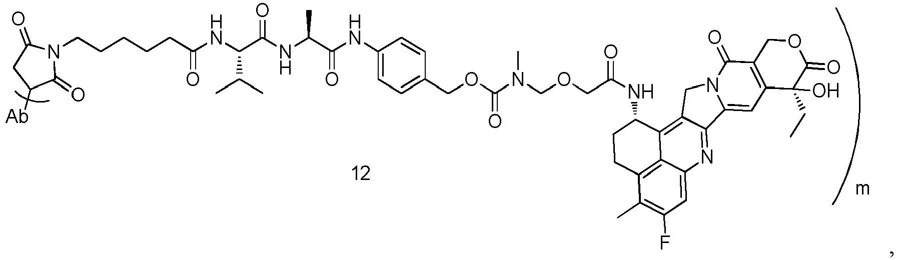 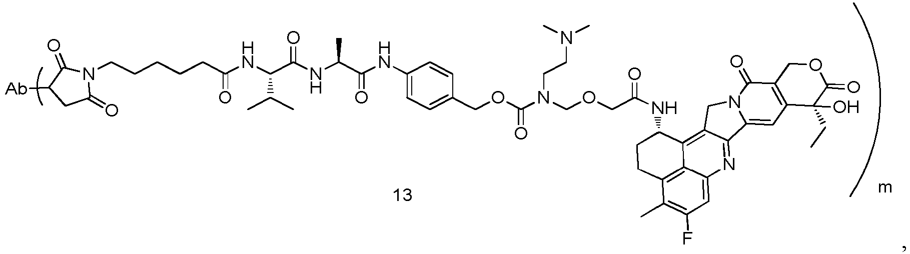 or 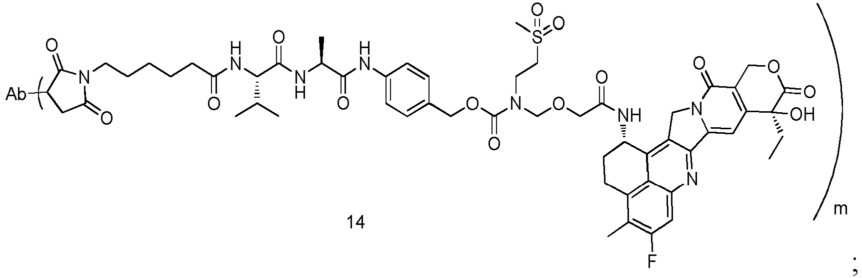 wherein Ab is the TROP2 antibody or the variant of the TROP2 antibody; m is preferably 3.90, 4.00, or 4.10; the amino acid sequence of the light chain in the variant of the TROP2 antibody is shown in SEQ ID NO: 1, and the amino acid sequence of the heavy chain is shown in SEQ ID NO: 2.

In a preferred embodiment of the present disclosure, the antibody-drug conjugate is preferably any one of the following compounds: 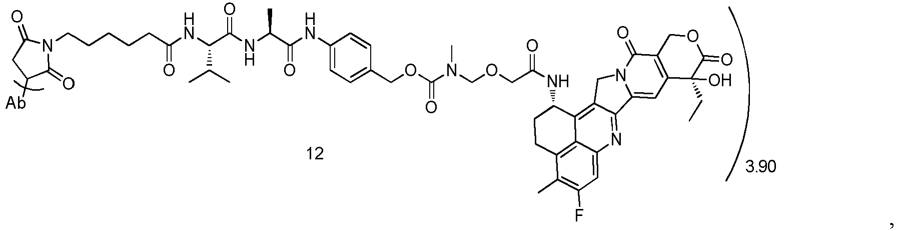 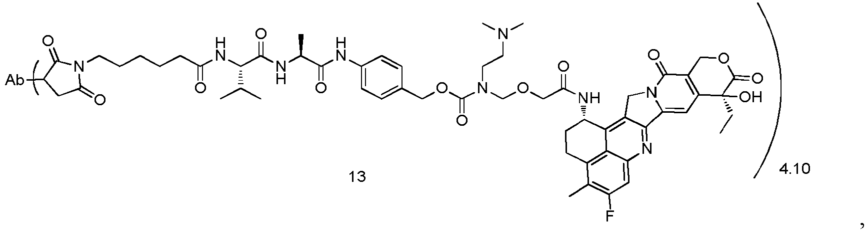 or 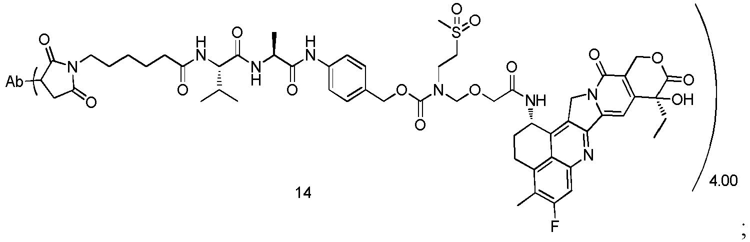 wherein Ab is the TROP2 antibody or the variant of the TROP2 antibody.

In a preferred embodiment of the present disclosure, the antibody-drug conjugate is preferably any one of the following compounds: 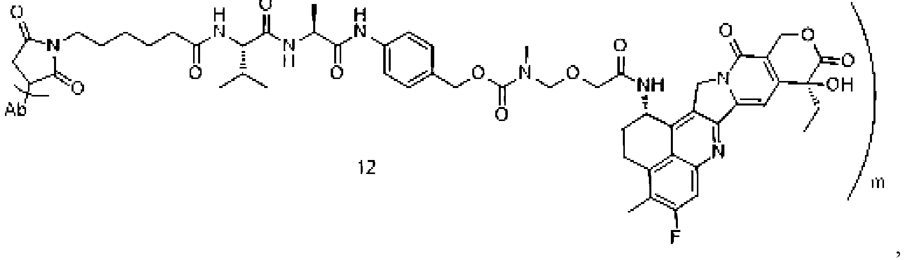 Ab is the TROP2 antibody, and m is preferably 3.90; 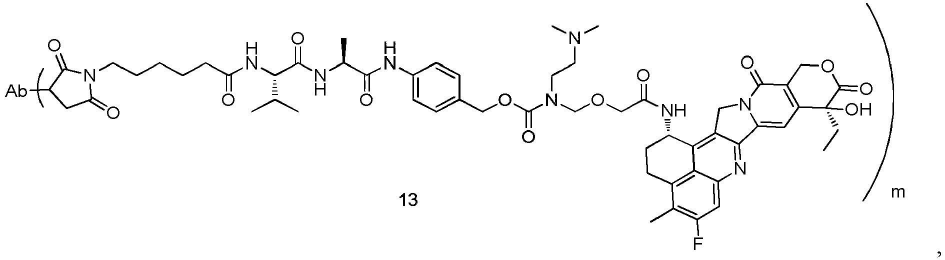 Ab is the TROP2 antibody, and m is preferably 4.10; 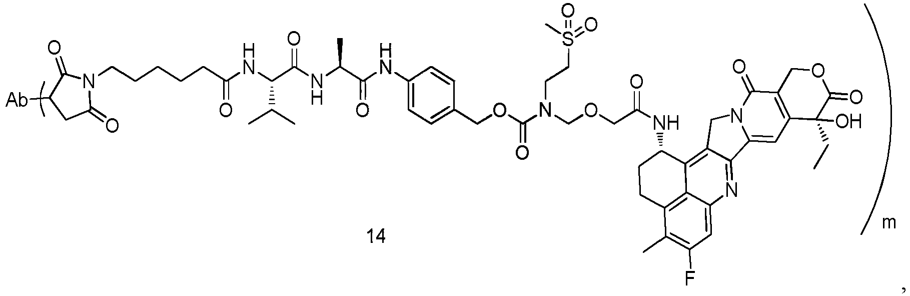 Ab is the TROP2 antibody, and m is preferably 4.00; 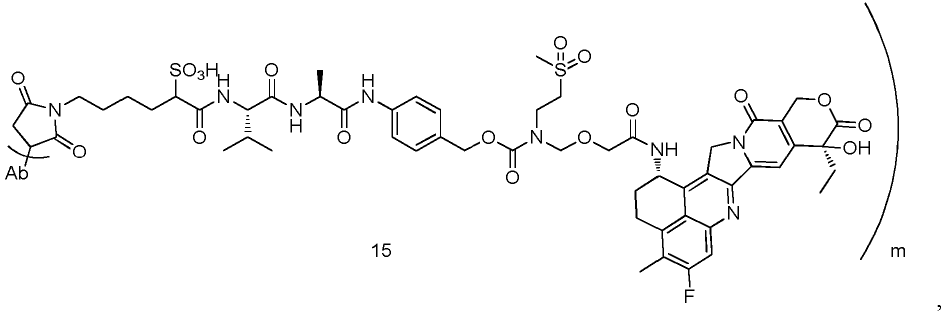 Ab is the TROP2 antibody, and m is preferably 4.12; 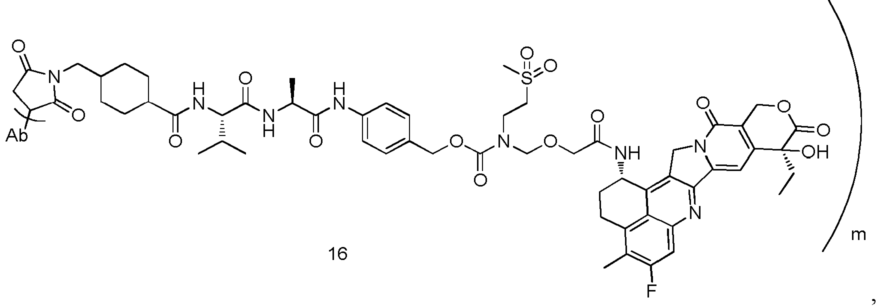 Ab is the TROP2 antibody, and m is preferably 4.03; 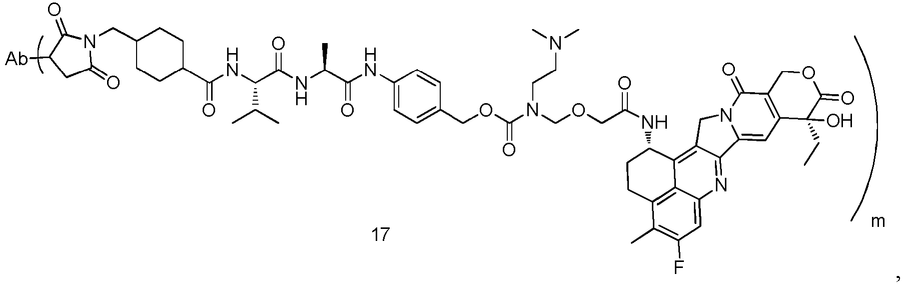 Ab is the TROP2 antibody, and m is preferably 3.98; 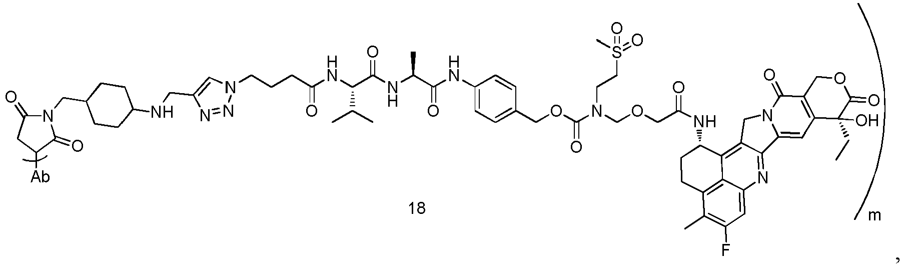 Ab is the TROP2 antibody, and m is preferably 3.96; 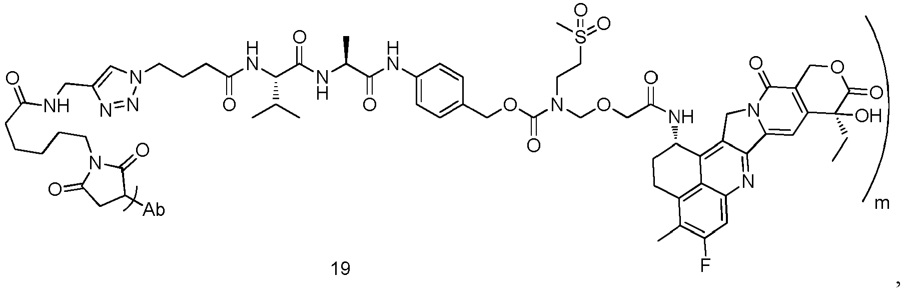 Ab is the TROP2 antibody, and m is preferably 4.13; 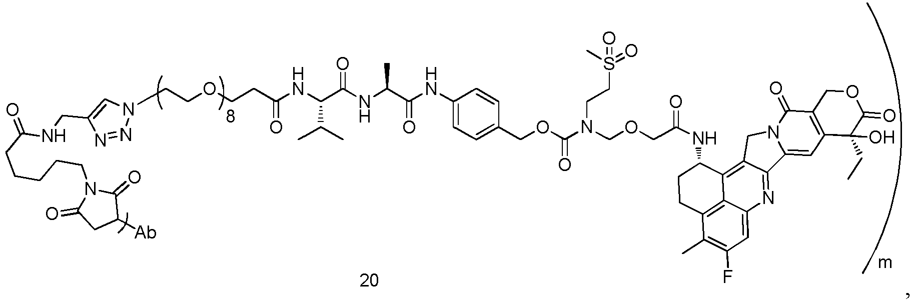 Ab is the TROP2 antibody, and m is preferably 3.88; 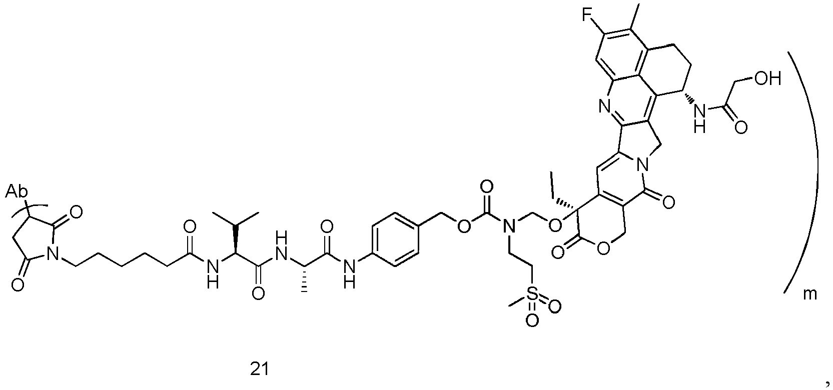 Ab is the TROP2 antibody, and m is preferably 4.05; 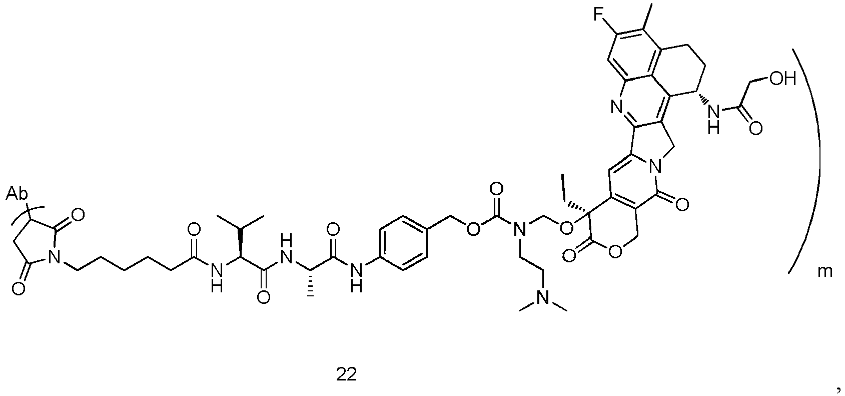 Ab is the TROP2 antibody, and m is preferably 4.00.

The present disclosure also provides a method for preparing the antibody-drug conjugate, comprising the following step: carrying out a coupling reaction between a compound of formula II and Ab-hydrogen as shown below; 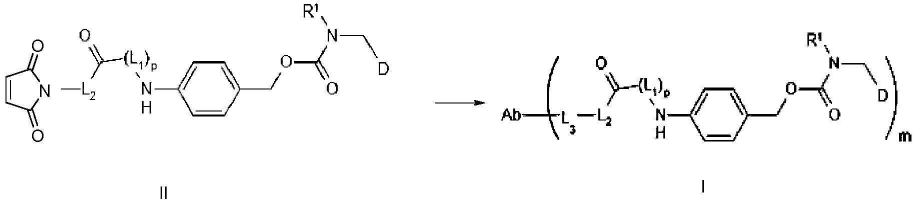 wherein L₁, L₂, L₃, R¹, p, and Ab are defined as above.

In the present disclosure, the conditions and operations of the coupling reaction can be conventional conditions and operations for the coupling reaction in the art.

The present disclosure also provides a pharmaceutical composition, comprising substance X and a pharmaceutically acceptable excipient, wherein the substance X is the antibody-drug conjugate, the pharmaceutically acceptable salt thereof, the solvate thereof, or the solvate of the pharmaceutically acceptable salt thereof.

In the pharmaceutical composition, the substance X may be used in a therapeutically effective amount.

The present disclosure also provides substance X for use in inhibiting TROP2 protein. The present disclosure also provides substance X for use in treating and/or preventing a tumor, and the tumor is preferably a TROP2 positive tumor.

In the use, the TROP2 positive tumor is preferably one or more than one of TROP2 positive gastric cancer, triple negative breast cancer, and human pancreatic cancer.

In some embodiments of the present disclosure, for the gastric cancer, the gastric cancer cells are *NCI-N87* cells;
in some embodiments of the present disclosure, for the triple negative breast cancer, the triple negative breast cancer cells are MDA-MB-468 cells;
in some embodiments of the present disclosure, for the pancreatic cancer, the pancreatic cancer cells are BXPC3 cells.

Unless otherwise indicated, the following terms appearing in the specification and claims of the present disclosure have the following meanings:
The pharmaceutical excipient may be an excipient widely used in the field of pharmaceutical production. The excipient is mainly used to provide a safe, stable, and functional pharmaceutical composition, and can also provide a method for the subject to dissolve the active ingredient at a desired rate after administration, or to facilitate effective absorption of the active ingredient after the subject receives administration of the composition. The pharmaceutical excipient can be an inert filler or provide a certain function, such as stabilizing the overall pH value of the composition or preventing degradation of the active ingredient in the composition. The pharmaceutical excipients may include one or more of the following excipients: buffers, chelating agents, preservatives, solubilizers, stabilizers, vehicles, surfactants, colorants, flavoring agents and sweeteners.

Natural or natural sequence TROP2 can be isolated from nature or produced by recombinant DNA technology, chemical synthesis, or a combination of the above and similar techniques.

Antibody is interpreted in the broadest sense here, which can specifically bind to the target through at least one antigen recognition region located in the variable region of the immunoglobulin molecule, such as carbohydrate, polynucleotide, fat, polypeptide, etc. Specifically, it includes complete monoclonal antibodies, polyclonal antibodies, bispecific antibodies, and antibody fragments, as long as they have the required biological activity. Variants of antibodies of the present disclosure refer to amino acid sequence mutants, and covalent derivatives of natural polypeptides, provided that the biological activity equivalent to that of natural polypeptides is retained. The difference between amino acid sequence mutants and natural amino acid sequences is generally that one or more amino acids in the natural amino acid sequence are substituted or one or more amino acids are deleted and/or inserted in the polypeptide sequence. Deletion mutants include fragments of natural polypeptides and N-terminal and/or C-terminal truncation mutants. Generally, the amino acid sequence mutant is at least 70% (e.g., 70%, 75%, 80%, 85%, 90%, 95%, 98%, or 99%) identical to the natural sequence.

The antibodies of the present disclosure can be prepared using techniques well known in the art, such as hybridoma methods, recombinant DNA techniques, phage display techniques, synthetic techniques, or combinations thereof, or other techniques known in the art.

Description of the term "drug-antibody ratio"(DAR). L-D is a reactive group with the conjugation site on the antibody, L is a linker, D is a cytotoxic agent further coupled to the antibody connected to L. In the present disclosure, D is Dxd. The number of DAR of each antibody finally coupled to D is represented by m or m can also represent the number of D coupled to a single antibody. In some embodiments, m is 3.88, 3.90, 3.96, 3.97, 3.98, 4.00, 4.03, 4.05, 4.10, 4.12, or 4.13.

The linker refers to the direct or indirect connection between the antibody and the drug. The linker can be connected to the mAb in many ways, such as through surface lysine, reductive coupling to oxidized carbohydrates, and through cysteine residues released by reducing interchain disulfide bonds. Many ADC connection systems are known in the art, including connections based on hydrazones, disulfides, and peptides.

The term "pharmaceutically acceptable salt" refers to salt prepared from the compound of the present disclosure and a relatively non-toxic and pharmaceutically acceptable acid or alkali. When the compound of the present disclosure contains relatively acidic functional groups, an alkali addition salt can be obtained by contacting a sufficient amount of pharmaceutically acceptable alkali with the neutral form of the compound in a pure solution or an appropriate inert solvent. The pharmaceutically acceptable alkali addition salt includes, but is not limited to, lithium salt, sodium salt, potassium salt, calcium salt, aluminium salt, magnesium salt, zinc salt, bismuth salt, ammonium salt, and diethanolamine salt. When the compound of the present disclosure contains relatively alkaline functional groups, an acid addition salt can be obtained by contacting a sufficient amount of pharmaceutically acceptable acid with the neutral form of the compound in a pure solution or an appropriate inert solvent. The pharmaceutically acceptable acid includes inorganic acid, and the inorganic acid includes, but is not limited to, hydrochloric acid, hydrobromic acid, hydroiodic acid, nitric acid, carbonic acid, phosphoric acid, phosphorous acid, sulfuric acid, etc. The pharmaceutically acceptable acid includes organic acid, and the organic acid includes, but is not limited to, acetic acid, propionic acid, oxalic acid, isobutyric acid, maleic acid, malonic acid, benzoic acid, succinic acid, suberic acid, trans-butenedioic acid, lactic acid, mandelic acid, phthalic acid, benzenesulfonic acid, p-toluenesulfonic acid, citric acid, salicylic acid, tartaric acid, methanesulfonic acid, isonicotinic acid, acidic citric acid, oleic acid, tannic acid, pantothenic acid, bitartrate, ascorbic acid, gentisic acid, fumaric acid, gluconic acid, saccharic acid, formic acid, ethanesulfonic acid, pamoic acid (i.e., 4,4'-methylene-bis(3-hydroxy-2-naphthoic acid)), amino acid (e.g., glutamic acid and arginine), etc. When the compound of the present disclosure contains relatively acidic functional groups and relatively alkaline functional groups, it can be converted into an alkali addition salt or an acid addition salt. For details, see Berge et al., "Pharmaceutical Salts", Journal of Pharmaceutical Science 66: 1-19 (1977), or Handbook of Pharmaceutical Salts: Properties, Selection, and Use (P. Heinrich Stahl and Camille G. Wermuth, ed., Wiley-VCH, 2002).

The term "solvate" refers to a substance formed by combining the compound of the present disclosure with a stoichiometric or non-stoichiometric amount of solvent. Solvent molecules in the solvate can exist in an ordered or non-ordered arrangements. The solvent includes, but is not limited to, water, methanol, ethanol, etc.

The term "treatment" or its equivalent expression, when used for, for example, cancer, refers to a procedure or process for reducing or eliminating the number of cancer cells in a patient or alleviating the symptoms of cancer. The "treatment" of cancer or another proliferative disorder does not necessarily mean that the cancer cells or other disorders will actually be eliminated, but the number of cells or disorders will actually be reduced or the symptoms of cancers or other disorders will actually be alleviated. Generally, the method of treating cancer will be carried out even if it has only a low probability of success, but it is still considered to induce an overall beneficial course of action considering the patient's medical history and estimated survival expectation.

The term "prevention" refers to a reduced risk of acquiring or developing a disease or disorder.

The term "cycloalkyl" refers to a saturated cyclic hydrocarbon group with three to twenty carbon atoms (e.g., C₃-C₆ cycloalkyl), including monocyclic and polycyclic cycloalkyl. The cycloalkyl contains 3 to 20 carbon atoms, preferably 3 to 10 carbon atoms, and more preferably 3 to 6 carbon atoms. Examples of cycloalkyl include, but are not limited to, cyclopropyl, cyclobutyl, cyclopentyl, cyclohexyl, cycloheptyl, cyclooctyl, cyclononyl, and cyclodecyl.

The term "alkyl" refers to a straight or branched chain alkyl group with a specified number of carbon atoms. Examples of alkyl groups include methyl, ethyl, n-propyl, isopropyl, n-butyl, t-butyl, isobutyl, sec-butyl, n-pentyl, isopentyl, n-hexyl, n-heptyl, n-octyl, and similar alkyl groups.

The term "halogen" refers to fluorine, chlorine, bromine, or iodine.

The term "heteroaryl" refers to an aryl group (or aromatic ring) containing 1, 2, 3, or 4 heteroatoms independently selected from N, O, and S, which may be a monocyclic, bicyclic, or tricyclic aromatic system, such as furyl, pyridinyl, pyridazinyl, pyrimidinyl, pyrazinyl, thiophenyl, isoxazolyl, oxazolyl, diazolyl, imidazolyl, pyrrolyl, pyrazolyl, triazolyl, tetrazolyl, thiazolyl, isothiazolyl, thiadiazolyl, benzimidazolyl, indolyl, indazolyl, benzothiazolyl, benzisothiazolyl, benzoxazolyl, benzisoxazolyl, quinolyl, isoquinolyl, etc.

The term "aryl" refers to any stable monocyclic or bicyclic carbocycle, wherein all rings are aromatic rings. Examples of the aryl moiety include phenyl or naphthyl.

The above preferred conditions can be combined arbitrarily to obtain preferred embodiments of the present disclosure without violating common knowledge in the art.

Unless otherwise specified, room temperature in the present disclosure refers to 20 to 30°C.

The reagents and raw materials used in the present disclosure are all commercially available.

The positive progressive effects of the present disclosure are as follows:
1. The antibody-drug conjugate containing TROP2 antibody provided by the present disclosure has good targeting ability and has a good inhibitory effect on various tumor cells expressing TROP2, etc.
2. In vivo studies have shown that the antibody-drug conjugate of the present disclosure has better in vitro cytotoxicity and in vivo antitumor activity.
3. The antibody-drug conjugate of the present disclosure has good solubility and good druggability. There is no abnormal phenomenon such as precipitation during the coupling preparation process, which is very conducive to the preparation of the antibody-drug conjugate.

### BRIEF DESCRIPTION OF THE DRAWINGS

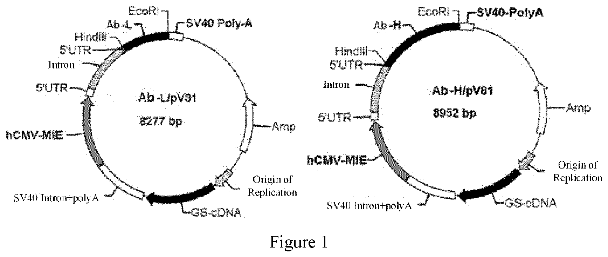

Figure 1 shows the construction of expression vectors for the light and heavy chains of an FDA018 antibody; wherein Ab-L is the light chain of the antibody, and Ab-H is the heavy chain of the antibody.

### DETAILED DESCRIPTION OF THE PREFERRED EMBODIMENT

The present disclosure will be further described below with reference to examples, but the present disclosure is not therefore limited to the scope of the examples. Experimental methods without specific conditions in the following examples are selected according to conventional methods and conditions, or according to the commercial specification.

### Description of abbreviations:

- PCR: polymerase chain reaction
- CHO: Chinese hamster ovary cells
- HTRF: homogeneous time-resolved fluorescence
- PB: phosphate buffer
- EDTA: ethylenediaminetetraacetic acid
- TECP: tris(2-carboxyethyl)phosphine
- DMSO: dimethyl sulfoxide
- DMF: N,N-dimethylformamide
- His: histidine
- HATU: 2-(7-azabenzotriazol-1-yl)-N,N,N',N'-tetramethyluronium hexafluorophosphate
- v/v: V/V, volume ratio
- UV: ultraviolet visible light
- ELISA: enzyme-linked immunosorbent assay
- BSA: bovine serum *albumin*
- rpm: revolutions per minute
- FBS: fetal bovine serum

### Example 1: Preparation of TROP2 antibody

In the present disclosure, the monoclonal antibody FDA018 with high affinity and specific targeting TROP2 was selected, the amino acid sequence of its light chain was shown in SEQ ID NO: 1, and the amino acid sequence of its heavy chain was shown in SEQ ID NO: 2. The light and heavy chain nucleotide sequences of FDA018 were obtained by whole gene synthesis (Suzhou Genewiz). They were separately constructed into the pV81 vector (as shown in Figure 1) by double digestion with EcoR I and Hind III (purchased from TAKARA), and then transformed into Trans 1-T1 competent cells (purchased from Beijing TransGen Biotech, product number: CD501-03) by ligation, which were picked for cloning, PCR identification and sent for sequencing confirmation. Positive clones were cultured and expanded for plasmid extraction, thus obtaining the antibody light chain eukaryotic expression plasmid FDA018-L/pV81 and the antibody heavy chain eukaryotic expression plasmid FDA018-H/pV81. These two plasmids were linearized by digestion with XbaI (purchased from Takara, product number: 1093S). The light and heavy chain eukaryotic expression plasmids were transformed into CHO cells adapted to suspension growth (purchased from ATCC) at a ratio of 1.5/1 by electroporation. After electroporation, the cells were seeded at 2000 to 5000 cells/well in a 96-well plate. After 3 weeks of culture, the expression level was measured by HTRF method (homogeneous time-resolved fluorescence). The top ten cell pools in terms of expression level were selected for expansion and cryopreservation. A cell was revived into a 125 mL shake flask (culture volume of 30 mL) and cultured at 37°C, 5.0% CO₂, and 130 rpm by vibration for 3 days, then expanded to a 1000 mL shake flask (culture volume of 300 mL) and cultured at 37°C, 5.0% CO₂, and 130 rpm by vibration. Starting on the 4th day, 5 to 8% of the initial culture volume of replenishment culture medium was added every other day. The culture was ended on 10th to 12th day and the harvest liquid was centrifuged at 9500 rpm for 15 minutes to remove the cell precipitate. The supernatant was collected and filtered through a 0.22 µm filter membrane. The treated sample was purified using a MabSelect affinity chromatography column (purchased from GE) to obtain antibody FDA018.

The amino acid sequence of the FDA018 light chain is shown below:

SEQ ID NO: 1:

DIQLTQSPSS LSASVGDRVS ITCKASQDVS IAVAWYQQKP GKAPKLLIYS 50

ASYRYTGVPD RFSGSGSGTD FTLTISSLQP EDFAVYYCQQ HYITPLTFGA 100

GTKVEIKRTV AAPSVFIFPP SDEQLKSGTA SVVCLLNNFY PREAKVQWKV 150

DNALQSGNSQ ESVTEQDSKD STYSLSSTLT LSKADYEKHK VYACEVTHQG 200

LSSPVTKSFN RGEC 214

The amino acid sequence of FDA018 heavy chain is shown below:

SEQ ID NO: 2:

QVQLQQSGSE LKKPGASVKV SCKASGYTFT NYGMNWVKQA PGQGLKWMGW 50

INTYTGEPTY TDDFKGRFAF SLDTSVSTAY LQISSLKADD TAVYFCARGG 100

FGSSYWYFDV WGQGSLVTVS SASTKGPSVF PLAPSSKSTS GGTAALGCLV 150

KDYFPEPVTV SWNSGALTSG VHTFPAVLQS SGLYSLSSVV TVPSSSLGTQ 200

TYICNVNHKP SNTKVDKRVE PKSCDKTHTC PPCPAPELLG GPSVFLFPPK 250

PKDTLMISRT PEVTCVVVDV SHEDPEVKFN WYVDGVEVHN AKTKPREEQY 300

NSTYRVVSVL TVLHQDWLNG KEYKCKVSNK ALPAPIEKTI SKAKGQPREP 350

QVYTLPPSRE EMTKNQVSLT CLVKGFYPSD IAVEWESNGQ PENNYKTTPP 400

VLDSDGSFFL YSKLTVDKSR WQQGNVFSCS VMHEALHNHY TQKSLSLSPG 450

K 451

### Example 2: Synthesis of linker-drug conjugates

### Example 2-1: Synthesis of LE12

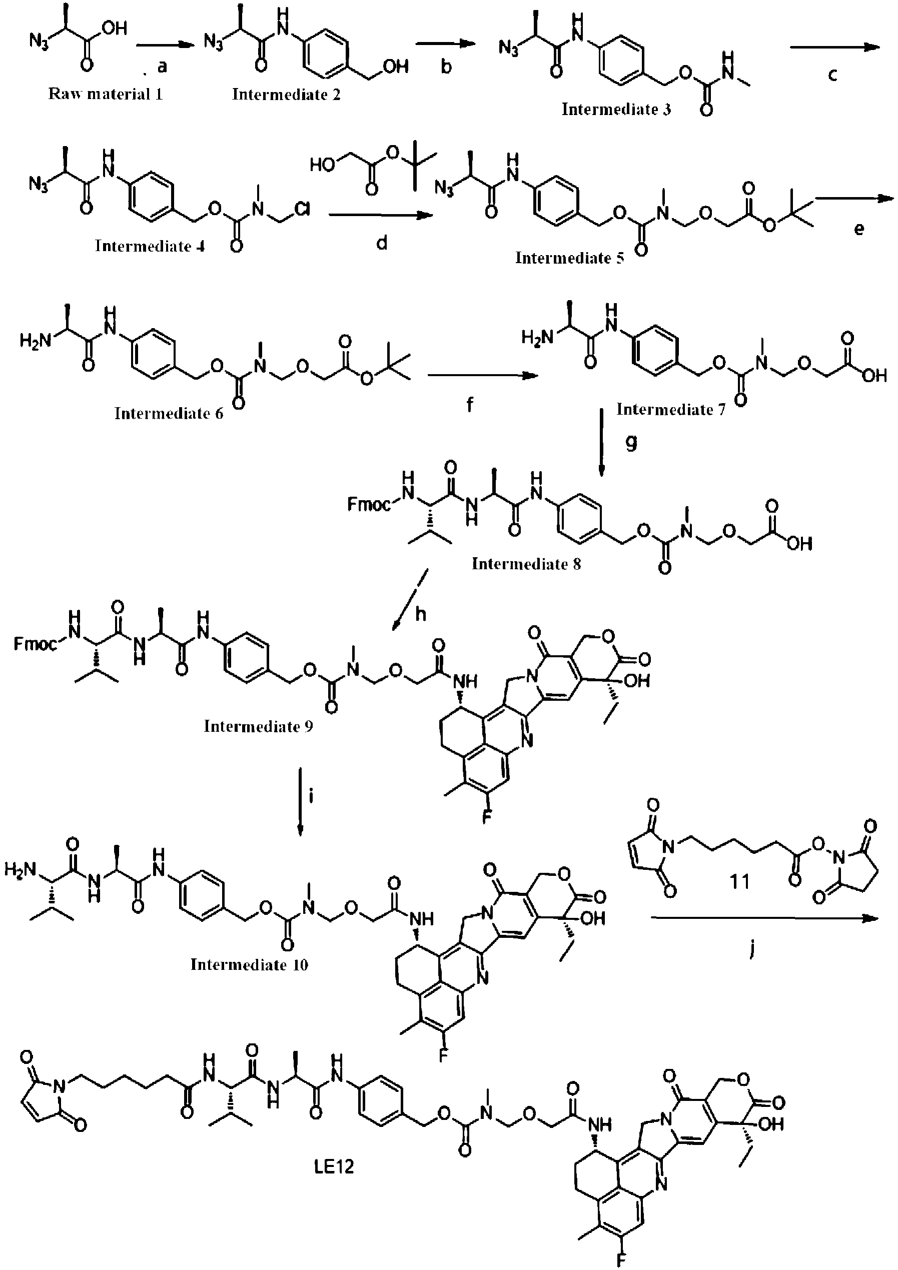

### Synthesis of Intermediate 2:

(S)-2-Azidopropionic acid (10 g, 86.9 mmol) and 4-aminobenzyl alcohol (21.40 g, 173.8 mmol) were dissolved in a mixed solvent of 300 mL of dichloromethane and methanol (in a volume ratio of 2:1), and then 2-ethoxy-1-ethoxycarbonyl-1,2-dihydroquinoline (21.49 g, 86.9 mmol) was added thereto. The reaction was carried out at room temperature for 5 hours. The solvent was then evaporated under reduced pressure and the obtained crude product was purified by silica gel column chromatography [dichloromethane: ethyl acetate = 1:1 (v/v)] to obtain intermediate 2 (16.3 g, yield of 85%), ESI-MS m/z: 221 (M+H).

### Synthesis of Intermediate 3:

Intermediate 2 (15 g, 68.2 mmol) was mixed with bis(p-nitrophenyl)carbonate (22.82 g, 75.02 mmol) and dissolved in 200 mL of anhydrous N,N-dimethylformamide, and then 25 mL of triethylamine was added thereto, and the reaction was carried out at room temperature for 2 hours. After the complete reaction of the raw materials was monitored by liquid chromatography-mass spectrometry, methylamine hydrochloride (6.91 g, 102.3 mmol) was added thereto, and the reaction was continued at room temperature for 1 hour. After the reaction was completed, most of the solvent was removed by distillation under reduced pressure, then 200 mL of water and 200 mL of ethyl acetate were added thereto. The organic phase was collected after the phases were separated, and the organic phase was dried and concentrated, and then the obtained crude product was purified by silica gel column chromatography [dichloromethane: ethyl acetate = 10:1 (v/v)] to obtain intermediate 3 (18.9 g, yield of 100%), ESI-MS m/z: 278 (M+H).

### Synthesis of Intermediate 5:

Intermediate 3 (10 g, 36.1 mmol) was mixed with polyformaldehyde (1.63 g, 54.2 mmol) and dissolved in 150 mL of anhydrous dichloromethane. Trimethylchlorosilane (6.28 g, 57.76 mmol) was slowly added thereto and the reaction was carried out at room temperature for 2 hours to obtain a crude solution of intermediate 4. The reaction was monitored by liquid chromatography-mass spectrometry after sampling and quenching with methanol. After the reaction was completed, the reaction mixture was filtered and then tert-butyl hydroxyacetate (9.54 g, 72.2 mmol) and triethylamine (10 mL, 72.2 mmol) were added to the filtrate and the reaction was continued at room temperature for 2 hours. After the reaction was completed, most of the solvent was removed by distillation under reduced pressure, and then the obtained crude product was purified by silica gel column chromatography [petroleum ether: ethyl acetate = 3:1 (v/v)] to obtain intermediate 5 (11.2 g, yield of 74%), ESI-MS m/z: 422 (M+H).

### Synthesis of Intermediate 6:

Intermediate 5 (10 g, 23.8 mmol) was dissolved in 80 mL of anhydrous tetrahydrofuran, and 80 mL of water was added thereto, and then tris(2-carboxyethylphosphine) hydrochloride (13.6 g, 47.6 mmol) was added thereto and the reaction was carried out for 4 hours at room temperature. After the reaction was completed, the tetrahydrofuran was removed by distillation under reduced pressure, and then the mixture was extracted with ethyl acetate. The obtained organic phase was dried and evaporated to remove the solvent under reduced pressure, and purified by silica gel column chromatography [dichloromethane: methanol = 10:1 (v/v)] to obtain intermediate 6 (8.1 g, yield of 86%), ESI-MS m/z: 396 (M+H).

### Synthesis of Intermediate 8:

Intermediate 6 (5 g, 12.7 mmol) was dissolved in 60 mL of a mixed solvent of dichloromethane and methanol (v/v = 2:1), and 3 mL of trifluoroacetic acid was slowly added thereto, and the reaction was carried out at room temperature for 30 min. After the reaction was completed, an equal volume of water and ethyl acetate were added thereto, and the organic phase was dried and concentrated, and the obtained crude product was directly used in the next step.

The crude product obtained from the previous step was dissolved in 50 mL of anhydrous N,N-dimethylformamide, and then Fmoc-L-valine hydroxysuccinimide ester (8.3 g, 19.1 mmol) and triethylamine (5 mL) were added thereto, and the reaction was carried out at room temperature for 2 hours. After the reaction was completed, most of the solvent was removed by distillation under reduced pressure, and then the obtained crude product was purified by silica gel column chromatography [dichloromethane: methanol = 10:1 (v/v)] to obtain intermediate 8 (5.4 g, yield of 64%), ESI-MS m/z: 661 (M+H).

### Synthesis of Intermediate 9:

Intermediate 8 (1 g, 1.5 mmol) was mixed with Exatecan methanesulfonate (0.568 g, 1 mmol) in 30 mL of anhydrous N,N-dimethylformamide, and then 2-(7-azabenzotriazol-1-yl)-N,N,N',N'-tetramethyluronium hexafluorophosphate (1.14 g, 3.0 mmol) and 2 mL of triethylamine were added thereto, and the reaction was carried out at room temperature for 2 hours. After the reaction was completed, the solvent was removed by distillation under reduced pressure, and then the obtained crude product was purified by silica gel column chromatography [chloroform: methanol = 10:1 (v/v)] to obtain intermediate 9 (0.94 g, yield of 87%), ESI-MS m/z: 1078 (M+H).

### Synthesis of Compound LE12:

Intermediate 9 (1 g, 0.929 mmol) was dissolved in 20 mL of anhydrous DMF, then 0.5 mL of 1,8-diazabicyclo[5.4.0]undec-7-ene was added thereto, and the reaction was carried out at room temperature for 1 hour. After the reaction of the raw materials was completed, N-succinimidyl 6-maleimidohexanoate (428.5 mg, 1.39 mmol) was added directly, and the reaction mixture was stirred at room temperature for 1 hour. The solvent was removed by distillation under reduced pressure, and then the obtained crude product was purified by silica gel column chromatography [chloroform: methanol = 8:1 (v/v)] to obtain the title compound (0.7 g, yield of 73%), ESI-MS m/z: 1035 (M+H).

### Example 2-2: Synthesis of Compound LE13

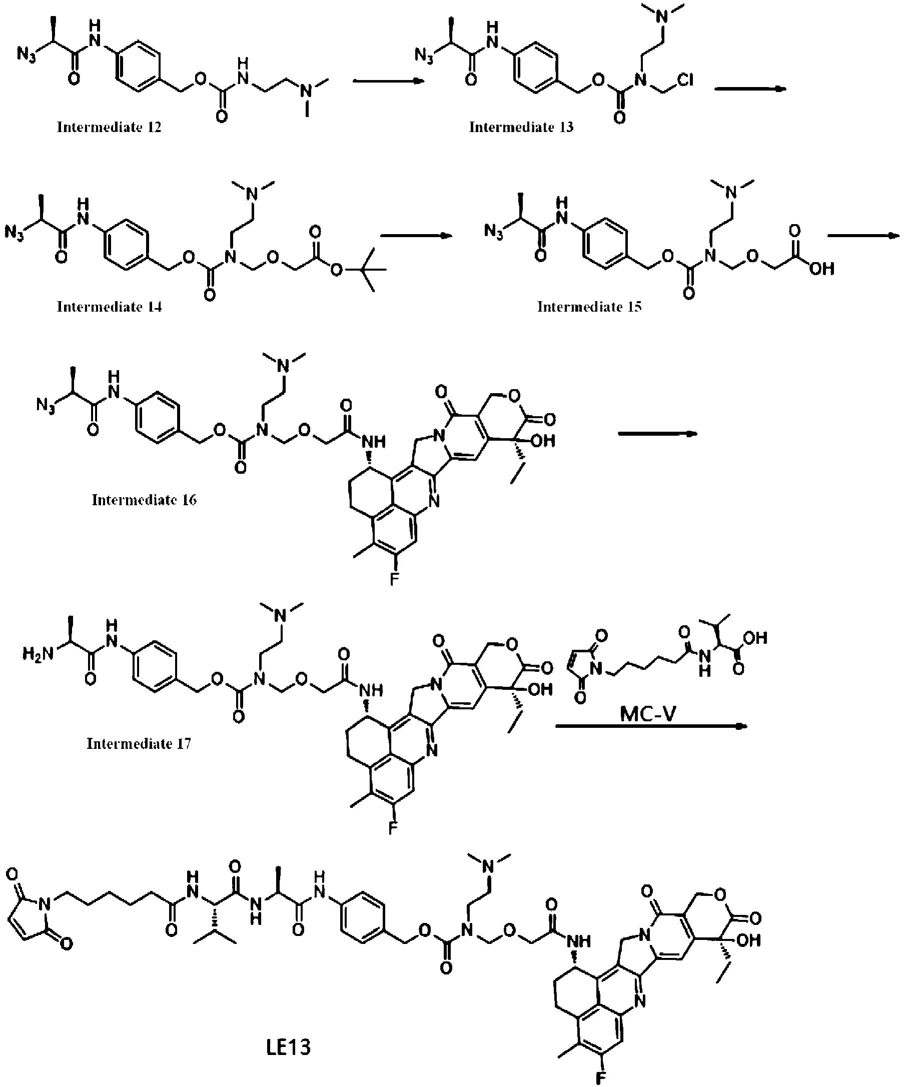

### Synthesis of Intermediate 14

Commercially available intermediate 12 (267 mg, 0.8 mmol) was mixed with paraformaldehyde (50 mg, 1.6 mmol) and dissolved in 20 mL of anhydrous dichloromethane. Then, trimethylchlorosilane (0.3 mL, 3.4 mmol) was added slowly. After the addition was completed, the reaction was carried out at room temperature for 2 hours. Then, the reaction was monitored by liquid chromatography-mass spectrometry after sampling and quenching with methanol. After the reaction was completed, the reaction mixture was filtered, and then tert-butyl 2-hydroxyacetate (211 mg, 1.6 mmol) and pempidine (0.5 mL) were added to the filtrate, and the reaction was continued at room temperature for about 2 hours. After the reaction was completed, most of the solvent was removed by distillation under reduced pressure, and the obtained crude product was purified by silica gel column chromatography [dichloromethane: methanol = 20:1 (v/v)] to obtain intermediate 14 (260 mg, yield of 68%), ESI-MS m/z: 479 (M+H).

### Synthesis of Intermediate 15

Intermediate 14 (238 mg, 0.50 mmol) was dissolved in 6 mL of a mixed solvent of dichloromethane and methanol (v/v = 2:1), and 0.3 mL of trifluoroacetic acid was slowly added thereto, and the reaction was carried out at room temperature for 30 min. After the reaction was completed, an equal volume of water and ethyl acetate were added thereto, and the organic phase was dried and concentrated, and the obtained crude product was directly used in the next step.

### Synthesis of Intermediate 16

The crude product obtained from the previous step was mixed with Exatecan methanesulfonate (170 mg, 0.30 mmol) in 5 mL of anhydrous N,N-dimethylformamide, and then 2-(7-azabenzotriazol-1-yl)-N,N,N',N'-tetramethyluronium hexafluorophosphate (341 mg, 0.90 mmol) and 0.60 mL of triethylamine were added thereto, and the reaction was carried out at room temperature for 2 hours. After the reaction was completed, the solvent was removed by distillation under reduced pressure, and then the obtained crude product was purified by silica gel column chromatography [chloroform: methanol = 10:1 (v/v)] to obtain intermediate 16 (210 mg, 83%), ESI-MS m/z: 840 (M+H).

### Synthesis of Intermediate 17

Intermediate 16 (100 mg, 0.12 mmol) was dissolved in 15 mL of anhydrous tetrahydrofuran, and 3 mL of water was added thereto, then 0.3 mL of 1 mol/L triethylphosphine aqueous solution was added thereto, and the reaction was carried out at room temperature for 4 hours. After the reaction was monitored to be completed, the reaction mixture was distilled under reduced pressure to remove tetrahydrofuran. Sodium bicarbonate was added to the remaining aqueous solution to adjust the pH to neutral, and then dichloromethane was added for extraction. The obtained organic phase was dried and evaporated under reduced pressure to remove the solvent. The obtained crude product was purified by silica gel column chromatography [dichloromethane: methanol = 10:1 (v/v)] to obtain intermediate 17 (69 mg, yield of 71%), ESI-MS m/z: 814 (M+H).

### Synthesis of Compound LE13

Intermediate 17 (120 mg, 0.15 mmol) obtained according to the previous synthesis method was mixed with the commercially available raw material MC-V(102 mg, 0.33 mmol) in 40 mL of dichloromethane, and the condensation agent 2-ethoxy-1-ethoxycarbonyl-1,2-dihydroquinoline (82 mg, 0.33 mmol) was added to react overnight at room temperature. After the reaction was completed, the solvent was evaporated under reduced pressure and the obtained crude product was purified by silica gel column chromatography [dichloromethane: methanol = 10:1 (v/v)] to obtain compound LE13 (116 mg, yield of 70%), ESI-MS m/z: 1106.5 (M+H).

### Example 2-3: Synthesis of Compound LE14

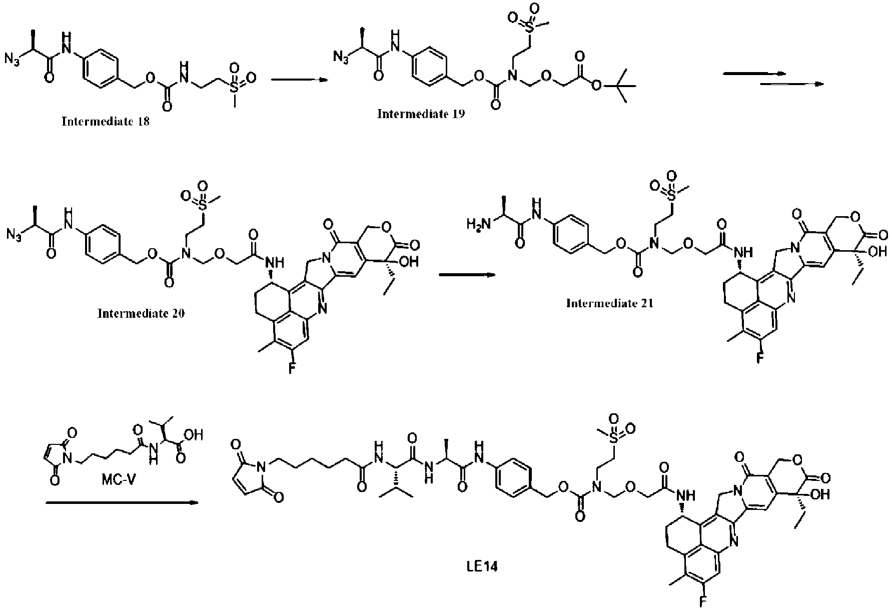

### Synthesis of Intermediate 19

Commercially available intermediate 18 (300 mg, 0.8 mmol) was mixed with polyformaldehyde (50 mg, 1.6 mmol) and dissolved in 20 mL anhydrous dichloromethane. Then, trimethylchlorosilane (0.3 mL, 3.4 mmol) was slowly added thereto, and the reaction was carried out at room temperature for 2 hours. The reaction was monitored by liquid chromatography-mass spectrometry after sampling and quenching with methanol. After the reaction was completed, the reaction mixture was filtered and then tert-butyl 2-hydroxyacetate (211 mg, 1.6 mmol) and triethylamine (0.22 m, 1.6 mmol) were added to the filtrate. The reaction was continued at room temperature for about 2 hours. After the reaction was completed, most of the solvent was removed by distillation under reduced pressure and the obtained crude product was purified by silica gel column chromatography [dichloromethane: methanol = 20:1 (v/v)] to obtain intermediate 19 (349 mg, yield of 85%), ESI-MS m/z: 514 (M+H), ¹H NMR (400 MHz, CDCl₃) δ 8.13 (s, 1H), 7.56 (d, J = 7.5 Hz, 2H), 7.35 (s, 2H), 5.14 (s, 2H), 4.91 (s, 2H), 4.25 (q, J = 7.1 Hz, 1H), 3.99 (d, J = 42.5 Hz, 2H), 3.85 (t, J = 6.2 Hz, 2H), 3.40 (dd, J = 18.5, 7.6 Hz, 2H), 2.89 (d, J = 48.6 Hz, 3H), 1.65 (d, J = 6.8 Hz, 3H), 1.46 (s, 9H).

### Synthesis of Intermediate 20

Intermediate 19 (257 mg, 0.50 mmol) was dissolved in 6 mL of a mixed solvent of dichloromethane and methanol (v/v = 2:1), and 0.3 mL of trifluoroacetic acid was slowly added thereto, and the reaction was carried out at room temperature for 30 min. After the reaction was completed, an equal volume of water and ethyl acetate were added thereto, and the organic phase was dried and concentrated, and the obtained crude product was directly used in the next step.

The obtained crude product was mixed with Exatecan methanesulfonate (170 mg, 0.30 mmol) in 5 mL of anhydrous N,N-dimethylformamide, and then 2-(7-azabenzotriazol-1-yl)-N,N,N',N'-tetramethyluronium hexafluorophosphate (341 mg, 0.90 mmol) and 0.60 mL of triethylamine were added thereto, and the reaction was carried out at room temperature for 2 hours. After the reaction was completed, the solvent was removed by distillation under reduced pressure, and then the obtained crude product was purified by silica gel column chromatography [dichloromethane: methanol = 20:1 (v/v)] to obtain intermediate 20 (212 mg, yield of 81%), ESI-MS m/z: 875 (M+H). ¹H NMR (400 MHz, CDCl₃) δ 8.27 (d, J = 34.7 Hz, 1H), 7.63 - 7.35 (m, 5H), 7.21 - 7.10 (m, 1H), 5.71 - 5.48 (m, 2H), 5.24 - 4.95 (m, 3H), 4.95 - 4.72 (m, 4H), 4.45 (s, 1H), 4.33 - 3.97 (m, 3H), 3.75 (s, 2H), 3.39 - 2.99 (m, 4H), 2.76 (d, J = 15.3 Hz, 3H), 2.43 - 2.15 (m, 5H), 2.04 (s, 1H), 1.94 - 1.75 (m, 2H), 1.62 (d, J = 6.6 Hz, 3H), 1.11 - 0.89 (m, 3H).

### Synthesis of Intermediate 21

Intermediate 20 (77 mg, 0.09 mmol) was dissolved in 12 mL of anhydrous tetrahydrofuran, and 3 mL of water was added thereto, then 0.3 mL of 1 mol/L triethylphosphine aqueous solution was added thereto, and the reaction was carried out at room temperature for 4 hours. After the reaction was completed, the reaction mixture was distilled under reduced pressure to remove tetrahydrofuran. Sodium bicarbonate was added to the remaining aqueous solution to adjust the pH to neutral, and then dichloromethane was added for extraction. The obtained organic phase was dried and evaporated under reduced pressure to remove the solvent. The obtained crude product was purified by silica gel column chromatography [dichloromethane: methanol = 10:1 (v/v)] to obtain intermediate 21 (53 mg, yield of 69%), ESI-MS m/z: 849 (M+H). ¹H NMR (400 MHz, DMSO) δ 8.52 (s, 1H), 7.79 (d, J = 10.8 Hz, 1H), 7.67 - 7.55 (m, 2H), 7.47 - 7.21 (m, 3H), 6.51 (s, 1H), 5.60 (s, 1H), 5.52 - 5.32 (m, 2H), 5.30 - 5.11 (m, 2H), 5.11 - 4.94 (m, 2H), 4.94 - 4.74 (m, 2H), 4.02 (s, 2H), 3.81 - 3.66 (m, 2H), 3.60 - 3.35 (m, 4H), 3.24 - 3.08 (m, 2H), 2.94 (d, J = 30.8 Hz, 3H), 2.39 (s, 3H), 2.28 - 2.04 (m,2H), 2.00 - 1.73 (m, 2H), 1.22 (d, J = 6.6 Hz, 3H), 0.96 - 0.70 (m, 3H).

### Synthesis of Compound LE14

Intermediate 21 (134 mg, 0.16 mmol) was mixed with the commercially available raw material MC-V (102 mg, 0.33 mmol) in 40 mL of dichloromethane, and the condensation agent 2-ethoxy-1-ethoxycarbonyl-1,2-dihydroquinoline (82 mg, 0.33 mmol) was added to react overnight at room temperature. After the reaction was completed, the solvent was evaporated under reduced pressure and the obtained crude product was purified by silica gel column chromatography [dichloromethane: methanol=10:1 (v/v)] to obtain compound LE14 (137 mg, yield of 75%), ESI-MS m/z: 1141.4 (M+H). ¹H NMR (400 MHz, DMSO) δ 9.97 (s, 1H), 8.52 (s, 1H), 8.27 - 8.09 (m, 1H), 7.88 - 7.70 (m, 2H), 7.63 - 7.51 (m, 2H), 7.28 (s, 3H), 6.99 (s, 2H), 6.51 (s, 1H), 5.59 (s, 1H), 5.50 - 5.32 (m, 2H), 5.17 (s, 2H), 4.98 (s, 2H), 4.85 (d, J = 17.3 Hz, 2H), 4.43 - 4.33 (m, 1H), 4.21 - 4.12 (m, 1H), 4.03 (s, 2H), 3.74 - 3.64 (m, 2H), 3.20 - 3.03 (m, 3H), 3.02 - 2.84 (m, 4H), 2.36 (s, 3H), 2.23 - 2.09 (m, 4H), 2.01 - 1.90 (m, 1H), 1.90 - 1.78 (m, 2H), 1.55 - 1.39 (m, 4H), 1.30 (d, J = 6.7 Hz, 3H), 1.23 - 1.11 (m, 2H), 0.93 - 0.77 (m, 9H).

### Example 2-4: Synthesis of Compound LE15-LE20

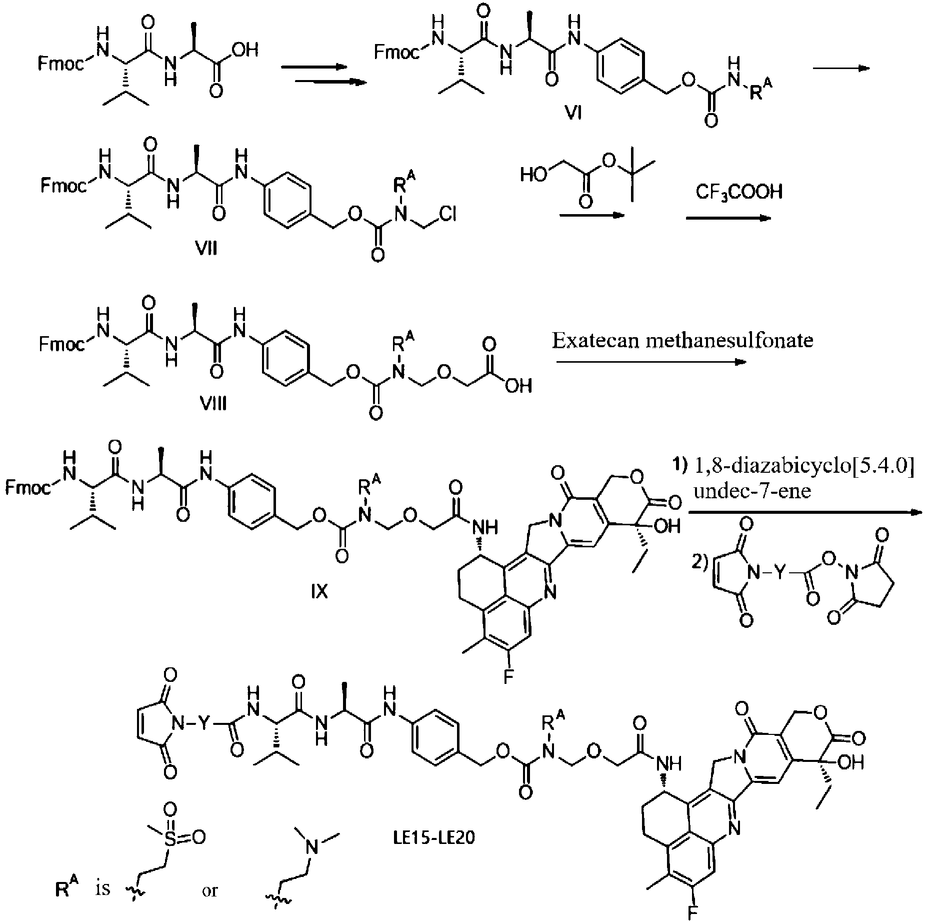

Intermediate VI could be prepared by replacing the methylamine hydrochloride in step b with the corresponding commercially available amino compound, using Fmoc-L-valyl-L-alanine as the starting material and referring to steps a and b in the synthesis method of intermediate 3 in Example 2-1. The subsequent steps were carried out starting from intermediate VI, and with the same methods as those in steps c, d, f, and h of Example 2-1, and intermediate IX similar to intermediate 9 was obtained. Then, following the same steps i and j as Example 6, the amino protecting group was removed, and condensed with different commercially available maleimide compounds to obtain the final product. The structures of the amino compounds and maleimides used are shown in Table 1. Compound LE15: gray-white solid, ESI-MS m/z: 1121.2 (M+H); compound LE16: light yellow solid, ESI-MS m/z: 1167.1 (M+H); compound LE17: yellow solid, ESI-MS m/z: 1132.3 (M+H); compound LE18: light yellow solid, ESI-MS m/z: 1305.4 (M+H); compound LE19: light yellow solid, ESI-MS m/z: 1307.4 (M+H); compound LE20: light yellow solid, ESI-MS m/z: 1337.6 (M+H).

**Table 1. Intermediates used for the synthesis of LE15 to LE20**

| Product | R^{A} | Amino Compound | Maleimide Structure 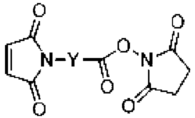 |
|---|---|---|---|
| LE15 | 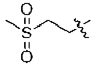 | Methylsulfonyl ethylamine hydrochloride | 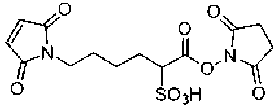 |
| LE16 | 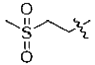 | Methylsulfonyl ethylamine hydrochloride | 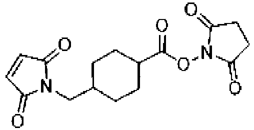 |
| LE17 | 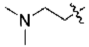 | Dimethylethyla mine hydrochloride | 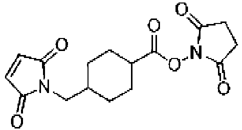 |
| LE18 | 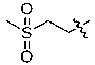 | Methylsulfonyl ethylamine hydrochloride | 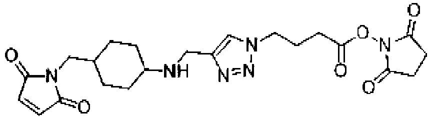 |
| LE19 | 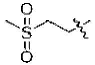 | Methylsulfonyl ethylamine hydrochloride | 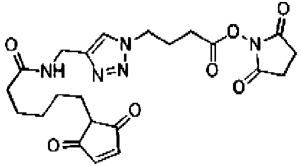 |
| LE20 | 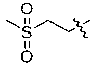 | Methylsulfonyl ethylamine hydrochloride | 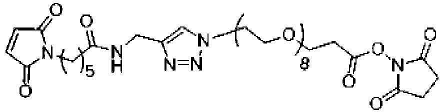 |

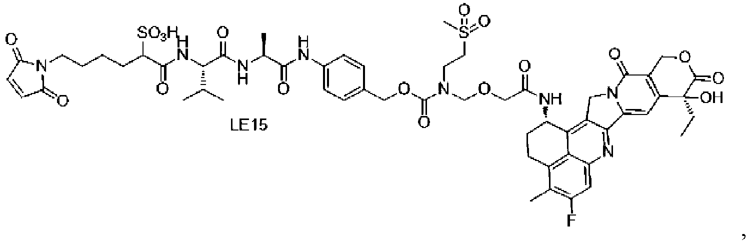

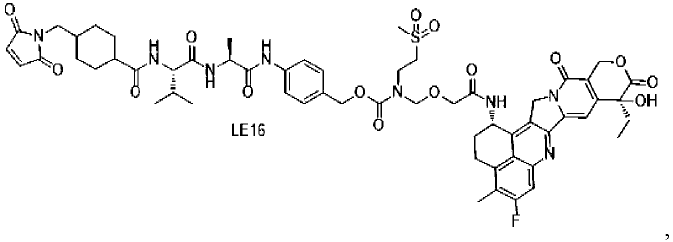

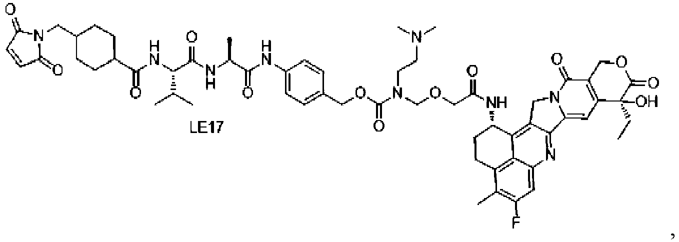

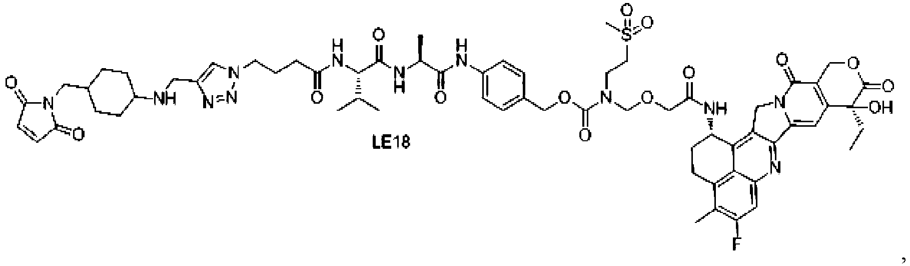

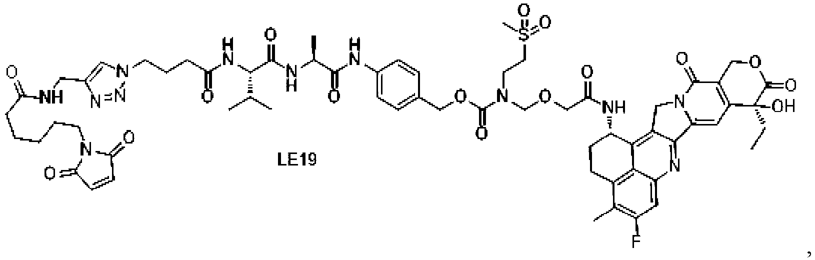

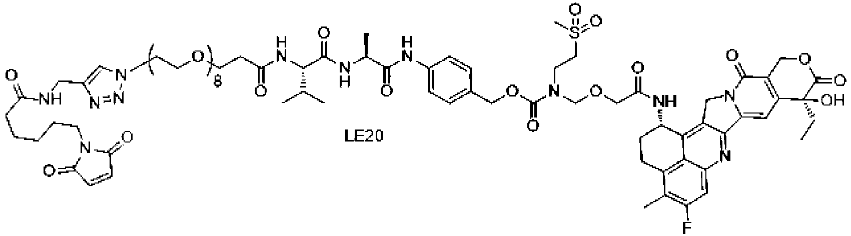

### Example 2-5: Synthesis of Compounds LE21 and LE22

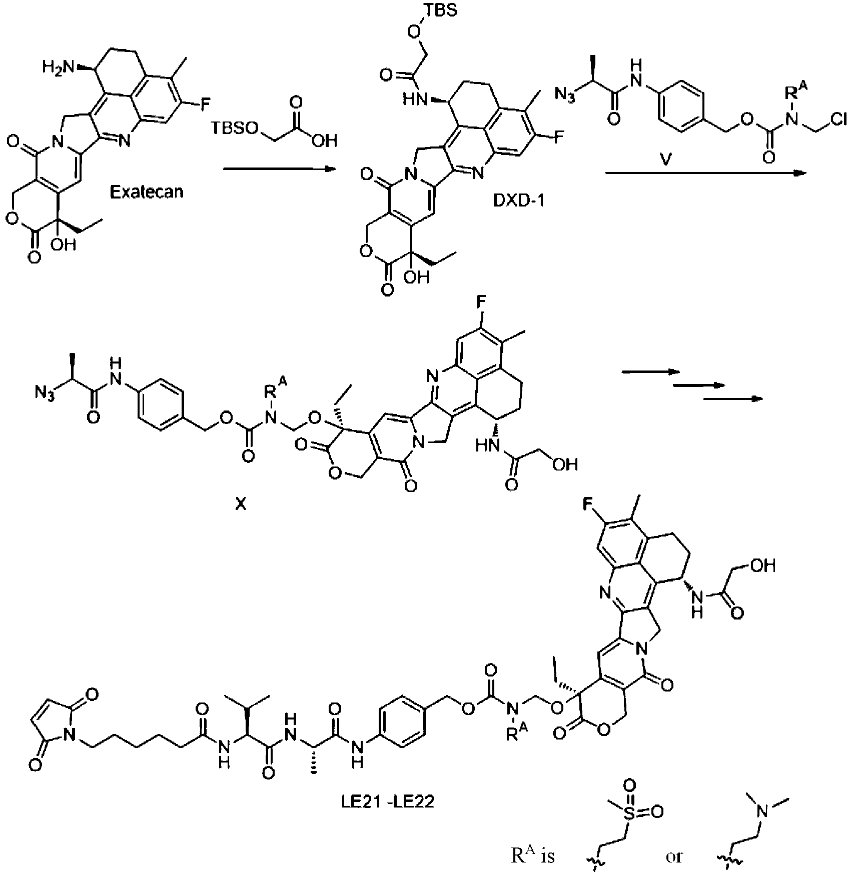

### Synthesis of Compound DXD-1

Commercially available Exatecan methanesulfonate (0.568 g, 1 mmol) was mixed with commercially available 2-(tert-butyldimethylsilyloxy)acetic acid (CAS: 105459-05-0, 0.38 g, 2 mmol) in 20 mL anhydrous dichloromethane. The condensation agent HATU (0.76 g, 2 mmol) and 1 mL of pyridine were added thereto and stirred at room temperature for 2 hours. After the reaction was completed, the solvent was evaporated to dryness under reduced pressure, and the obtained crude product was purified by column chromatography [dichloromethane: methanol = 50:1 (v/v)] to obtain title compound DXD-1 (0.55 g, yield of 90%), ESI-MS m/z: 608.1 (M+H). ¹H NMR (400 MHz, CDCl₃) δ 7.73 (d, J = 10.5 Hz, 1H), 7.64 (s, 1H), 7.05 (d, J = 9.2 Hz, 1H), 5.80 - 5.62 (m, 2H), 5.41 - 5.14 (m, 4H), 4.29 - 4.15 (m, 2H), 4.08-4.03 (m, 1H), 3.27 - 3.07 (m, 2H), 2.45 (s, 3H), 2.38 - 2.28 (m, 2H), 1.96 - 1.81 (m, 2H), 1.04 (t, J = 7.4 Hz, 3H), 0.80 (s, 9H), 0.11 (s, 3H), 0.03 (s, 3H).

### Preparation of Intermediate V

Intermediate V could be prepared by replacing the methylamine hydrochloride in step b with the corresponding commercially available amino compound, referring to the preparation method of compound 4 in Example 2-1.

### Synthesis of LE21 to LE22

Intermediate V was reacted with DXD-1, and then treated with 10% trifluoroacetic acid/dichloromethane solution to obtain intermediate X. Then, intermediate X was reacted according to the subsequent steps e, g, i, and j of compound 5 in Example 2-1: Intermediate X was reduced to obtain an amino compound, and the amino compound was then condensed with Fmoc-L-valine hydroxysuccinimide ester. Then the Fmoc protecting group of the amino group was removed from the obtained product, and the obtained amino product was then reacted with N-succinimidyl 6-maleimidohexanoate to obtain the final product. Compound LE21: yellow solid, ESI-MS m/z: 1141.2 (M+H); compound LE22: yellow solid, ESI-MS m/z: 1106.6 (M+H).

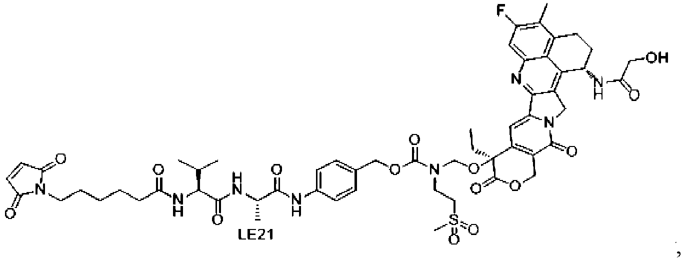

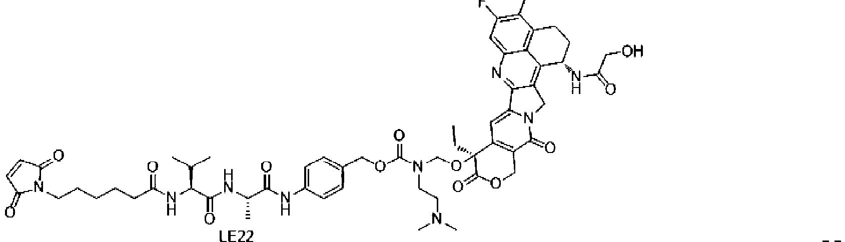

### Example 2-6: Synthesis of Compound LS13 (comparative example)

Referring to the synthesis method of LE15 in Examples 2 to 4, SN-38 (7-ethyl-10-hydroxycamptothecin) was reacted with intermediate VII (R¹ is methylsulfonyl ethyl) to obtain compound LS13 after deprotection, condensation and other steps: ¹H NMR (400 MHz, DMSO) δ 9.92 (d, *J* = 22.4 Hz, 1H), 8.14 (s, 1H), 8.08 (d, *J* = 9.1 Hz, 1H), 7.81 (d, *J* = 8.0 Hz, 1H), 7.70 - 7.50 (m, 3H), 7.47 (d, *J =* 7.2 Hz, 1H), 7.34 (d, *J* = 7.2 Hz, 1H), 7.27 (s, 1H), 7.20 (s, 1H), 6.98 (s, 2H), 6.51 (s, 1H), 5.61 (s, 2H), 5.48 - 5.35 (m, 2H), 5.27 (s, 2H), 5.10 (d, *J* = 20.6 Hz, 2H), 4.36 (s, 1H), 4.21 - 4.07 (m, 1H), 3.84 (s, 2H), 3.48 (s, 2H), 3.21 - 2.92 (m, 6H), 2.25 - 2.04 (m, 2H), 2.04 - 1.78 (m, 3H), 1.55 - 1.36 (m, 4H), 1.36 - 1.10 (m, 9H), 0.95 - 0.71 (m, 10H).

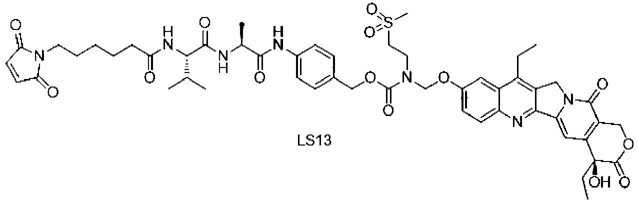

### Example 2-7: Synthesis of Compound GGFG-Dxd (comparative example)

Compound GGFG-Dxd was prepared according to the known synthesis method reported in WO2015146132A1. ESI-MS m/z: 1034.5 (M+H), ¹H-NMR (400 MHz, DMSO-*d₆*) δ 8.61 (t, *J* = 6.4 Hz, 1H), 8.50 (d, *J=* 8.5 Hz, 1H), 8.28 (t, *J =* 5.1 Hz, 1H), 8.11 (d, *J=* 7.5 Hz, 1H), 8.05 (t, *J =* 5.7 Hz, 1H), 7.99 (t, *J* = 5.9 Hz, 1H), 7.77 (d, *J* = 11.0 Hz, 1H), 7.31 (s, 1H), 7.25 - 7.16 (m, 5H), 6.98 (s, 2H), 6.51 (s, 1H), 5.59 (dt, *J* = 7.4, 4.1 Hz, 1H), 5.41 (s, 2H), 5.20 (s, 2H), 4.64 (d, *J* = 6.1 Hz, 2H), 4.53 - 4.40 (m, 1H), 4.02 (s, 2H), 3.74 - 3.37 (m, 8H), 3.18 - 3.00 (m, 2H), 3.04 - 2.97 (m, 1H), 2.77 (dd, *J* = 13.5, 9.4 Hz, 1H), 2.38 (s, 3H), 2.19 (dd, *J* = 14.9, 8.5 Hz, 2H), 2.11 - 2.05 (m, 2H), 1.86 (dd, *J* = 14.0, 6.7 Hz, 2H), 1.45 (s, 4H), 1.20 - 1.14 (m, 2H), 0.87 (t, *J* = 7.1 Hz, 3H).

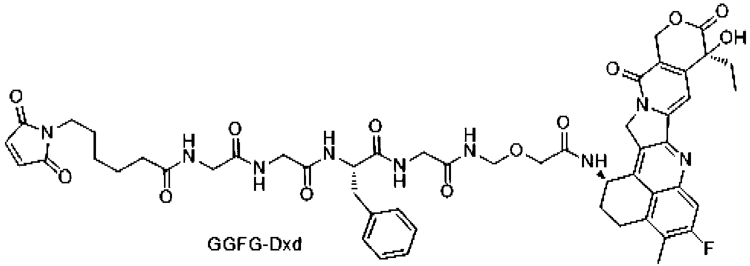

### Example 3: Preparation of Antibody-Drug Conjugates

The antibody FDA018 against TROP2 was prepared according to the method of Example 1 and was replaced into 50 mM PB/1.0 mM EDTA buffer (pH 7.0) using a G25 desalting column. 5 equivalents of TECP were added thereto and the mixture was stirred at 25°C for 1 hours to fully open the disulfide bonds between the antibody chains. Then, citric acid was used to adjust the pH of the reduced antibody solution to 5.0, and the sample was replaced with 20 mM citrate buffer and 1 mM EDTA buffer (pH 5.0) using a G25 desalting column. The temperature of the water bath was maintained at 25°C for coupling reaction. The linker-drug conjugates LE12 to LE22, LS13, and GGFG-Dxd prepared according to the above Example 2 were dissolved in DMSO respectively and 4.5 equivalents of linker-drug conjugate were added dropwise to the reduced antibody solution. Additional DMSO was added to a final concentration of 5% (v/v) and the reaction was stirred at 25°C for 0.5 hours. After the reaction was completed, the sample was filtered through a 0.22 µm membrane. The tangential flow filtration system was used to purify and remove unconjugated small molecules. The buffer was a 25 mM His, 6% sucrose solution (pH 6.0). After purification, the sample was stored in a -20°C refrigerator. The absorbance values were measured at 280 nm and 370 nm by UV method, respectively, and the DAR value was calculated. The results are shown in Table 2 below.

The coupling reaction was carried out in the same manner as in this example and all samples were prepared according to the highest DAR (i.e., excessive coupling). The occurrence of precipitation during each coupling reaction was observed and the polymer ratio and recovery rate after each coupling reaction were calculated. The results are also shown in Table 2.

**Table 2 Coupling conditions for preparing different antibody-drug conjugates (ADCs)**

| ADC Number | Antibody | Linker-Drug Conjugate | DAR Value | Whether Precipitation | Aggregation Ratio | Recovery Rate |
|---|---|---|---|---|---|---|
| FDA018-LE12 | FDA018 | LE12 | 3.90 | No | 0.5% | 89% |
| FDA018-LE13 | FDA018 | LE13 | 4.10 | No | 0.2% | 98% |
| FDA018-LE14 | FDA018 | LE14 | 4.00 | No | 0.1% | 93% |
| FDA018-LE15 | FDA018 | LE15 | 4.12 | No | 0.4% | 91% |
| FDA018-LE16 | FDA018 | LE16 | 4.03 | No | 0.3% | 87% |
| FDA018-LE17 | FDA018 | LE17 | 3.98 | No | 0.3% | 88% |
| FDA018-LE18 | FDA018 | LE18 | 3.96 | No | 0.5% | 92% |
| FDA018-LE19 | FDA018 | LE19 | 4.13 | No | 0.3% | 91% |
| FDA018-LE20 | FDA018 | LE20 | 3.88 | No | 0.1% | 92% |
| FDA018-LE21 | FDA018 | LE21 | 4.05 | No | 0.4% | 91% |
| FDA018-LE22 | FDA018 | LE22 | 4.00 | No | 0.1% | 92% |
| FDA018-LS13 | FDA018 | LS13 | 3.97 | No | 0.6% | 89% |
| FDA018-GGFG-Dxd | FDA018 | GGFG-Dxd | 4.03 | No | 0.2% | 92% |

| | | | | | | |
|---|---|---|---|---|---|---|
| "/" indicates that the recovery rate is not calculated | | | | | | |

No precipitation was produced in the preparation procedure of the antibody-drug conjugate of the present disclosure, and the aggregation ratio was within the normal range, indicating that the linker-drug conjugates provided by the present disclosure have good physicochemical properties.

### Effect Example 1: In Vitro Killing Activity Evaluation of Antibody-Drug Conjugates

NCI-N87 (ATCC) cells were selected as the cell line for in vitro activity detection. 2000 cells per well were seeded in a 96-well cell culture plate and cultured for 20 to 24 hours. The antibody-drug conjugates prepared according to the method of Example 3 were formulated into test solutions with 11 concentration gradients of 1000, 166.7, 55.6, 18.6, 6.17, 2.06, 0.69, 0.23, 0.08, 0.008, and 0 nM using L15 cell culture medium containing 10% FBS. The diluted test solutions were added to the culture plate containing the seeded cells at 100 µL/well and incubated for 144 hours at 37°C in a 5% CO₂ incubator. CellTiter-Glo^{®} Luminescent Cell Viability Assay Reagent (50 µL/well) was added and the plate was shaken at 500 rpm at room temperature for 10 minutes to mix well. The data were read using a SpectraMaxL microplate reader (OD 570 nm, reading at 2 s intervals) and the IC50 results were calculated as shown in Table 3.

Using the same method as above, the cytotoxic activity of each antibody-drug conjugate against MDA-MB-468 and BXPC3 tumor cells purchased from ATCC was tested. The results are shown in Table 3. From the results in Table 3, it can be seen that the antibody-drug conjugates provided by the present disclosure have excellent in vitro killing activity against cells such as NCI-N87, MDA-MB-468, and BXPC3. However, the antibody-drug conjugate has no killing activity on Calu-6 negative cells, indicating that the prepared ADC has specific targeted killing activity.

**Table 3: In vitro killing activity of antibody-drug conjugates**

| ADC Number | IC50 (nM) | | | |
|---|---|---|---|---|
| | MDA-MB-468 Cell | NCI-N87 Cell | BXPC3 Cell | Calu-6 Cell |
| FDA018-LE12 | 1.03 | 1.12 | 0.68 | >1000 |
| FDA018-LE13 | 2.0 | 2.34 | 1.23 | >1000 |
| FDA018-LE14 | 0.8 | 1.08 | 0.50 | >1000 |
| FDA018-LE15 | 0.93 | 0.98 | 0.96 | >1000 |
| FDA018-LE16 | 1.64 | 1.35 | 1.58 | >1000 |
| FDA018-LE17 | 0.82 | 1.12 | 0.62 | >1000 |
| FDA018-LE18 | 0.86 | Not Tested | Not Tested | >1000 |
| FDA018-LE19 | 1.03 | Not Tested | Not Tested | >1000 |
| FDA018-LE20 | Not Tested | 1.15 | Not Tested | >1000 |
| FDA018-LE21 | Not Tested | 1.23 | 1.23 | >1000 |
| FDA018-LE22 | Not Tested | 3.25 | 1.69 | >1000 |
| FDA018-LS13 | 67.38 | Not Tested | 61.32 | >1000 |
| FDA018-GGFG-DXD | 1.2 | 1.10 | 0.61 | >1000 |

### Effect Example 2: In Vitro Plasma Stability Assay

This example evaluates the stability of the antibody-drug conjugate prepared according to the method of Example 3 in human plasma. Specifically, in this example, the antibody-drug conjugate of Example 3 was added to human plasma and placed in a 37°C water bath for 1, 3, 7, 14, 21, and 28 days. An internal standard (Exatecan as an internal standard substance) was added and extracted and then detected by high-performance liquid chromatography to detect the release of free drugs. The results are shown in Table 4.

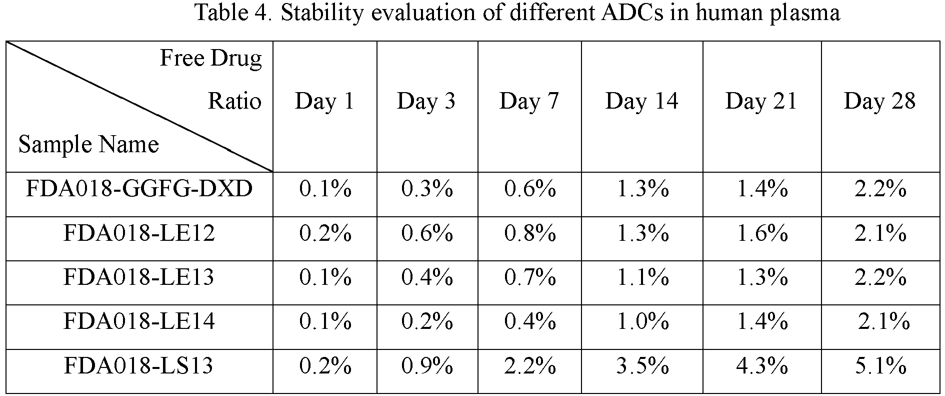

The plasma stability results show that the stability of the ADC obtained using the new technical solution is not inferior to FDA018-GGFG-DXD, and some are even better. At the same time, the above activity test results also prove that some of the newly obtained ADCs have better activity than FDA018-GGFG-DXD.

### Effect Example 3: In Vitro Enzyme Digestion Experiment of Linker-Drug Conjugates

The linker-drug conjugate (LE14 and GGFG-Dxd) was co-incubated with cathepsin B in three different pH (5.0, 6.0, 7.0) buffers. Samples were taken at different time points and entered into a high-performance liquid chromatography-mass spectrometry instrument. The external standard method (with DXD as the external standard) was used to determine the release percentage of the drug. The experimental results (as shown in Table 5) show that GGFG-Dxd has a slow speed of enzyme digestion within the pH range used, while LE14 of the present disclosure can be quickly enzymatically digested within the pH range of 5.0 to 7.0.

**Table 5. In vitro enzyme digestion of LE14 and GGFG-Dxd at different pH**

| Time (h) | Percentage of drug release in the sample % | | | | | |
|---|---|---|---|---|---|---|
| | GGFG-Dxd | | | LE14 | | |
| | pH 5.0 | pH 6.0 | pH 7.0 | pH 5.0 | pH 6.0 | pH 7.0 |
| 0 | 22.0 | 22.35 | 22.16 | 13.82 | 14.32 | 16.59 |
| 1 | 24.82 | 24.8 | 25.76 | 96.0 | 96.12 | 98.32 |
| 2 | 25.85 | 27.32 | 29.45 | 98.35 | 96.75 | 98.45 |
| 3 | 27.46 | 29.32 | 32.00 | 99.12 | 98.52 | 99.12 |
| 4 | 29.68 | 32.0 | 34.72 | 99.21 | 98.45 | 99.12 |
| 5 | 31.72 | 33.15 | 37.17 | 99.45 | 98.92 | 99.87 |
| 6 | 34.17 | 36.38 | 38.43 | 98.23 | 99.15 | 99.47 |

### Effect Example 4: In Vitro Enzyme Digestion Experiment of FDA018-LS13 (comparative example)

NCI-N87 cell line was selected as the experimental cell lines. After the sample was incubated in cathepsin B system (100 mM sodium acetate-acetic acid buffer, 4 mM dithiothreitol, pH 5.0) at 37°C for 4 hours, the obtained sample was diluted with culture medium to different concentrations. 8 concentrations (1.5 to 10-fold dilution) were set from 70 nM to 0.003 nM of SN-38 concentration. The killing (inhibitory) ability of the cell line was observed for 144 hours. The IC50 value was calculated by reading the fluorescence data after chemical luminescent staining with CellTiter-Glo^{®} Luminescent Cell Viability Assay.

The above enzyme digestion samples obtained by incubating in a cathepsin B system at 37°C for 4 hours were precipitated with an appropriate amount of ethanol to remove protein and detected by high-performance liquid chromatography to release small molecule compounds. The 4-hour release rate was measured with an equal amount of SN-38 as a reference, and the results showed that the release rate reached 99%.

The experimental results (as shown in Table 6) show that after enzyme digestion treatment, the cytotoxic activity of FDA018-LS13 is almost the same as that of SN-38 at an equivalent dose, which also indicates that FDA018-LS13 has almost completely released SN-38 under the action of cathepsin B and played a role. However, FDA018-LS13 may have undergone unpredictable changes when it is endocytosed into lysosomes, resulting in SN-38 not being able to function effectively.

**Table 6. Changes in killing activity of FDA018-LS13 on NCI-N87 cell line before and after enzyme digestion by cathepsin B system**

| | IC50 (based on SN-38 equivalent, nM) | |
|---|---|---|
| Sample | Before Enzyme Digestion | After Enzyme Digestion |
| FDA018-LS13 | > 100 nM | 6.48 nM |
| SN-38 | 7.18 nM | 7.43 nM |

### Effect Example 5: Testing Antitumor Activity of FDA018-LE14 in NCI-N87 Human Gastric Cancer Model

6 to 8 week old female Balb/c nude mice were subcutaneously injected with 5×10⁶ human gastric cancer cells (NCI-N87) dissolved in 100 µL of PBS solution on the right side of the neck and back. When the tumor grew to an average volume of 150 to 200 mm³, mice were randomly divided into 5 groups according to tumor size and mouse weight, with 6 animals in each group. The groups were a blank control group, and two dose groups of the antibody-drug conjugates FDA018-GGFG-DXD and FDA018-LE14, respectively. Specifically, group 01 was a blank control group; group 02 was FDA018-GGFG-DXD group at 4.0 mg/kg; group 03 was a FDA018-GGFG-DXD group at 2.0 mg/kg; group 04 was a FDA018-LE14 group at 4.0 mg/kg; group 05 was FDA018-LE14 group at 2.0 mg/kg; administered intraperitoneally once a week. The animal weight and tumor volume were measured twice a week, and the survival status of the experimental animals was observed during the experiment process. As shown in Table 7, the average tumor volume of the mice in the blank control group was 1388.47 mm³ at the end of treatment. The average tumor volume of the FDA018-GGFG-DXD treatment group at 2.0 mg/kg was 1235.21 mm³ on the 14th day after the end of treatment, and the average tumor volume of the FDA018-GGFG-DXD treatment group at 4.0 mg/kg was 721.09 mm³ on the 14th day after the end of treatment. The average tumor volume of the FDA018-LE14 treatment group at 2.0 mg/kg was 1342.31 mm³ on the 14th day after the end of treatment, and the average tumor volume of the FDA018-LE14 treatment group at 4.0 mg/kg was 435.36 mm³ on the 14th day after the end of treatment. The experimental results show that FDA018-LE14 has good in vivo antitumor activity, and all experimental mice have no death or weight loss, indicating that FDA018-LE14 has good safety.

**Table 7. Antitumor activity of FDA018-LE14 in NCI-N87 human gastric cancer model**

| **Group** | **Observation Days** | | | | | | | | | | | |
|---|---|---|---|---|---|---|---|---|---|---|---|---|
| | **5** | **7** | **11** | **14** | **18** | **21** | **25** | **28** | **32** | **35** | **39** | **42** |
| Average Tumor Volume/mm³ | | | | | | | | | | | | |
| **01** | 205.31 ±11.93 | 283.81 ±26.98 | 395.50 ±38.06 | 489.36 ±34.44 | 621.74 ±32.84 | 721.41 ±19.24 | 783.85 ±21.21 | 890.79 ±35.51 | 994.80 ±47.44 | 1176.92 ±75.40 | 1348. 83±7 2.37 | 1388. 47±7 1.37 |
| **02** | 205.35 ±11.17 | 260.96 ±23.58 | 235.65 ±35.14 | 202.27 ±36.43 | 250.53 ±47.43 | 341.51 ±59.92 | 363.76 ±56.95 | 412.44 ±73.14 | 479.74 ±88.12 | 527.30± 90.84 | 655.9 6±13 5.74 | 721.0 9±14 9.38 |
| **03** | 205.54 ±11.39 | 315.13 ±29.11 | 332.46 ±42.69 | 284.41 ±50.34 | 419.84 ±85.78 | 489.93 ±88.24 | 529.24 ±100.8 4 | 628.75 ±111.5 8 | 725.02 ±131.4 3 | 892.29± 180.36 | 1065. 85±1 96.79 | 1235. 21±2 91.76 |
| **04** | 205.32 ±10.90 | 339.51 ±32.35 | 268.58 ±33.83 | 192.55 ±36.69 | 191.16 ±47.20 | 217.61 ±72.84 | 250.78 ±83.71 | 273.13 ±87.46 | 324.77 ±101.8 4 | 368.20± 113.68 | 426.1 2±14 2.41 | 435.3 6±14 9.14 |
| **05** | 205.32 ±10.9 | 296.64 ±32.35 | 320.03 ±33.83 | 307.45 ±34.59 | 386.87 ±44.27 | 510.03 ±43.55 | 595.45 ±68.98 | 699.04 ±57.29 | 809.63 ±87.10 | 922.72± 89.34 | 1192. 74±1 36.02 | 1342. 31±1 73.91 |

## Claims

1. An antibody-drug conjugate of formula **I,** a pharmaceutically acceptable salt thereof, a solvate thereof, or a solvate of the pharmaceutically acceptable salt thereof; 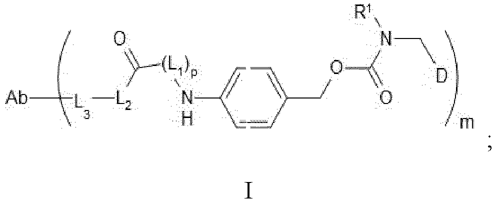
wherein Ab is a TROP2 antibody or a variant of the TROP2 antibody;
the amino acid sequence of the light chain in the TROP2 antibody is shown in SEQ ID NO: 1, and the amino acid sequence of the heavy chain is shown in SEQ ID NO: 2;
the variant of the TROP2 antibody is at least 70%, 75%, 80%, 85%, 90%, 95%, 98%, or 99% identical to the TROP2 antibody;
m is 3.5 to 4.5;
D is a cytotoxic drug topoisomerase inhibitor, wherein the cytotoxic drug topoisomerase inhibitor is 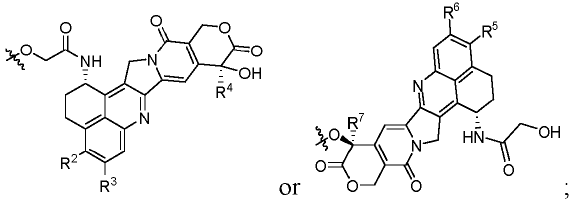
R² and R⁵ are each independently H, C₁-C₆ alkyl, or halogen; R³ and R⁶ are each independently H, C₁-C₆ alkyl, or halogen; R⁴ and R⁷ are each independently C₁-C₆ alkyl;
R¹ is C₁-C₆ alkyl substituted by one or more than one -NR¹⁻¹R¹⁻², C₁-C₆ alkyl substituted by one or more than one R¹⁻³S(O)₂-, C₁-C₆ alkyl, C₃-C₁₀ cycloalkyl, C₆-C₁₄ aryl, or 5- to 14-membered heteroaryl; the heteroatom in the 5- to 14-membered heteroaryl is selected from one or more than one of N, O, and S, and the number of heteroatoms is 1, 2, 3, or 4; the R¹⁻¹, R¹⁻², and R¹⁻³ are each independently C₁-C₆ alkyl;
L₁ is independently one or more than one of a phenylalanine residue, alanine residue, glycine residue, aspartic acid residue, cysteine residue, glutamic acid residue, histidine residue, isoleucine residue, leucine residue, lysine residue, methionine residue, proline residue, serine residue, threonine residue, tryptophan residue, tyrosine residue, and valine residue; p is 2 to 4;
L₂ is 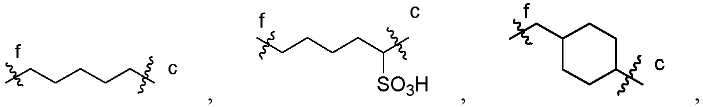 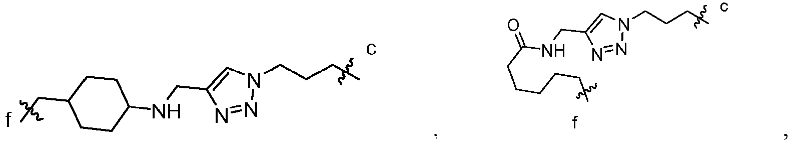 or 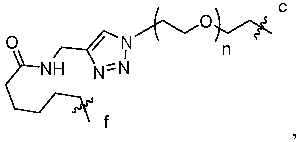 wherein n is independently 1 to 12, the c-terminal is connected to L₁ through a carbonyl group, and the f-terminal is connected to the d-terminal of L₃;
L₃ is 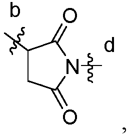 wherein the b-terminal is connected to the Ab, and the d-terminal is connected to the f-terminal of L₂.

2. The antibody-drug conjugate, the pharmaceutically acceptable salt thereof, the solvate thereof, or the solvate of the pharmaceutically acceptable salt thereof according to claim 1, wherein
the b-terminal of L₃ is connected to the sulfhydryl group on the antibody in the form of a thioether;
and/or, when R¹ is C₁-C₆ alkyl substituted by one or more than one -NR¹⁻¹R¹⁻², the C₁-C₆ alkyl is methyl, ethyl, n-propyl, isopropyl, n-butyl, isobutyl, or tert-butyl, preferably ethyl;
and/or, when R¹ is C₁-C₆ alkyl substituted by more than one -NR¹⁻¹R¹⁻², the "more than one" is two or three;
and/or, when R¹⁻¹ and R¹⁻² are each independently C₁-C₆ alkyl, the C₁-C₆ alkyl is methyl, ethyl, n-propyl, isopropyl, n-butyl, isobutyl, or tert-butyl, preferably methyl;
and/or, when R¹ is C₁-C₆ alkyl substituted by one or more than one R¹⁻³S(O)₂-, the C₁-C₆ alkyl is C₁-C₄ alkyl, preferably methyl, ethyl, n-propyl, isopropyl, n-butyl, isobutyl, or tert-butyl, most preferably ethyl;
and/or, when R¹ is C₁-C₆ alkyl substituted by more than one R¹⁻³S(O)₂-, the "more than one" is two or three;
and/or, when R¹⁻³ is C₁-C₆ alkyl, the C₁-C₆ alkyl is methyl, ethyl, n-propyl, isopropyl, n-butyl, isobutyl, or tert-butyl, preferably methyl;
and/or, when R¹ is C₁-C₆ alkyl, the C₁-C₆ alkyl is methyl, ethyl, n-propyl, isopropyl, n-butyl, isobutyl, or tert-butyl, preferably methyl;
and/or, n is 8 to 12;
and/or, p is 2;
and/or, when R¹⁻¹, R¹⁻², and R¹⁻³ are independently C₁-C₆ alkyl, the C₁-C₆ alkyl is C₁-C₄ alkyl, preferably methyl, ethyl, n-propyl, isopropyl, n-butyl, isobutyl, or tert-butyl, most preferably methyl.

3. The antibody-drug conjugate, the pharmaceutically acceptable salt thereof, the solvate thereof, or the solvate of the pharmaceutically acceptable salt thereof according to claim 1, wherein
when R² and R⁵ are each independently C₁-C₆ alkyl, the C₁-C₆ alkyl is C₁-C₄ alkyl, further preferably methyl, ethyl, n-propyl, isopropyl, n-butyl, isobutyl, or tert-butyl, most preferably methyl;
and/or, when R² and R⁵ are each independently halogen, the halogen is fluorine, chlorine, bromine, or iodine, preferably fluorine;
and/or, when R³ and R⁶ are each independently C₁-C₆ alkyl, the C₁-C₆ alkyl is C₁-C₄ alkyl, further preferably methyl, ethyl, n-propyl, isopropyl, n-butyl, isobutyl, or tert-butyl, most preferably methyl;
and/or, when R³ and R⁶ are each independently halogen, the halogen is fluorine, chlorine, bromine, or iodine, further preferably fluorine;
and/or, when R⁴ and R⁷ are each independently C₁-C₆ alkyl, the C₁-C₆ alkyl is C₁-C₄ alkyl, further preferably methyl, ethyl, n-propyl, isopropyl, n-butyl, isobutyl, or tert-butyl, most preferably ethyl;
and/or, when R¹ is C₁-C₆ alkyl substituted by one or more than one -NR¹⁻¹R¹⁻², the -NR¹⁻¹R¹⁻² is -N(CH₃)₂;
and/or, when R¹ is C₁-C₆ alkyl substituted by one -NR¹⁻¹R¹⁻², the C₁-C₆ alkyl substituted by one -NR¹⁻¹R¹⁻² is 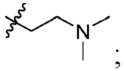
and/or, when R¹ is C₁-C₆ alkyl substituted by one R¹⁻³S(O)₂-, the C₁-C₆ alkyl substituted by one R¹⁻³S(O)₂- is 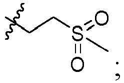
and/or, m is 3.8 to 4.2, such as 3.88, 3.90, 3.96, 3.97, 3.98, 4.00, 4.03, 4.05, 4.10, 4.12, or 4.13;
and/or, (L₁)ₚ is 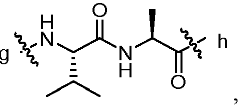 wherein the g-terminal is connected to the c-terminal of L₂ through a carbonyl group;
and/or, n is 8, 9, 10, 11, or 12.

4. The antibody-drug conjugate, the pharmaceutically acceptable salt thereof, the solvate thereof, or the solvate of the pharmaceutically acceptable salt thereof according to claim 1, wherein
R² and R⁵ are each independently C₁-C₆ alkyl;
and/or, R³ and R⁶ are each independently halogen;
and/or, R⁴ and R⁷ are ethyl;
and/or, L₁ is one or more than one of the phenylalanine residue, alanine residue, glycine residue, isoleucine residue, leucine residue, proline residue, and valine residue; preferably one or more than one of the phenylalanine residue, alanine residue, glycine residue, and valine residue; further preferably the valine residue and/or the alanine residue, and the "more than one" is preferably two or three;
and/or, R¹ is C₁-C₆ alkyl substituted by one or more than one -NR¹⁻¹R¹⁻², C₁-C₆ alkyl substituted by one or more than one R¹⁻³S(O)₂-, C₁-C₆ alkyl, or C₃-C₁₀ cycloalkyl, preferably C₁-C₆ alkyl substituted by one or more than one -NR¹⁻¹R¹⁻², C₁-C₆ alkyl substituted by one or more than one R¹⁻³S(O)₂-, or C₁-C₆ alkyl, further preferably C₁-C₆ alkyl substituted by one or more than one -NR¹⁻¹R¹⁻², or C₁-C₆ alkyl substituted by one or more than one R¹⁻³S(O)₂-, most preferably C₁-C₆ alkyl substituted by one or more than one R¹⁻³S(O)₂-;
preferably, D is

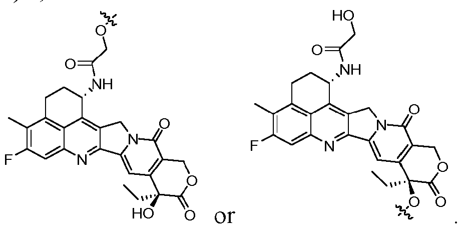

5. The antibody-drug conjugate, the pharmaceutically acceptable salt thereof, the solvate thereof, or the solvate of the pharmaceutically acceptable salt thereof according to any one of claims 1 to 4, wherein the antibody-drug conjugate is any one of the following schemes:
scheme I:
Ab is the TROP2 antibody or the variant of the TROP2 antibody;
D is 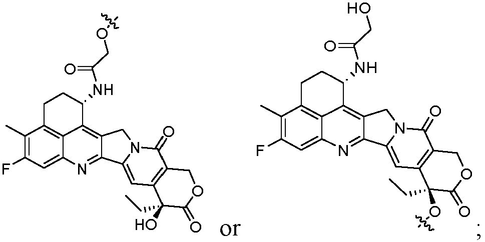
L₁ is independently one or more than one of the phenylalanine residue, alanine residue, glycine residue, isoleucine residue, leucine residue, proline residue, and valine residue;
scheme II:
Ab is the TROP2 antibody or the variant of the TROP2 antibody;
D is 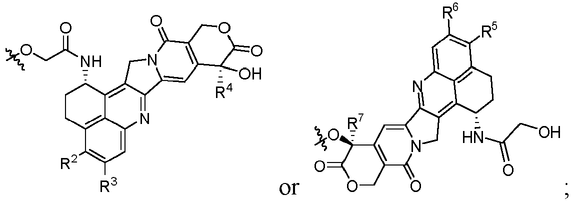 wherein R² and R⁵ are each independently C₁-C₆ alkyl; R³ and R⁶ are each independently halogen; D is preferably 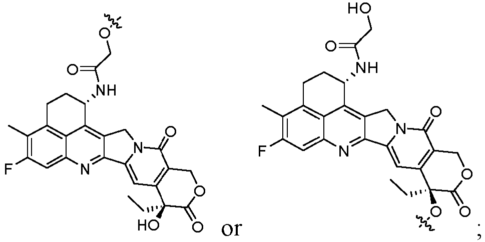
R¹ is C₁-C₆ alkyl substituted by one or more than one -NR¹⁻¹R¹⁻², C₁-C₆ alkyl substituted by one or more than one R¹⁻³S(O)₂-, or C₁-C₆ alkyl;
L₁ is independently one or more than one of the phenylalanine residue, alanine residue, glycine residue, and valine residue;
scheme III:
Ab is the TROP2 antibody;
D is 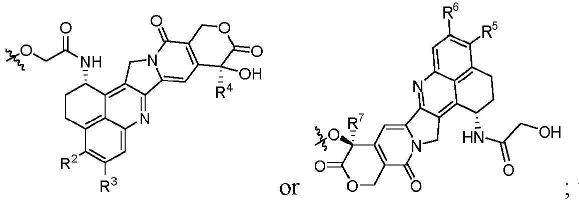 wherein R² and R⁵ are each independently C₁-C₆ alkyl; R³ and R⁶ are each independently halogen; D is preferably 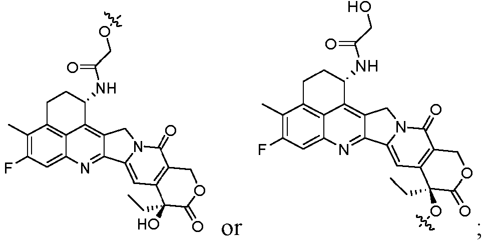
R¹ is C₁-C₆ alkyl substituted by one or more than one -NR¹⁻¹R¹⁻², C₁-C₆ alkyl substituted by one or more than one R¹⁻³S(O)₂-, or C₁-C₆ alkyl;
L₁ is independently the valine residue and/or the alanine residue;
scheme IV:
Ab is the TROP2 antibody;
D is 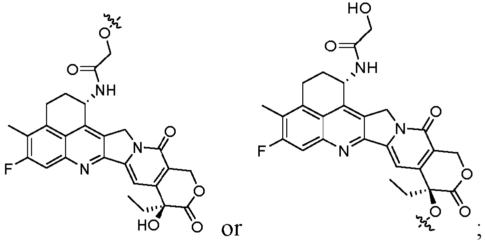
R¹ is C₁-C₆ alkyl substituted by one or more than one -NR¹⁻¹R¹⁻², C₁-C₆ alkyl substituted by one or more than one R¹⁻³S(O)₂-, or C₁-C₆ alkyl;
L₁ is independently the valine residue and/or the alanine residue;
scheme V:
Ab is the TROP2 antibody;
D is 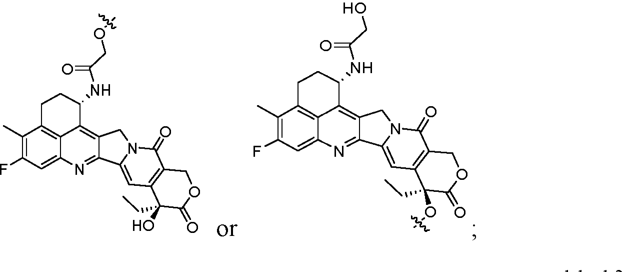
R¹ is C₁-C₆ alkyl substituted by one or more than one -NR¹⁻¹R¹⁻², C₁-C₆ alkyl substituted by one or more than one R¹⁻³S(O)₂-, or C₁-C₆ alkyl;
L₁ is independently the valine residue and/or the alanine residue.

6. The antibody-drug conjugate, the pharmaceutically acceptable salt thereof, the solvate thereof, or the solvate of the pharmaceutically acceptable salt thereof according to claim 1, wherein the antibody-drug conjugate is any one of the following compounds: 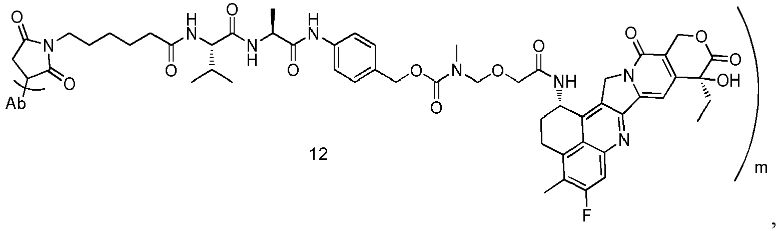 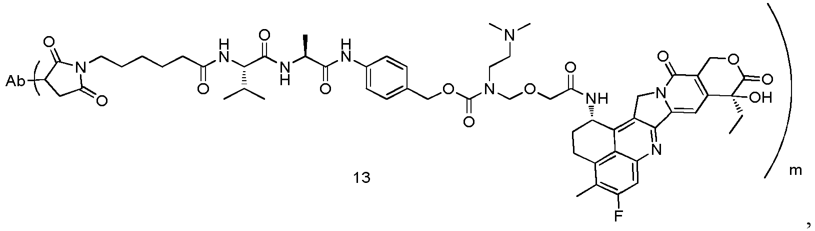 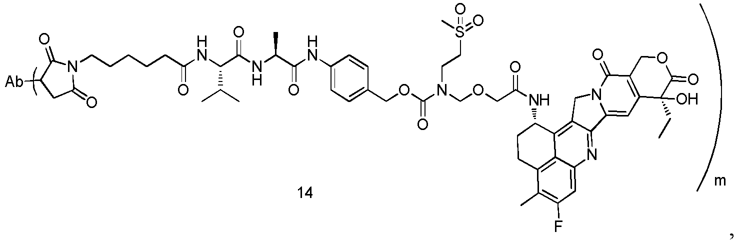 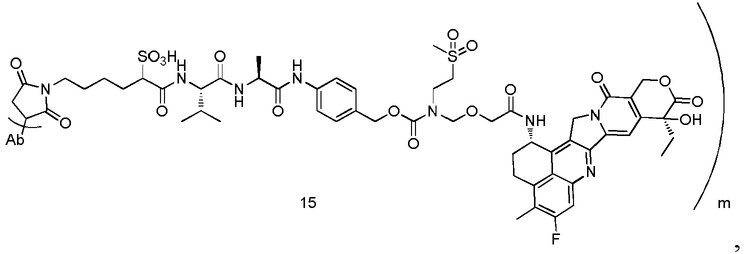 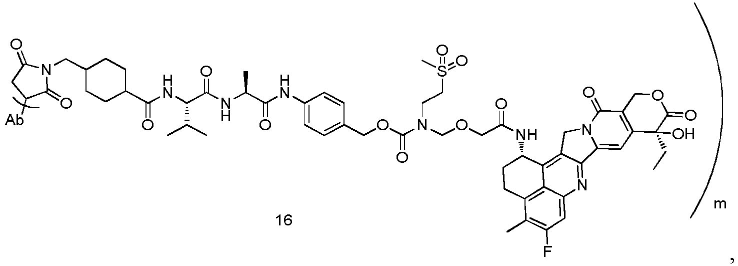 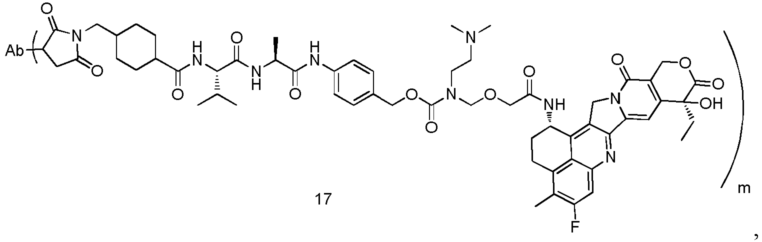 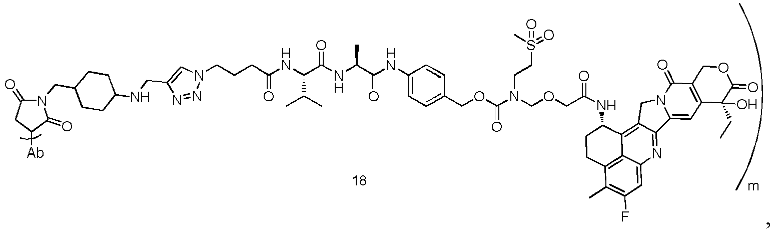 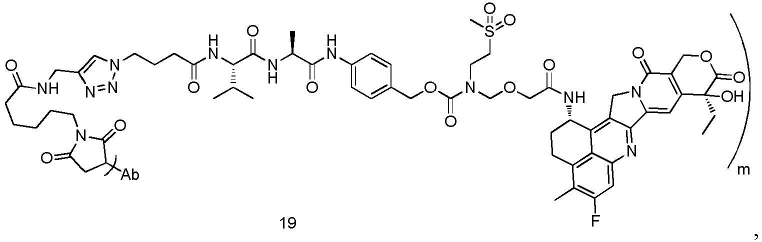 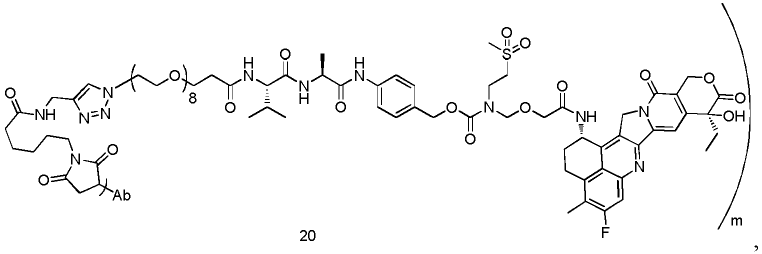 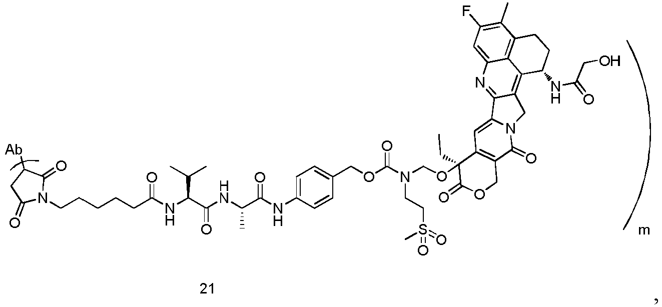 or 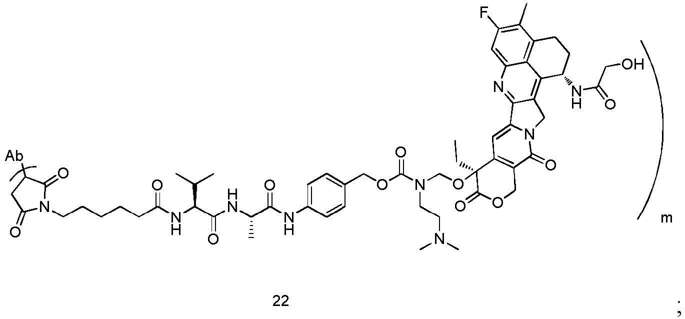 wherein Ab is the TROP2 antibody or the variant of the TROP2 antibody, and m is 3.8 to 4.2, preferably 3.88, 3.90, 3.96, 3.97, 3.98, 4.00, 4.03, 4.05, 4.10, 4.12, or 4.13; the amino acid sequence of the light chain in the variant of the TROP2 antibody is preferably shown in SEQ ID NO: 1, and the amino acid sequence of the heavy chain is preferably shown in SEQ ID NO: 2.

7. The antibody-drug conjugate, the pharmaceutically acceptable salt thereof, the solvate thereof, or the solvate of the pharmaceutically acceptable salt thereof according to claim 1, wherein the antibody-drug conjugate is any one of the following schemes:
scheme A: 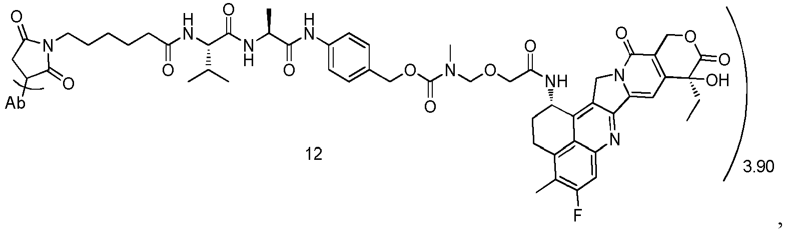 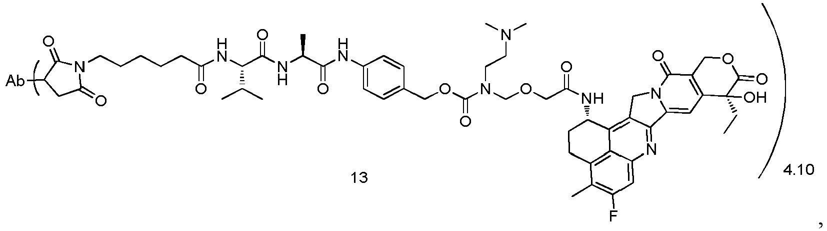 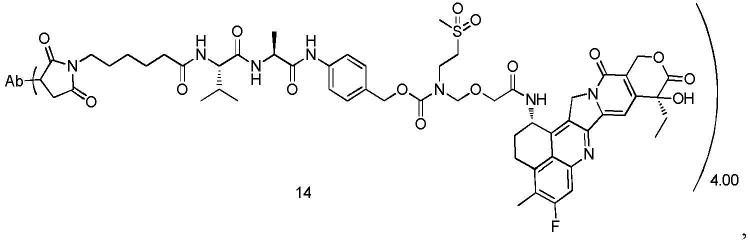 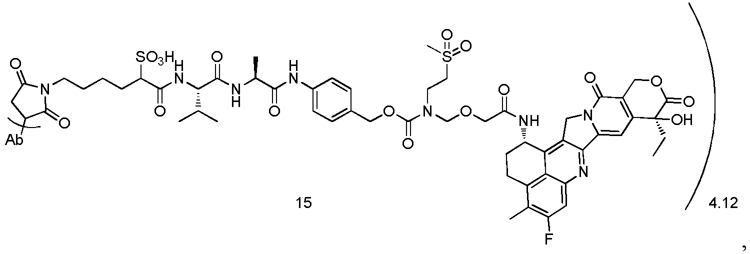 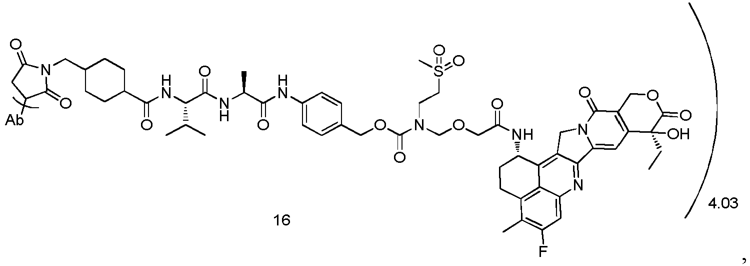 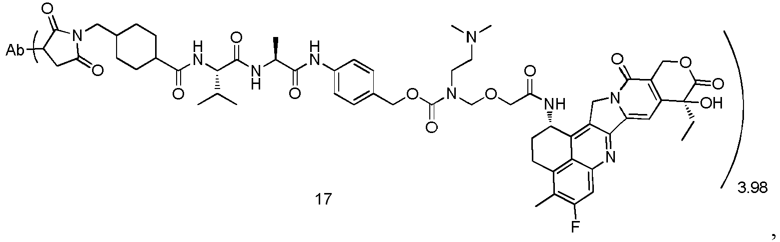 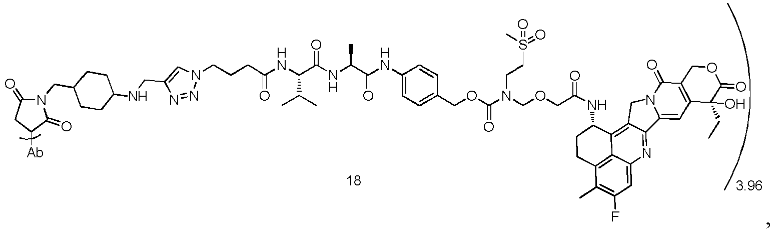 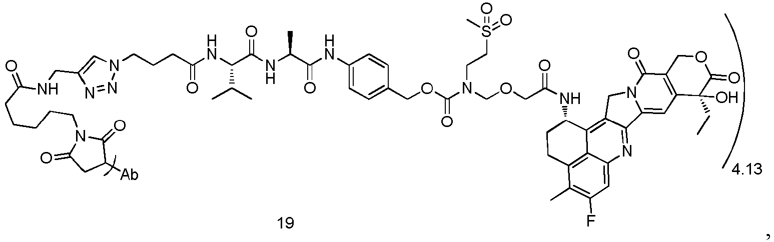 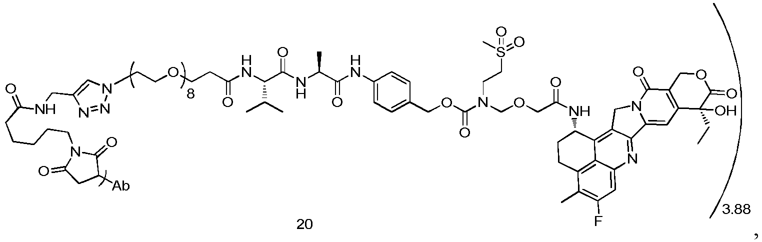 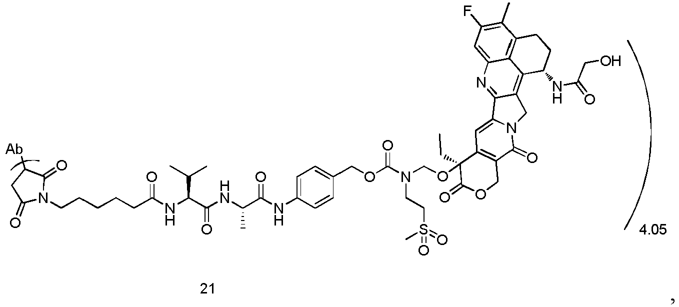 or 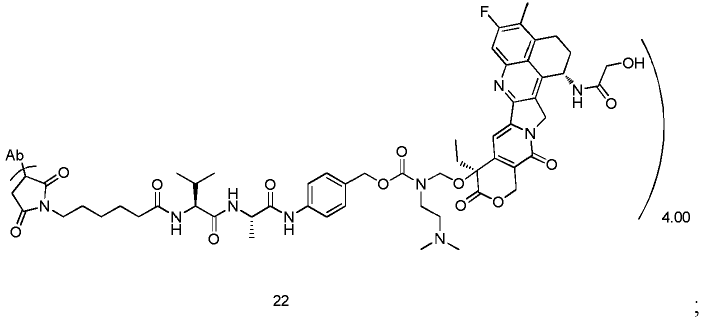
wherein Ab is the TROP2 antibody or the variant of the TROP2 antibody; the amino acid sequence of the light chain in the variant of the TROP2 antibody is shown in SEQ ID NO: 1, and the amino acid sequence of the heavy chain is shown in SEQ ID NO: 2;
scheme B: 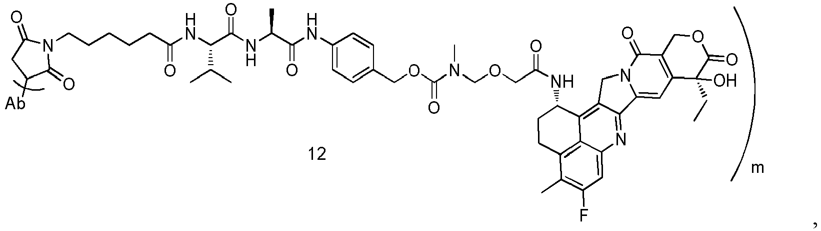 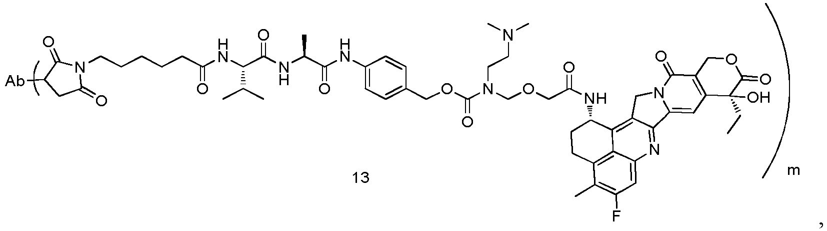 or 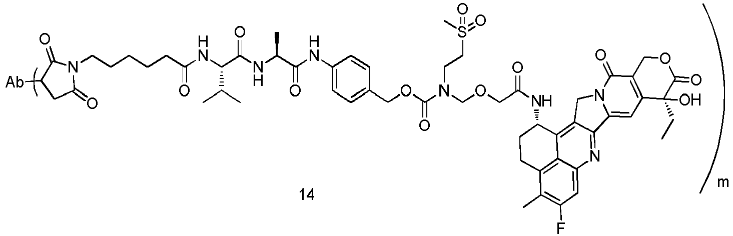
wherein Ab is the TROP2 antibody or the variant of the TROP2 antibody; m is 3.90, 4.00, or 4.10; the amino acid sequence of the light chain in the variant of the TROP2 antibody is shown in SEQ ID NO: 1, and the amino acid sequence of the heavy chain is shown in SEQ ID NO: 2;
scheme C: 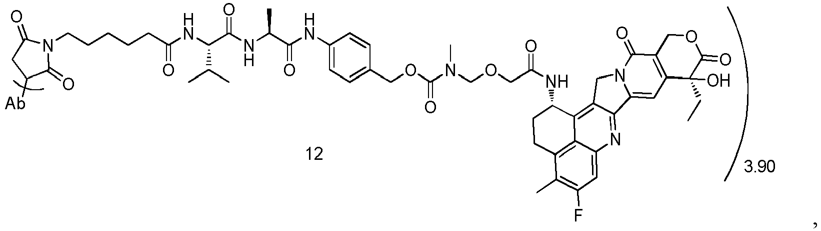 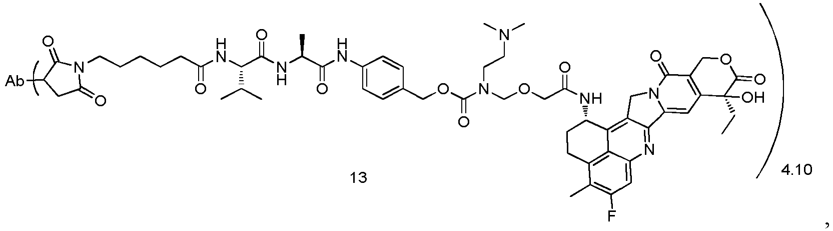 or 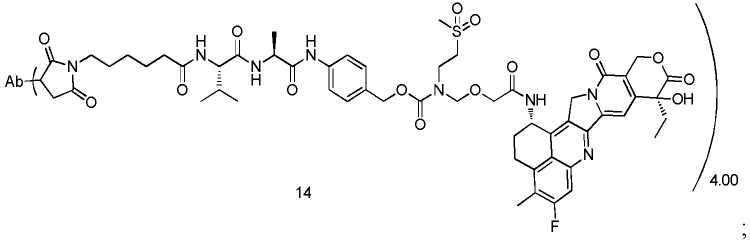
wherein Ab is the TROP2 antibody or the variant of the TROP2 antibody;
scheme D: 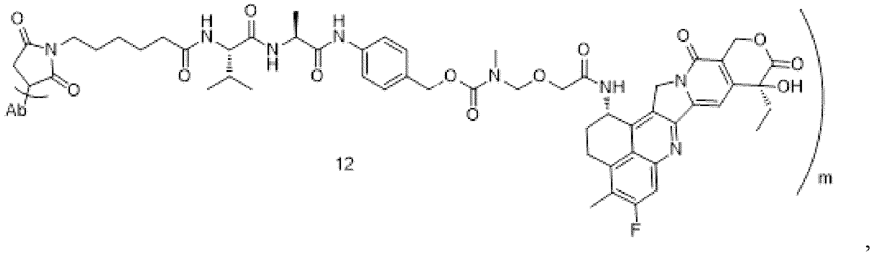 Ab is the TROP2 antibody, and m is 3.90; 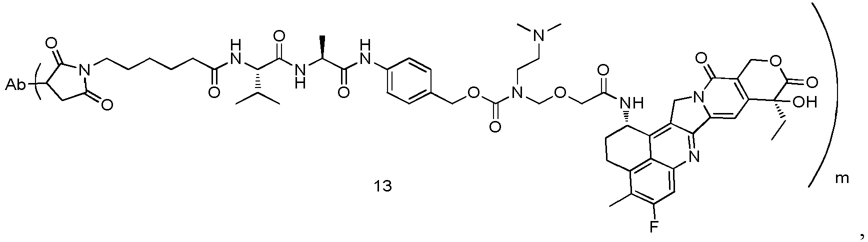 Ab is the TROP2 antibody, and m is 4.10; 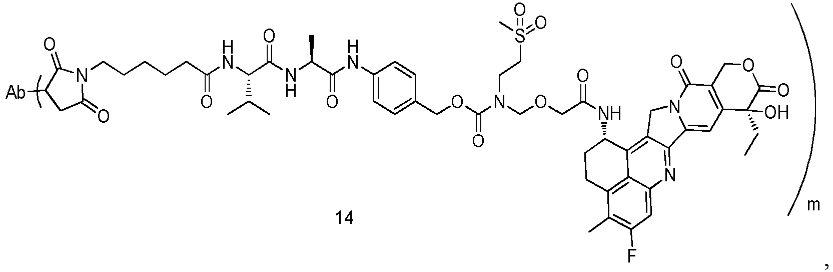 Ab is the TROP2 antibody, and m is 4.00; 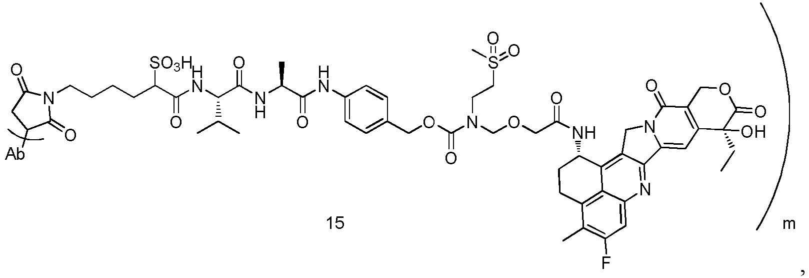 Ab is the TROP2 antibody, and m is 4.12; 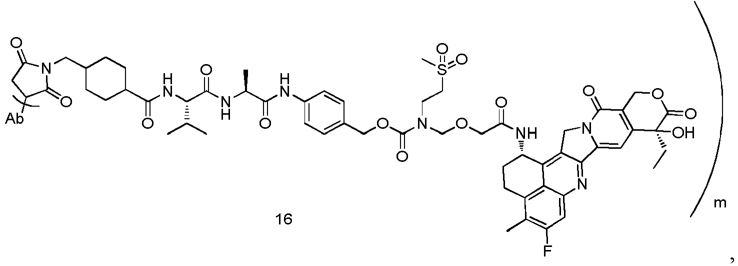 Ab is the TROP2 antibody, and m is 4.03; 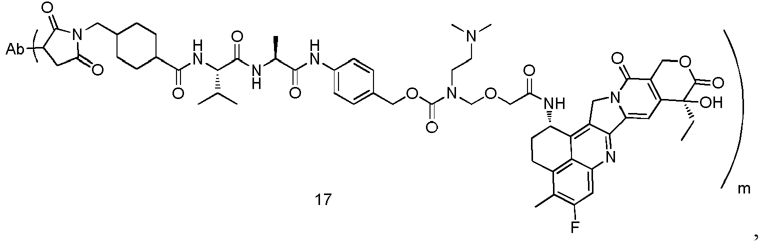 Ab is the TROP2 antibody, and m is 3.98; 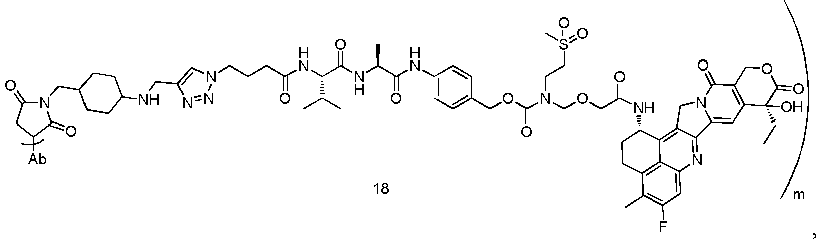 Ab is the TROP2 antibody, and m is 3.96; 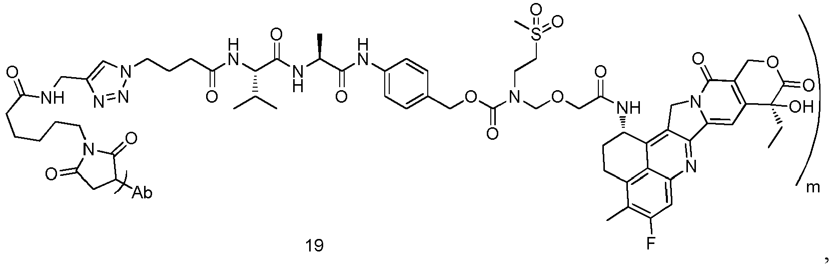 Ab is the TROP2 antibody, and m is 4.13; 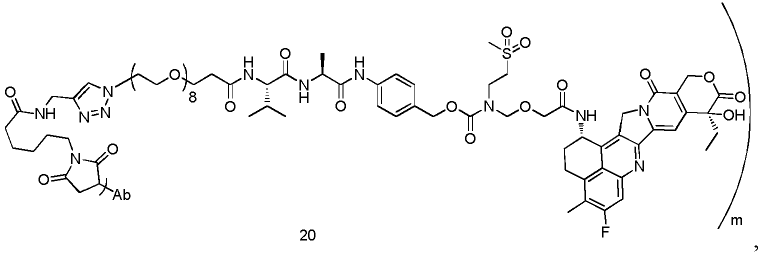 Ab is the TROP2 antibody, and m is 3.88; 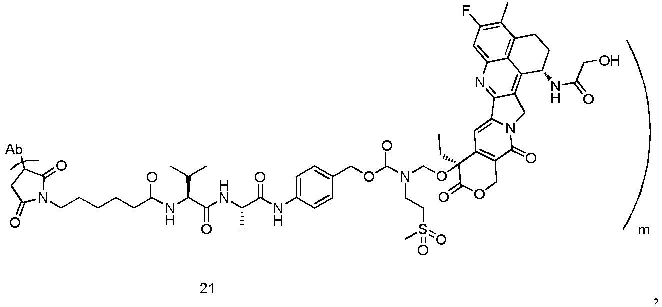 Ab is the TROP2 antibody, and m is 4.05; 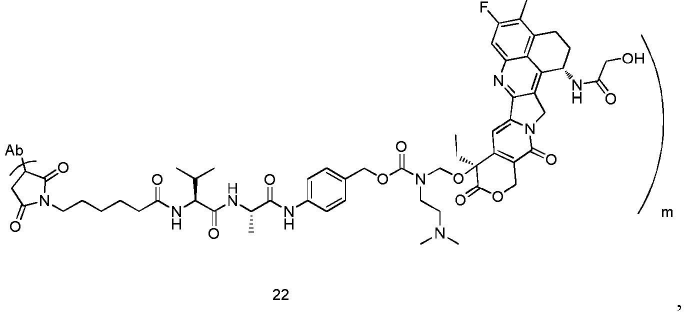 Ab is the TROP2 antibody, and m is 4.00.

8. A method for preparing the antibody-drug conjugate according to any one of claims 1 to 7, comprising the following steps: carrying out a coupling reaction between a compound of formula II and Ab-hydrogen as shown below;

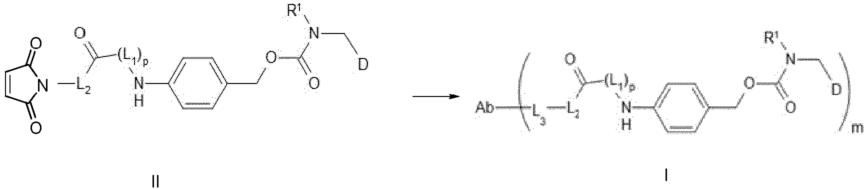

9. A pharmaceutical composition, comprising substance X and a pharmaceutically acceptable excipient, wherein the substance X is the antibody-drug conjugate, the pharmaceutically acceptable salt thereof, the solvate thereof, or the solvate of the pharmaceutically acceptable salt thereof according to any one of claims 1 to 7, the amount of the substance X is preferably a therapeutically effective amount.

10. Substance X for use in inhibiting TROP2 protein, wherein the substance X is the antibody-drug conjugate, the pharmaceutically acceptable salt thereof, the solvate thereof, or the solvate of the pharmaceutically acceptable salt thereof according to any one of claims 1 to 7.

11. Substance X for use in treating and/or preventing a tumor, wherein the substance X is the antibody-drug conjugate, the pharmaceutically acceptable salt thereof, the solvate thereof, or the solvate of the pharmaceutically acceptable salt thereof according to any one of claims 1 to 7.

12. Substance X for use according to claim 11, wherein the tumor is a TROP2 positive tumor; and the TROP2 positive tumor is one or more than one of TROP2 positive gastric cancer, triple negative breast cancer, and human pancreatic cancer.

13. Substance X for use according to claim 12, wherein the gastric cancer cells are *NCI-N87* cells;
and/or, the triple-negative breast cancer cells are MDA-MB-468 cells;
and/or, the pancreatic cancer cells are preferably BXPC3 cells.

## Patentansprüche

1. Antikörper-Wirkstoff-Konjugat der Formel **I,** pharmazeutisch annehmbares Salz davon, Solvat davon, oder Solvat des pharmazeutisch annehmbaren Salzes davon; 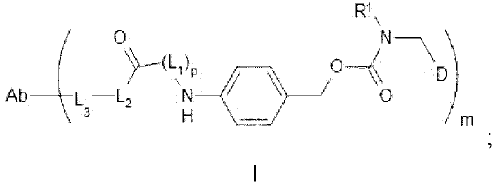
wobei Ab ein TROP2-Antikörper oder eine Variante des TROP2-Antikörpers ist;
die Aminosäuresequenz der leichten Kette im TROP2-Antikörper in der SEQ ID Nr: 1 dargestellt ist und die Aminosäuresequenz der schweren Kette in der SEQ ID Nr: 2 dargestellt ist;
die Variante des TROP2-Antikörpers zumindest 70 %, 75 %, 80 %, 85 %, 90 %, 95 %, 98 % oder 99 % identisch mit dem TROP2-Antikörper ist;
m 3,5 bis 4,5 beträgt;
D ein zytotoxischer Topoisomerase-Inhibitorwirkstoff ist, wobei der zytotoxische Topoisomerase-Inhibitorwirkstoff 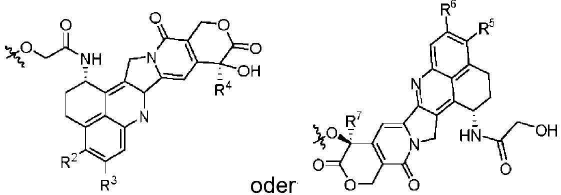 ist;
R² und R⁵ jeweils unabhängig voneinander H, C₁-C₆-Alkyl oder Halogen sind; R³ und R⁶ jeweils unabhängig voneinander H, C₁-C₆-Alkyl oder Halogen sind; R⁴ und R⁷ jeweils unabhängig voneinander C₁-C₆-Alkyl sind;
R¹ C₁-C₆-Alkyl ist, substituiert durch ein oder mehr als ein -NR¹⁻¹R¹⁻², C₁-C₆-Alkyl, substituiert durch ein oder mehr als ein R¹⁻³S(O)₂-, C₁-C₆-Alkyl, C₃-C₁₀-Cycloalkyl, C₆-C₁₄-Aryl oder 5- bis 14-gliedriges Heteroaryl; wobei das Heteroatom im 5- bis 14-gliedrigen Heteroaryl aus einem oder mehr als einem von N, O und S ausgewählt ist und die Anzahl von Heteroatomen 1, 2, 3 oder 4 beträgt; das R¹⁻¹, R¹⁻² und R¹⁻³ jeweils unabhängig voneinander C₁-C₆-Alkyl sind;
L₁ unabhängig einer oder mehr als einer von einem Phenylalaninrest, Alaninrest, Glycinrest, Asparaginsäurerest, Cysteinrest, Glutaminsäurerest, Histidinrest, Isoleucinrest, Leucinrest, Lysinrest, Methioninrest, Prolinrest, Serinrest, Threoninrest, Tryptophanrest, Tyrosinrest und Valinrest ist; p 2 bis 4 beträgt;
L₂ ist 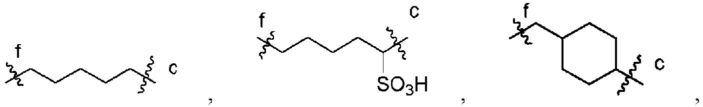 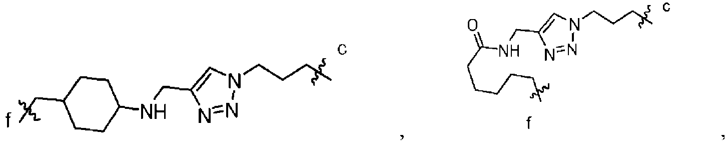 oder 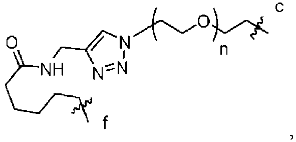 wobei n unabhängig 1 bis 12 beträgt, der c-Terminus mit L₁ durch eine Carbonylgruppe verbunden ist und der f-Terminus mit dem d-Terminus von L₃ verbunden ist;
L₃ ist 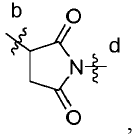 wobei der b-Termin mit dem Ab verbunden ist und der d-Terminus mit dem f-Terminus von L₂ verbunden ist.

2. Antikörper-Wirkstoff-Konjugat, pharmazeutisch annehmbares Salz davon, Solvat davon, oder Solvat des pharmazeutisch annehmbaren Salzes davon nach Anspruch 1, wobei,
wobei der b-Terminus von L₃ in Form eines Thioethers mit der Sulfhydrylgruppe am Antikörper verbunden ist;
und/oder, wenn R¹ C₁-C₆-Alkyl ist, substituiert durch ein oder mehr als ein -NR¹⁻¹R¹⁻², ist das C₁-C₆-Alkyl Methyl, Ethyl, n-Propyl, Isopropyl, n-Butyl, Isobutyl oder tert-Butyl, vorzugsweise Ethyl;
und/oder, wenn R¹ C₁-C₆-Alkyl ist, substituiert durch mehr als ein -NR¹⁻¹R¹⁻², ist das "mehr als ein" zwei oder drei;
und/oder, wenn R¹⁻¹ und R¹⁻² jeweils unabhängig voneinander C₁-C₆-Alkyl sind, ist das C₁-C₆-Alkyl Methyl, Ethyl, n-Propyl, Isopropyl, n-Butyl, Isobutyl oder tert-Butyl, vorzugsweise Methyl;
und/oder, wenn R¹ C₁-C₆-Alkyl ist, substituiert durch ein oder mehr als ein R¹⁻³S(O)₂-, ist das C₁-C₆-Alkyl C₁-C₄-Alkyl, vorzugsweise Methyl, Ethyl, n-Propyl, Isopropyl, n-Butyl, Isobutyl oder tert-Butyl, besonders bevorzugt Ethyl;
und/oder, wenn R¹ C₁-C₆-Alkyl ist, substituiert durch mehr als ein R¹⁻³S(O)₂-, ist das "mehr als ein" zwei oder drei;
und/oder, wenn R¹⁻³ C₁-C₆-Alkyl ist, ist das C₁-C₆-Alkyl Methyl, Ethyl, n-Propyl, Isopropyl, n-Butyl, Isobutyl oder tert-Butyl, vorzugsweise Methyl;
und/oder, wenn R¹ C₁-C₆-Alkyl ist, ist das C₁-C₆-Alkyl Methyl, Ethyl, n-Propyl, Isopropyl, n-Butyl, Isobutyl oder tert-Butyl, vorzugsweise Methyl;
und/oder n 8 bis 12 beträgt;
und/oder p 2 beträgt;
und/oder, wenn R¹⁻¹, R¹⁻² und R¹⁻³ unabhängig voneinander C₁-C₆-Alkyl sind, ist das C₁-C₆-Alkyl C₁-C₄-Alkyl, vorzugsweise Methyl, Ethyl, n-Propyl, Isopropyl, n-Butyl, Isobutyl oder tert-Butyl, besonders bevorzugt Methyl.

3. Antikörper-Wirkstoff-Konjugat, pharmazeutisch annehmbares Salz davon, Solvat davon, oder Solvat des pharmazeutisch annehmbaren Salzes davon nach Anspruch 1, wobei,
wenn R² und R⁵ jeweils unabhängig voneinander C₁-C₆-Alkyl sind, ist das C₁-C₆-Alkyl C₁-C₄-Alkyl, weiter vorzugsweise Methyl, Ethyl, n-Propyl, Isopropyl, n-Butyl, Isobutyl oder tert-Butyl, besonders bevorzugt Methyl;
und/oder, wenn R² und R⁵ jeweils unabhängig voneinander Halogen sind, ist das Halogen Fluor, Chlor, Brom oder Iod, vorzugsweise Fluor;
und/oder, wenn R³ und R⁶ jeweils unabhängig voneinander C₁-C₆-Alkyl sind, ist das C₁-C₆-Alkyl C₁-C₄-Alkyl, weiter vorzugsweise Methyl, Ethyl, n-Propyl, Isopropyl, n-Butyl, Isobutyl oder tert-Butyl, besonders bevorzugt Methyl;
und/oder, wenn R³ und R⁶ jeweils unabhängig voneinander Halogen sind, ist das Halogen Fluor, Chlor, Brom oder Iod, weiter vorzugsweise Fluor;
und/oder, wenn R⁴ und R⁷ jeweils unabhängig voneinander C₁-C₆-Alkyl sind, ist das C₁-C₆-Alkyl C₁-C₄-Alkyl, weiter vorzugsweise Methyl, Ethyl, n-Propyl, Isopropyl, n-Butyl, Isobutyl oder tert-Butyl, besonders bevorzugt Ethyl;
und/oder, wenn R¹ C₁-C₆-Alkyl ist, substituiert durch ein oder mehr als ein -NR¹⁻¹R¹⁻², ist das -NR¹⁻¹R¹⁻² -N(CH₃)₂;
und/oder, wenn R¹ C₁-C₆-Alkyl ist, substituiert durch ein -NR¹⁻¹R¹⁻², ist das C₁-C₆-Alkyl, substituiert durch ein -NR¹⁻¹R¹⁻² 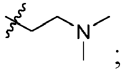
und/oder, wenn R¹ C₁-C₆-Alkyl ist, substituiert durch ein R¹⁻³S(O)₂-, ist das C₁-C₆-Alkyl, substituiert durch ein R¹⁻³S(O)₂- 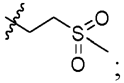
und/oder m beträgt 3,8 bis 4,2, wie etwa 3,88, 3,90, 3,96, 3,97, 3,98, 4,00, 4,03, 4,05, 4,10, 4,12 oder 4,13;
und/oder (L₁)ₚ ist 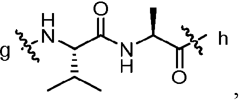 wobei der g-Terminus mit dem c-Terminus von L₂ durch eine Carbonylgruppe verbunden ist;
und/oder n 8, 9, 10, 11 oder 12 beträgt.

4. Antikörper-Wirkstoff-Konjugat, pharmazeutisch annehmbares Salz davon, Solvat davon, oder Solvat des pharmazeutisch annehmbaren Salzes davon nach Anspruch 1, wobei
R² und R⁵ jeweils unabhängig voneinander C₁-C₆-Alkyl sind;
und/oder R³ und R⁶ jeweils unabhängig voneinander ein Halogen sind;
und/oder R⁴ und R⁷ Ethyl sind;
und/oder L₁ einer oder mehr als einer von einem Phenylalaninrest, Alaninrest, Glycinrest, Isoleucinrest, Leucinrest, Prolinrest und Valinrest ist; vorzugsweise einer oder mehr als einer von einem Phenylalaninrest, Alaninrest, Glycinrest und Valinrest; weiter vorzugsweise der Valinrest und/oder der Alaninrest; und das "mehr als ein" ist vorzugsweise zwei oder drei;
und/oder R¹ C₁-C₆-Alkyl ist, substituiert durch ein oder mehr als ein -NR¹⁻¹R¹⁻², C₁-C₆-Alkyl, substituiert durch ein oder mehr als ein R¹⁻³S(O)₂-, C₁-C₆-Alkyl, oder C₃-C₁₀-Cycloalkyl, vorzugsweise C₁-C₆-Alkyl, substituiert durch ein oder mehr als ein -NR¹⁻¹R¹⁻², C₁-C₆-Alkyl, substituiert durch ein oder mehr als ein R¹⁻³S(O)₂-, oder C₁-C₆-Alkyl, weiter vorzugsweise C₁-C₆-Alkyl, substituiert durch ein oder mehr als ein -NR¹⁻¹R¹⁻², oder C₁-C₆-Alkyl, substituiert durch ein oder mehr als ein R¹⁻³S(O)₂-, besonders bevorzugt C₁-C₆-Alkyl, substituiert durch ein oder mehr als ein R¹⁻³S(O)₂-;
vorzugsweise ist D

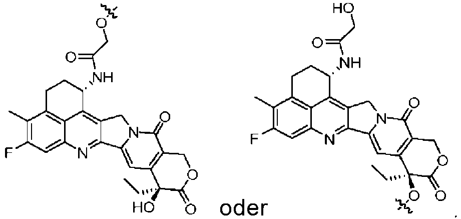

5. Antikörper-Wirkstoff-Konjugat, pharmazeutisch annehmbares Salz davon, Solvat davon, oder Solvat des pharmazeutisch annehmbaren Salzes davon nach einem der Ansprüche 1 bis 4, wobei das Antikörper-Wirkstoff-Konjugat ein beliebiges der folgenden Schemata ist:
Schema I:
wobei Ab ein TROP2-Antikörper oder eine Variante des TROP2-Antikörpers ist;
D ist 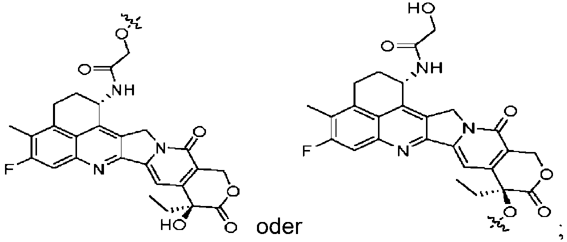
L₁ unabhängig einer oder mehr als einer von einem Phenylalaninrest, Alaninrest, Glycinrest, Isoleucinrest, Leucinrest, Prolinrest und Valinrest ist;
Schema II:
wobei Ab ein TROP2-Antikörper oder eine Variante des TROP2-Antikörpers ist;
D ist 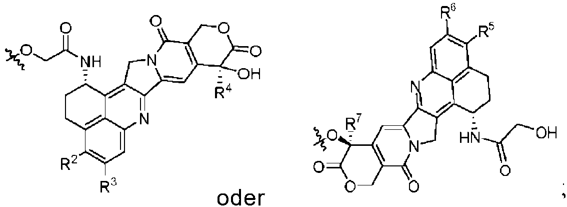 wobei R² und R⁵ jeweils unabhängig voneinander C₁-C₆-Alkyl sind; R³ und R⁶ jeweils unabhängig voneinander Halogen sind;
vorzugsweise ist D 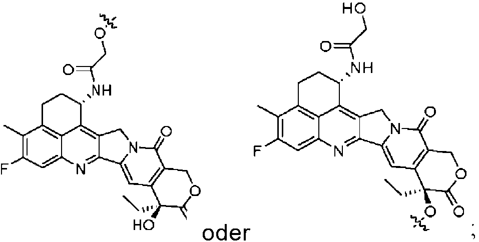
R¹ C₁-C₆-Alkyl ist, substituiert durch ein oder mehr als ein -NR¹⁻¹R¹⁻², C₁-C₆-Alkyl, substituiert durch ein oder mehr als ein R¹⁻³S(O)₂-, oder C₁-C₆-Alkyl;
L₁ unabhängig einer oder mehr als einer von einem Phenylalaninrest, Alaninrest, Glycinrest und Valinrest ist;
Schema III:
wobei Ab der TROP2-Antikörper ist;
D ist 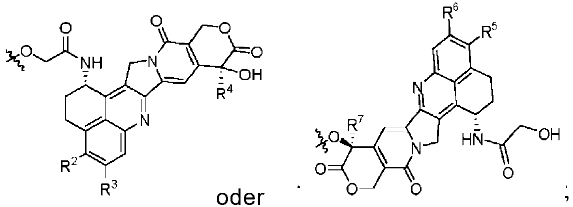 wobei R² und R⁵ jeweils unabhängig voneinander C₁-C₆-Alkyl sind; R³ und R⁶ jeweils unabhängig voneinander Halogen sind;
vorzugsweise ist D 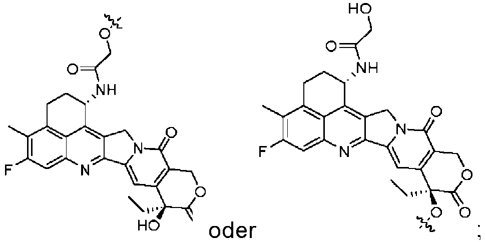
R¹ C₁-C₆-Alkyl ist, substituiert durch ein oder mehr als ein -NR¹⁻¹R¹⁻², C₁-C₆-Alkyl, substituiert durch ein oder mehr als ein R¹⁻³S(O)₂-, oder C₁-C₆-Alkyl;
L₁ unabhängig der Valinrest und/oder der Alaninrest ist;
Schema IV:
wobei Ab der TROP2-Antikörper ist;
D ist 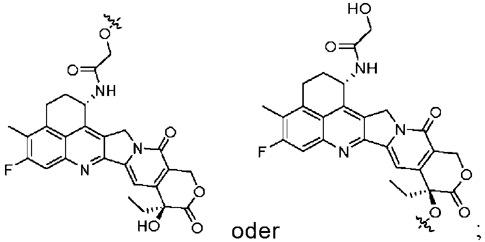
R¹ C₁-C₆-Alkyl ist, substituiert durch ein oder mehr als ein -NR¹⁻¹R¹⁻², C₁-C₆-Alkyl, substituiert durch ein oder mehr als ein R¹⁻³S(O)₂-, oder C₁-C₆-Alkyl;
L₁ unabhängig der Valinrest und/oder der Alaninrest ist;
Schema V:
wobei Ab der TROP2-Antikörper ist;
D ist 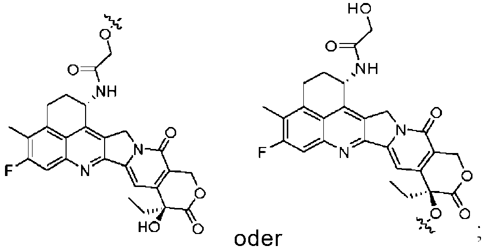
R¹ C₁-C₆-Alkyl ist, oder stituiert durch ein oder mehr als ein -NR¹⁻¹R¹⁻², C₁-C₆-Alkyl, substituiert durch ein oder mehr als ein R¹⁻³S(O)₂-, oder C₁-C₆-Alkyl;
L₁ unabhängig der Valinrest und/oder der Alaninrest ist.

6. Antikörper-Wirkstoff-Konjugat, pharmazeutisch annehmbares Salz davon, Solvat davon, oder Solvat des pharmazeutisch annehmbaren Salzes davon nach Anspruch 1, wobei das Antikörper-Wirkstoff-Konjugat ein beliebiges der folgenden Schemata ist: 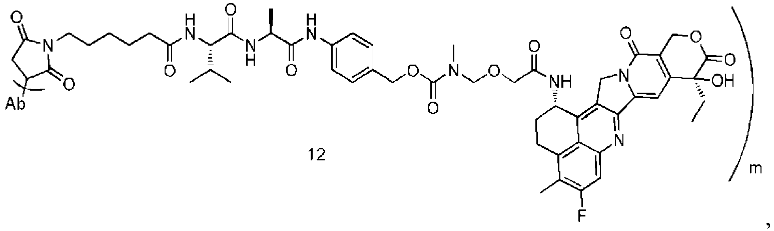 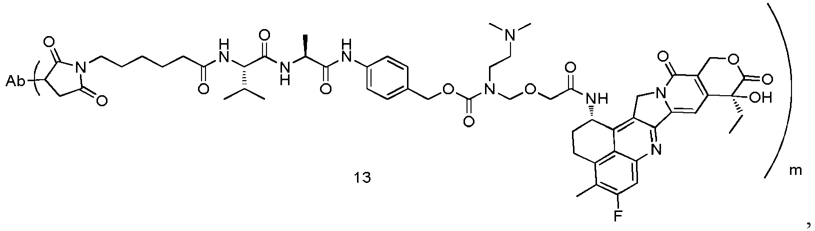 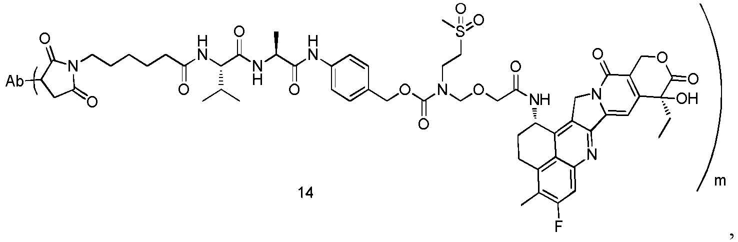 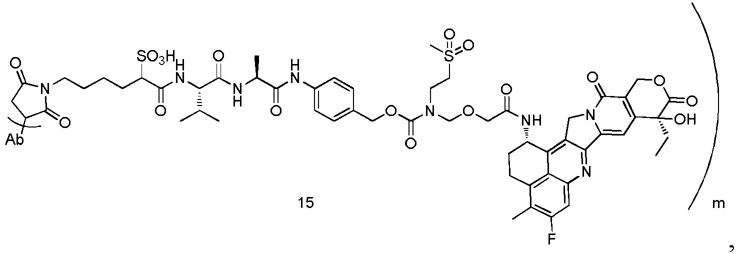 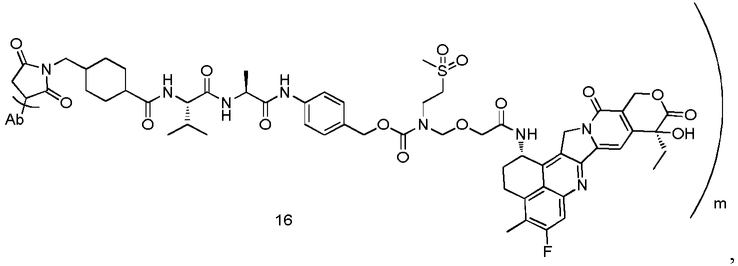 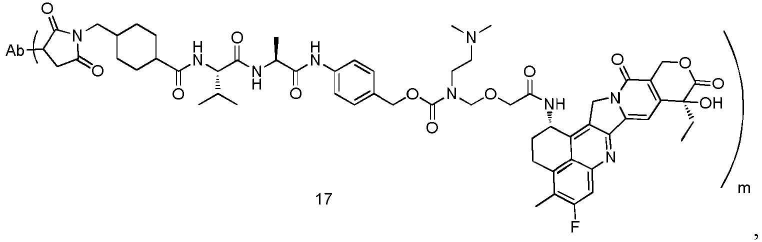 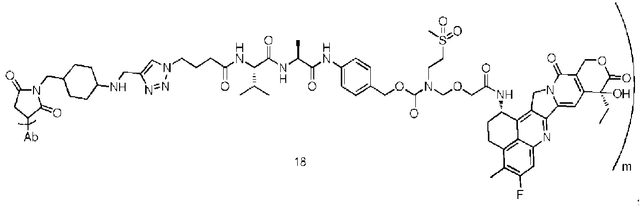 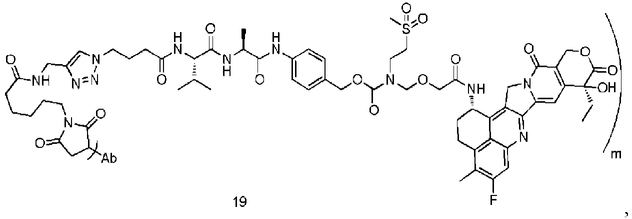 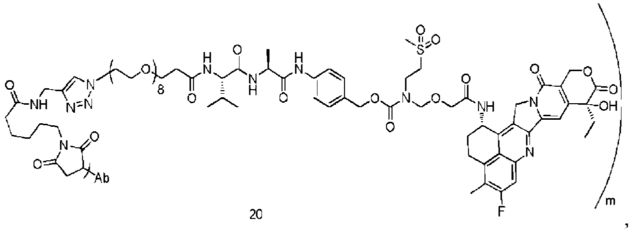 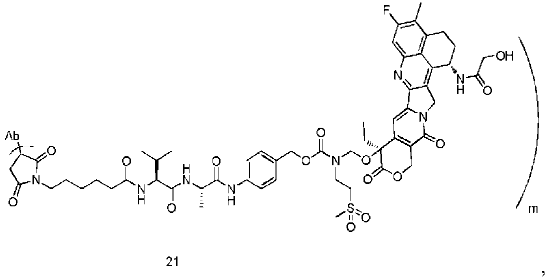 oder 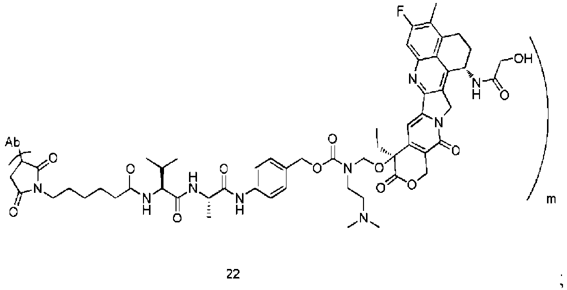 wobei Ab der TROP2-Antikörper oder die Variante des TROP2-Antikörpers ist und m 3,8 bis 4,2 beträgt, vorzugsweise 3,88, 3,90, 3,96, 3,97, 3,98, 4,00, 4,03, 4,05, 4,10, 4,12 oder 4,13; die Aminosäuresequenz der leichten Kette in der Variante des TROP2-Antikörpers vorzugsweise in SEQ ID NO: 1 dargestellt ist und die Aminosäuresequenz der schweren Kette vorzugsweise in SEQ ID NO: 2 dargestellt ist.

7. Antikörper-Wirkstoff-Konjugat, pharmazeutisch annehmbares Salz davon, Solvat davon, oder Solvat des pharmazeutisch annehmbaren Salzes davon nach Anspruch 1, wobei das Antikörper-Wirkstoff-Konjugat ein beliebiges der folgenden Schemata ist:
Schema A: 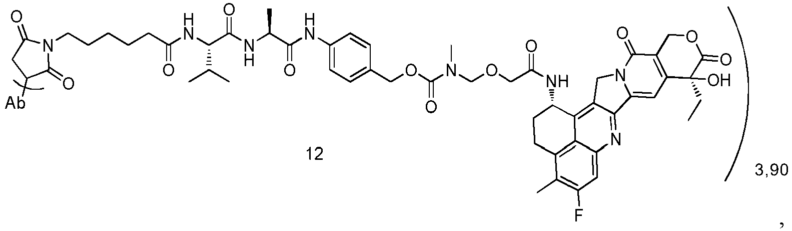 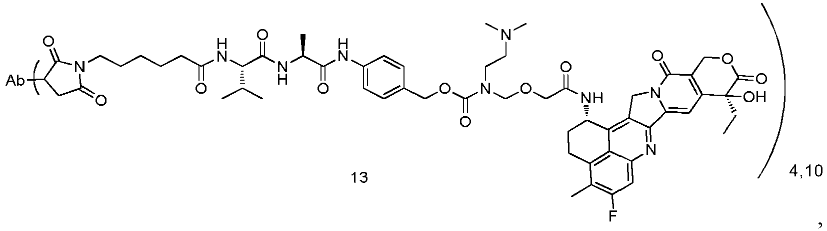 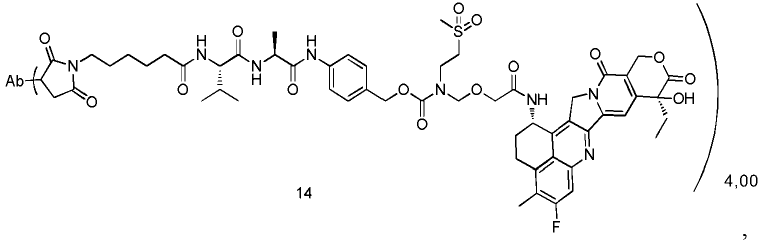 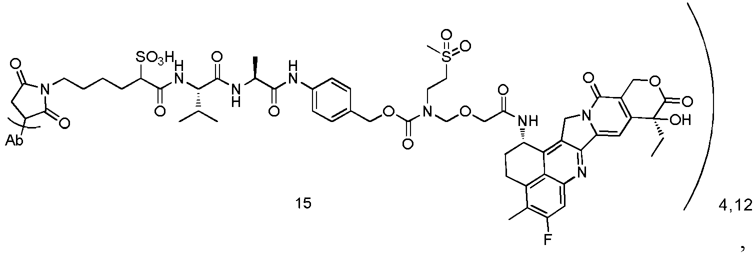 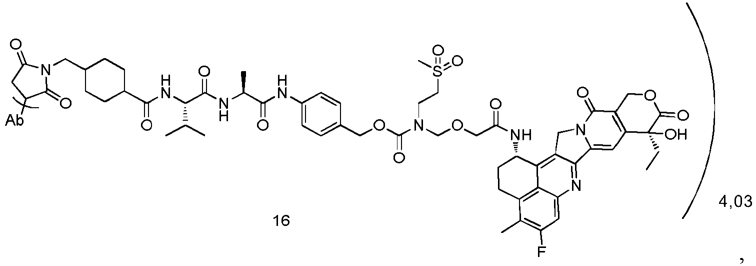 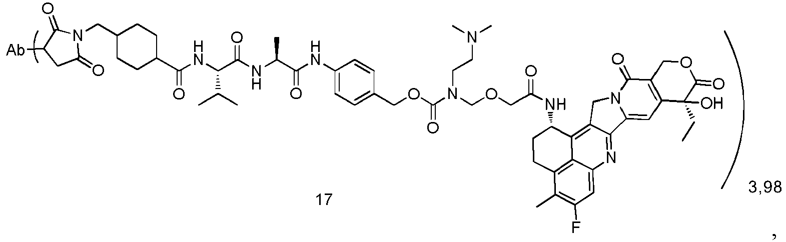 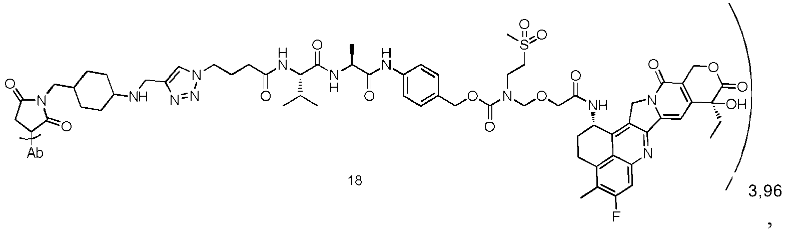 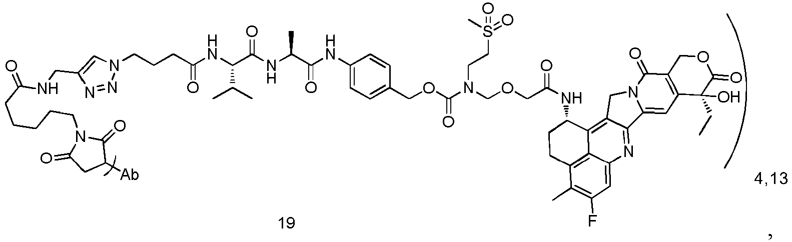 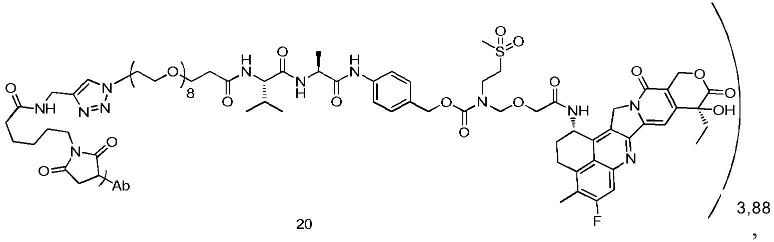 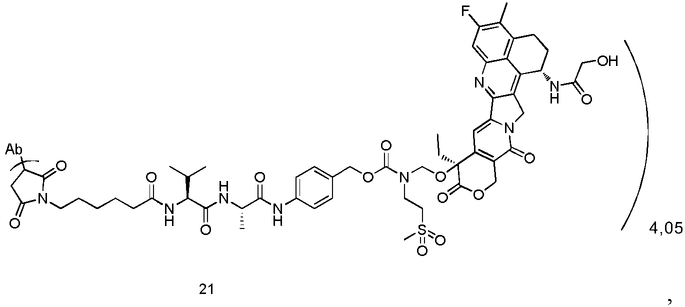 oder 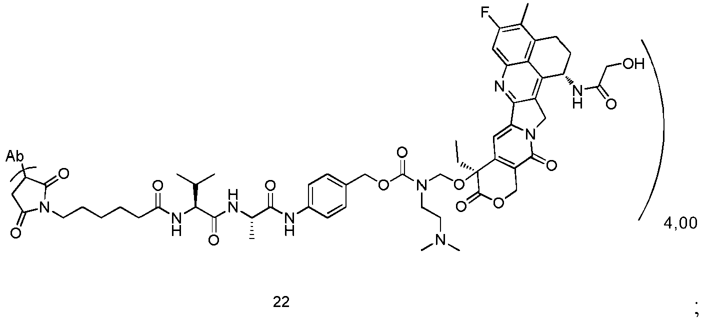
wobei Ab der TROP2-Antikörper oder die Variante des TROP2-Antikörpers ist; die Aminosäuresequenz der leichten Kette in der Variante des TROP2-Antikörpers in SEQ ID NO: 1 dargestellt ist und die Aminosäuresequenz der schweren Kette in SEQ ID NO: 2 dargestellt ist;
Schema B: 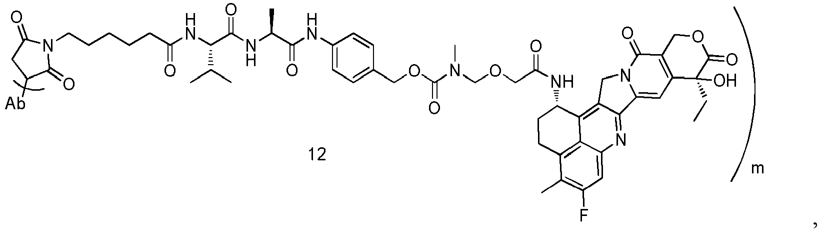 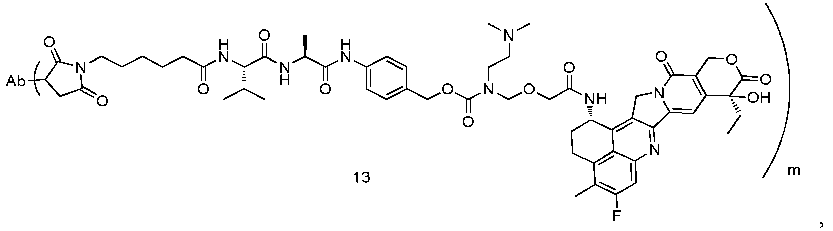 oder 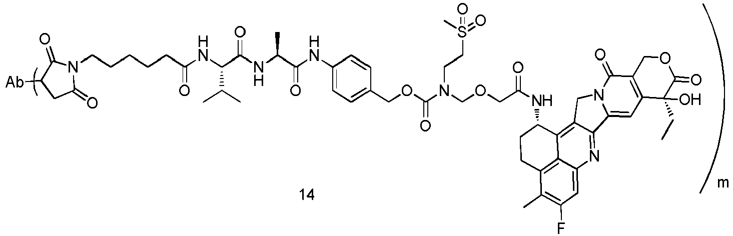
wobei Ab der TROP2-Antikörper oder die Variante des TROP2-Antikörpers ist, m 3,90, 4,00, 4,10 beträgt; die Aminosäuresequenz der leichten Kette in der Variante des TROP2-Antikörpers in SEQ ID NO: 1 dargestellt ist und die Aminosäuresequenz der schweren Kette in SEQ ID NO: 2 dargestellt ist.
Schema C: 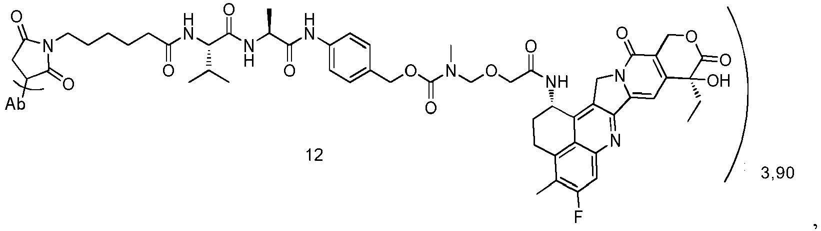 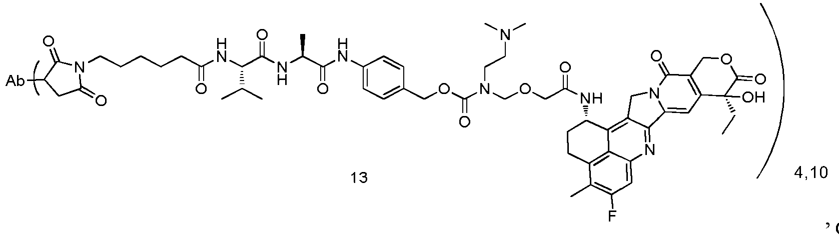 oder 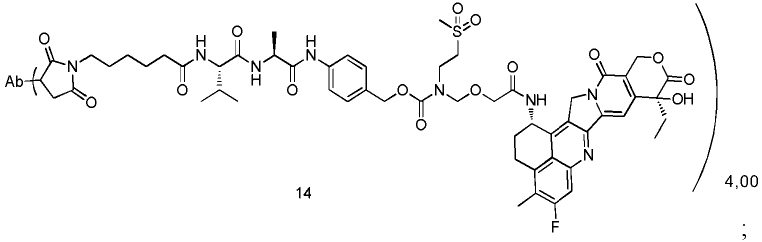
wobei Ab ein TROP2-Antikörper oder eine Variante des TROP2-Antikörpers ist;
Schema D: 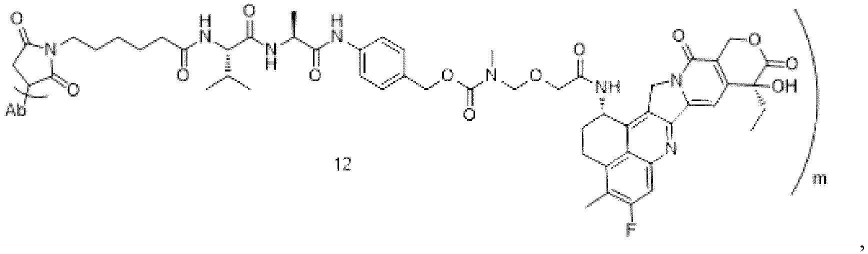
wobei Ab der TROP2-Antikörper ist und m 3,90 beträgt; 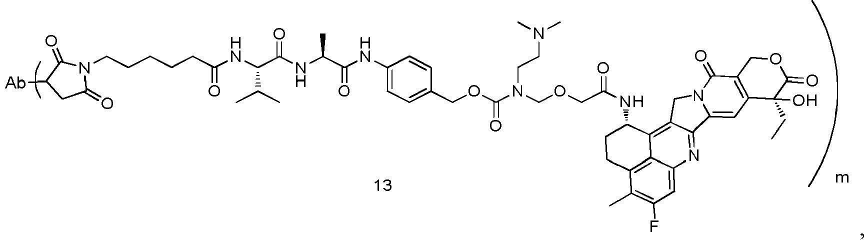
wobei Ab der TROP2-Antikörper ist und m 4,10 beträgt; 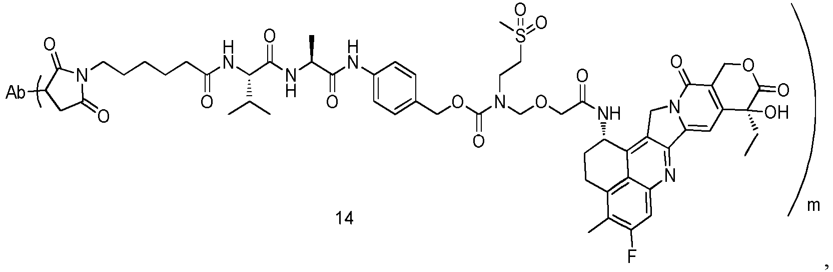
wobei Ab der TROP2-Antikörper ist und m 4,00 beträgt; 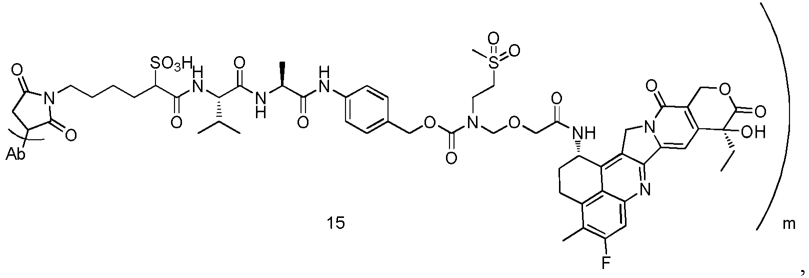
wobei Ab der TROP2-Antikörper ist und m 4,12 beträgt; 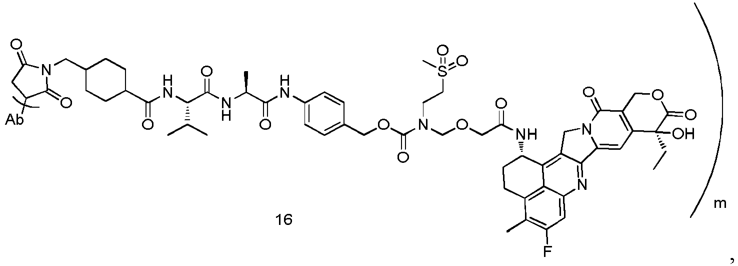
wobei Ab der TROP2-Antikörper ist und m 4,03 beträgt; 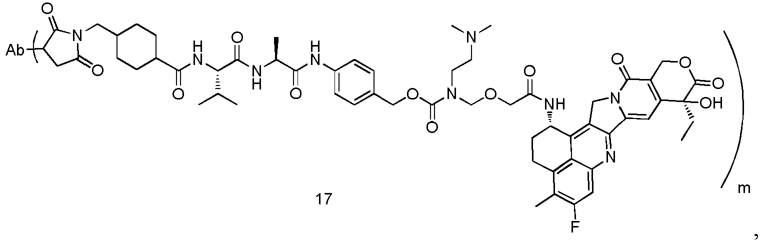
wobei Ab der TROP2-Antikörper ist und m 3,98 beträgt; 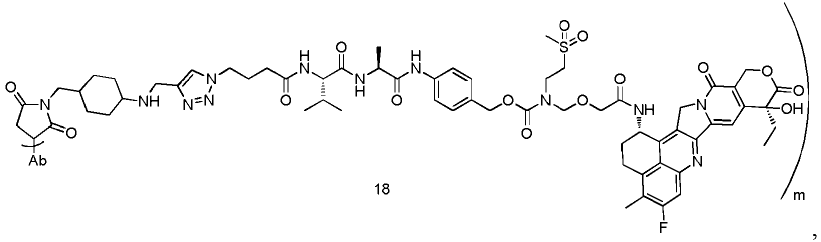
wobei Ab der TROP2-Antikörper ist und m 3,96 beträgt; 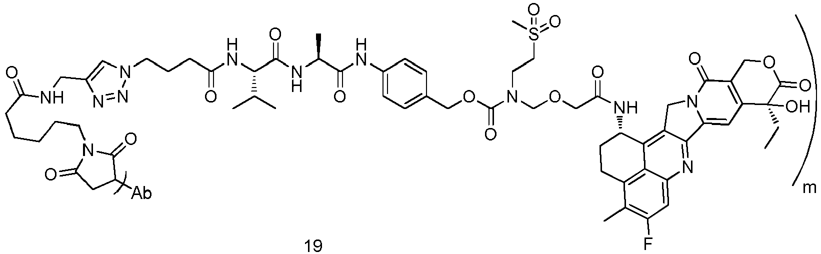
wobei Ab der TROP2-Antikörper ist und m 4,13 beträgt; 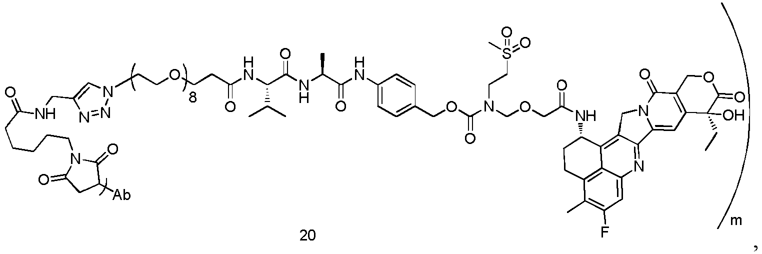
wobei Ab der TROP2-Antikörper ist und m 3,88 beträgt; 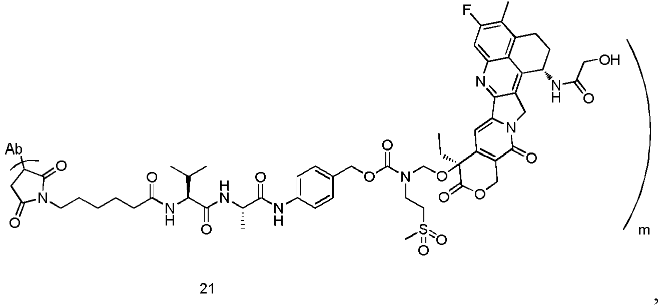
wobei Ab der TROP2-Antikörper ist und m 4,05 beträgt; 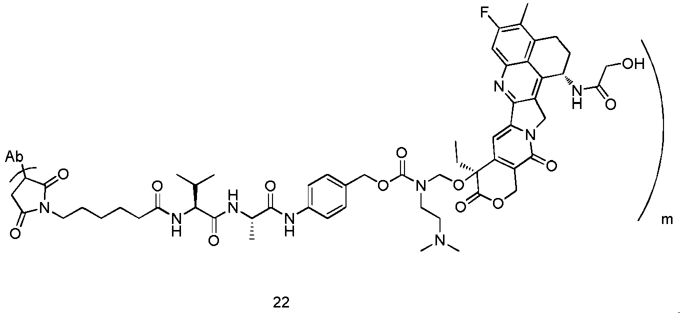
wobei Ab der TROP2-Antikörper ist und m 4,00 beträgt;

8. Verfahren zum Herstellen des Antikörper-Wirkstoff-Konjugats nach einem der Ansprüche 1 bis 7, umfassend die folgenden Schritte: Durchführen einer Kupplungsreaktion zwischen einer Verbindung der Formel II und Ab-Wasserstoff, wie unten dargestellt;

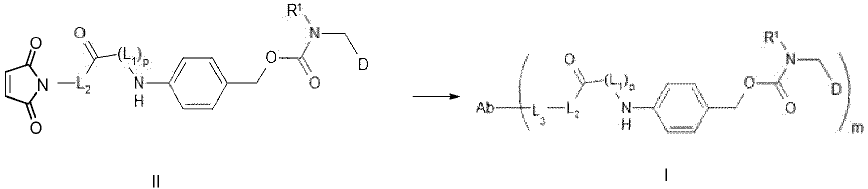

9. Pharmazeutische Zusammensetzung, umfassend Substanz X und einen pharmazeutisch annehmbaren Hilfsstoff; wobei die Substanz X das Antikörper-Wirkstoff-Konjugat, das pharmazeutisch annehmbare Salz davon, das Solvat davon, oder das Solvat des pharmazeutisch annehmbaren Salzes davon nach einem der Ansprüche 1 bis 7 ist, die Menge der Substanz X vorzugsweise eine therapeutisch wirksame Menge ist.

10. Substanz X zur Verwendung bei einer Hemmung des TROP2-Proteins, wobei die Substanz X das Antikörper-Wirkstoff-Konjugat, das pharmazeutisch annehmbare Salz davon, das Solvat davon, oder das Solvat des pharmazeutisch annehmbaren Salzes davon nach einem der Ansprüche 1 bis 7 ist.

11. Substanz X zur Verwendung bei einer Behandlung und/oder Vorbeugung eines Tumors, wobei die Substanz X das Antikörper-Wirkstoff-Konjugat, das pharmazeutisch annehmbare Salz davon, das Solvat davon, oder das Solvat des pharmazeutisch annehmbaren Salzes davon nach einem der Ansprüche 1 bis 7 ist.

12. Substanz X zur Verwendung nach Anspruch 11, wobei es sich bei dem Tumor um einen positiven TROP2-Tumor handelt und es sich bei dem positiven TROP2-Tumor um einen oder mehr als einen von einem positiven TROP2-Magenkrebs, dreifach negativen Brustkrebs und menschlichen Bauchspeicheldrüsenkrebs handelt.

13. Substanz X zur Verwendung nach Anspruch 12, wobei die Magenkrebszellen *NCI-N87*-Zellen sind;
und/oder die Zellen des dreifach negativen Brustkrebses MDA-MB-468-Zellen sind;
und/oder die Bauchspeicheldrüsenzellen vorzugsweise BXPC3-Zellen sind.

## Revendications

1. Conjugué anticorps-médicament de formule **I,** sel pharmaceutiquement acceptable de celui-ci, solvate de celui-ci ou solvate du sel pharmaceutiquement acceptable de celui-ci ; 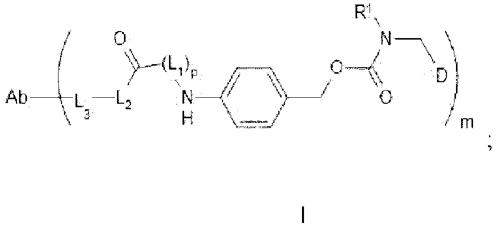
dans lequel Ab est un anticorps TROP2 ou un variant de l'anticorps TROP2 ;
la séquence d'acides aminés de la chaîne légère dans l'anticorps TROP2 est montrée dans SEQ ID NO : 1, et la séquence d'acides aminés de la chaîne lourde est montrée dans SEQ ID NO : 2 ;
le variant de l'anticorps TROP2 est au moins 70 %, 75 %, 80 %, 85 %, 90 %, 95 %, 98 % ou 99 % identique à l'anticorps TROP2 ;
m vaut de 3,5 à 4,5 ;
D est un médicament cytotoxique inhibiteur de topoisomérase, dans lequel le médicament cytotoxique 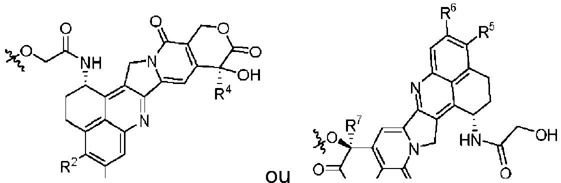 inhibiteur de topoisomérase est
R² et R⁵ sont chacun indépendamment H, un alkyle en C₁-C₆ ou un halogène ; R³ et R⁶ sont chacun indépendamment H, un alkyle en C₁-C₆ ou un halogène ; R⁴ et R⁷ sont chacun indépendamment un alkyle en C₁-C₆ ;
R¹ est un alkyle en C₁-C₆ substitué par un ou plus d'un -NR¹⁻¹R¹⁻², un alkyle en C₁-C₆ substitué par un ou plus d'un R¹⁻³S(O)₂-, un alkyle en C₁-C₆, un cycloalkyle en C₃-C₁₀, un aryle en C₆-C₁₄ ou un hétéroaryle à 5 à 14 chaînons ; l'hétéroatome dans l'hétéroaryle à 5 à 14 chaînons est sélectionné parmi un ou plus d'un de N, O et S, et le nombre d'hétéroatomes est 1, 2, 3 ou 4 ; les R¹⁻¹, R¹⁻² et R¹⁻³ sont chacun indépendamment un alkyle en C₁-C₆ ;
L₁ est indépendamment un ou plus d'un parmi un résidu de phénylalanine, un résidu d'alanine, un résidu de glycine, un résidu d'acide aspartique, un résidu de cystéine, un résidu d'acide glutamique, un résidu d'histidine, un résidu d'isoleucine, un résidu de leucine, un résidu de lysine, un résidu de méthionine, un résidu de proline, un résidu de sérine, un résidu de thréonine, un résidu de tryptophane, un résidu de tyrosine et un résidu de valine ; p vaut de 2 à 4 ; 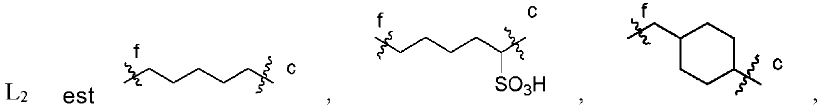 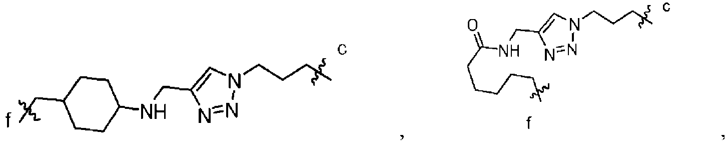 ou 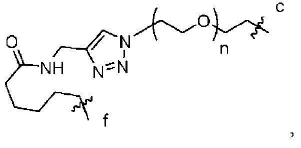 dans lequel n vaut indépendamment de 1 à 12, la terminaison c est reliée à L₁ par l'intermédiaire d'un groupe carbonyle, et la terminaison f est reliée à la terminaison d de L₃ ;
L₃ est 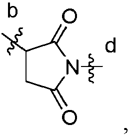 dans lequel la terminaison b est reliée à Ab, et la terminaison d est reliée à la terminaison f de L₂.

2. Conjugué anticorps-médicament, sel pharmaceutiquement acceptable de celui-ci, solvate de celui-ci ou solvate du sel pharmaceutiquement acceptable de celui-ci selon la revendication 1, dans lequel
la terminaison b de L₃ est reliée au groupe sulfhydryle sur l'anticorps sous la forme d'un thioéther ;
et/ou, lorsque R¹ est un alkyle en C₁-C₆ substitué par un ou plus d'un -NR¹⁻¹R¹⁻², l'alkyle en C₁-C₆ est du méthyle, de l'éthyle, du n-propyle, de l'isopropyle, du n-butyle, de l'isobutyle ou du tert-butyle, de préférence de l'éthyle ;
et/ou, lorsque R¹ est un alkyle en C₁-C₆ substitué par plus d'un -NR¹⁻¹R¹⁻², le « plus d'un » est deux ou trois ;
et/ou, lorsque R¹⁻¹ et R¹⁻² sont chacun indépendamment un alkyle en C₁-C₆, l'alkyle en C₁-C₆ est du méthyle, de l'éthyle, du n-propyle, de l'isopropyle, du n-butyle, de l'isobutyle ou du tert-butyle, de préférence du méthyle ;
et/ou, lorsque R¹ est un alkyle en C₁-C₆ substitué par un ou plus d'un R¹⁻³S(O)₂-, l'alkyle en C₁-C₆ est un alkyle en C₁-C₄, de préférence du méthyle, de l'éthyle, du n-propyle, de l'isopropyle, du n-butyle, de l'isobutyle ou du tert-butyle, le plus préférentiellement de l'éthyle ;
et/ou, lorsque R¹ est un alkyle en C₁-C₆ substitué par plus d'un R¹⁻³S(O)₂-, le « plus d'un » est deux ou trois ;
et/ou, lorsque R¹⁻³ est un alkyle en C₁-C₆, l'alkyle en C₁-C₆ est du méthyle, de l'éthyle, du n-propyle, de l'isopropyle, du n-butyle, de l'isobutyle ou du tert-butyle, de préférence du méthyle ;
et/ou, lorsque R¹ est un alkyle en C₁-C₆, l'alkyle en C₁-C₆ est du méthyle, de l'éthyle, du n-propyle, de l'isopropyle, du n-butyle, de l'isobutyle ou du tert-butyle, de préférence du méthyle ;
et/ou, n vaut de 8 à 12 ;
et/ou, p vaut 2 ;
et/ou, lorsque R¹⁻¹, R¹⁻² et R¹⁻³ sont indépendamment un alkyle en C₁-C₆, l'alkyle en C₁-C₆ est un alkyle en C₁-C₄, de préférence du méthyle, de l'éthyle, du n-propyle, de l'isopropyle, du n-butyle, de l'isobutyle ou du tert-butyle, le plus préférentiellement du méthyle.

3. Conjugué anticorps-médicament, sel pharmaceutiquement acceptable de celui-ci, solvate de celui-ci ou solvate du sel pharmaceutiquement acceptable de celui-ci selon la revendication 1, dans lequel
lorsque R² et R⁵ sont chacun indépendamment un alkyle en C₁-C₆, l'alkyle en C₁-C₆ est un alkyle en C₁-C₄, de façon encore préférée du méthyle, de l'éthyle, du n-propyle, de l'isopropyle, du n-butyle, de l'isobutyle ou du tert-butyle, le plus préférentiellement du méthyle ;
et/ou, lorsque R² et R⁵ sont chacun indépendamment un halogène, l'halogène est du fluor, du chlore, du brome ou de l'iode, de préférence du fluor ;
et/ou, lorsque R³ et R⁶ sont chacun indépendamment un alkyle en C₁-C₆, l'alkyle en C₁-C₆ est un alkyle en C₁-C₄, de façon encore préférée du méthyle, de l'éthyle, du n-propyle, de l'isopropyle, du n-butyle, de l'isobutyle ou du tert-butyle, le plus préférentiellement du méthyle ;
et/ou, lorsque R³ et R⁶ sont chacun indépendamment un halogène, l'halogène est du fluor, du chlore, du brome ou de l'iode, de façon encore préférée du fluor ;
et/ou, lorsque R⁴ et R⁷ sont chacun indépendamment un alkyle en C₁-C₆, l'alkyle en C₁-C₆ est un alkyle en C₁-C₄, de façon encore préférée du méthyle, de l'éthyle, du n-propyle, de l'isopropyle, du n-butyle, de l'isobutyle ou du tert-butyle, le plus préférentiellement de l'éthyle ;
et/ou, lorsque R¹ est un alkyle en C₁-C₆ substitué par un ou plus d'un -NR¹⁻¹R¹⁻², le -NR¹⁻¹R¹⁻² est -N(CH₃)₂ ;
et/ou, lorsque R¹ est un alkyle en C₁-C₆ substitué par un -NR¹⁻¹R¹⁻², l'alkyle en C₁-C₆ substitué par un -NR¹⁻¹R¹⁻² est 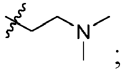
et/ou, lorsque R¹ est un alkyle en C₁-C₆ substitué par un R¹⁻³S(O)₂-, l'alkyle en C₁-C₆ substitué par un R¹⁻³S(O)₂- est 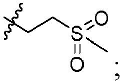
et/ou, m vaut de 3,8 à 4,2, tel que 3,88, 3,90, 3,96, 3,97, 3,98, 4,00, 4,03, 4,05, 4,10, 4,12 ou 4,13 ;
et/ou, (L₁)ₚ est 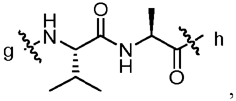 dans laquelle la terminaison g est reliée à la terminaison c de L₂ par l'intermédiaire d'un groupe carbonyle ;
et/ou, n vaut 8, 9, 10, 11 ou 12.

4. Conjugué anticorps-médicament, sel pharmaceutiquement acceptable de celui-ci, solvate de celui-ci ou solvate du sel pharmaceutiquement acceptable de celui-ci selon la revendication 1, dans lequel
R² et R⁵ sont chacun indépendamment un alkyle en C₁-C₆ ;
et/ou, R³ et R⁶ sont chacun indépendamment halogène ;
et/ou, R⁴ et R⁷ sont éthyle ;
et/ou, L₁ est un ou plus d'un parmi le résidu de phénylalanine, le résidu d'alanine, le résidu de glycine, le résidu d'isoleucine, le résidu de leucine, le résidu de proline et le résidu de valine ; de préférence un ou plus d'un parmi le résidu de phénylalanine, le résidu d'alanine, le résidu de glycine et le résidu de valine ; de façon encore préférée le résidu de valine et/ou le résidu d'alanine, et le « plus d'un » est de préférence deux ou trois ;
et/ou, R¹ est un alkyle en C₁-C₆ substitué par un ou plus d'un -NR¹⁻¹R¹⁻², un alkyle en C₁-C₆ substitué par un ou plus d'un R¹⁻³S(O)₂-, un alkyle en C₁-C₆ ou un cycloalkyle en C₃-C₁₀, de préférence un alkyle en C₁-C₆ substitué par un ou plus d'un -NR¹⁻¹R¹⁻², un alkyle en C₁-C₆ substitué par un ou plus d'un R¹⁻³S(O)₂-, ou un alkyle en C₁-C₆, de façon encore préférée un alkyle en C₁-C₆ substitué par un ou plus d'un -NR¹⁻¹R¹⁻², ou un alkyle en C₁-C₆ substitué par un ou plus d'un R¹⁻³S(O)₂-, le plus préférentiellement un alkyle en C₁-C₆ substitué par un ou plus d'un R¹⁻³S(O)₂- ;
de préférence, D est

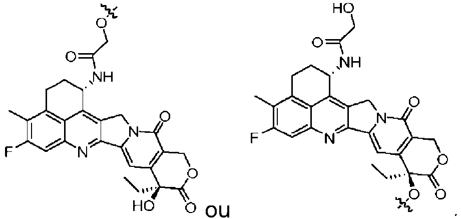

5. Conjugué anticorps-médicament, sel pharmaceutiquement acceptable de celui-ci, solvate de celui-ci ou solvate du sel pharmaceutiquement acceptable de celui-ci selon l'une quelconque des revendications 1 à 4, dans lequel le conjugué anticorps-médicament est l'un quelconque des schémas suivants :
schéma I :
Ab est l'anticorps TROP2 ou le variant de l'anticorps TROP2 ; D est 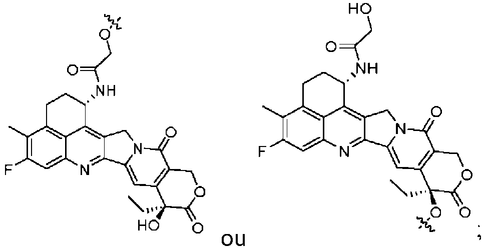
L₁ est indépendamment un ou plus d'un parmi le résidu de phénylalanine, le résidu d'alanine, le résidu de glycine, le résidu d'isoleucine, le résidu de leucine, le résidu de proline et le résidu de valine ;
schéma Il :
Ab est l'anticorps TROP2 ou le variant de l'anticorps TROP2 ;
D est 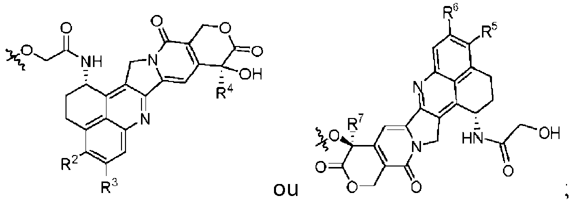 dans lequel R² et R⁵ sont chacun indépendamment un alkyle en C₁-C₆ ; R³ et R⁶ sont chacun indépendamment un halogène ; D est de préférence 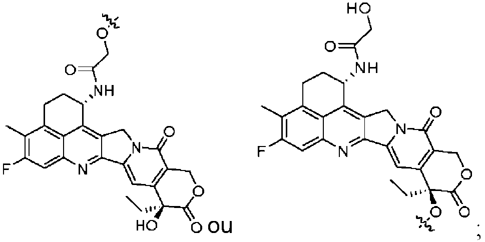
R¹ est un alkyle en C₁-C₆ substitué par un ou plus d'un -NR¹⁻¹R¹⁻², un alkyle en C₁-C₆ substitué par un ou plus d'un R¹⁻³S(O)₂-, ou un alkyle en C₁-C₆ ;
L₁ est indépendamment un ou plus d'un parmi le résidu de phénylalanine, le résidu d'alanine, le résidu de glycine et le résidu de valine ;
schéma III:
Ab est l'anticorps TROP2 ;
D est 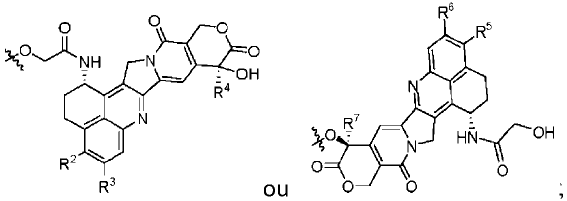 dans lequel R² et R⁵ sont chacun indépendamment un alkyle en C₁-C₆ ; R³ et R⁶ sont chacun indépendamment un halogène ; D est de préférence 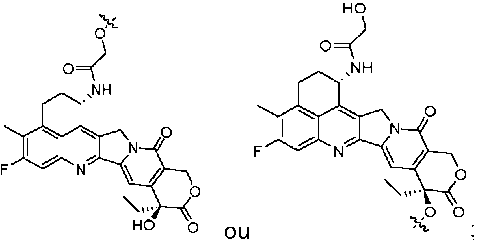
R¹ est un alkyle en C₁-C₆ substitué par un ou plus d'un -NR¹⁻¹R¹⁻², un alkyle en C₁-C₆ substitué par un ou plus d'un R¹⁻³S(O)₂-, ou un alkyle en C₁-C₆ ;
L₁ est indépendamment le résidu de valine et/ou le résidu d'alanine ;
schéma IV :
Ab est l'anticorps TROP2 ;
D est 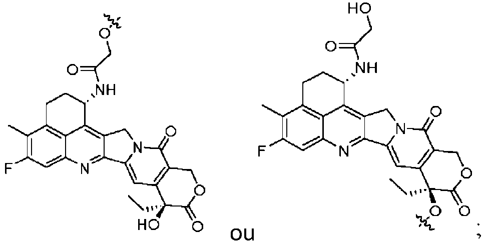
R¹ est un alkyle en C₁-C₆ substitué par un ou plus d'un -NR¹⁻¹R¹⁻², un alkyle en C₁-C₆ substitué par un ou plus d'un R¹⁻³S(O)₂-, ou un alkyle en C₁-C₆ ;
L₁ est indépendamment le résidu de valine et/ou le résidu d'alanine ;
schéma V :
Ab est l'anticorps TROP2 ;
D est 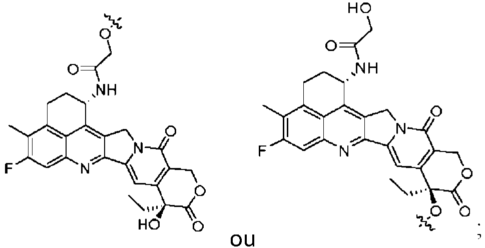
R¹ est un alkyle en C₁-C₆ substitué par un ou plus d'un -NR¹⁻¹R¹⁻², un alkyle en C₁-C₆ substitué par un ou plus d'un R¹⁻³S(O)₂-, ou un alkyle en C₁-C₆ ;
L₁ est indépendamment le résidu de valine et/ou le résidu d'alanine.

6. Conjugué anticorps-médicament, sel pharmaceutiquement acceptable de celui-ci, solvate de celui-ci ou solvate du sel pharmaceutiquement acceptable de celui-ci selon la revendication 1, dans lequel le conjugué anticorps-médicament est l'un quelconque des composés suivants : 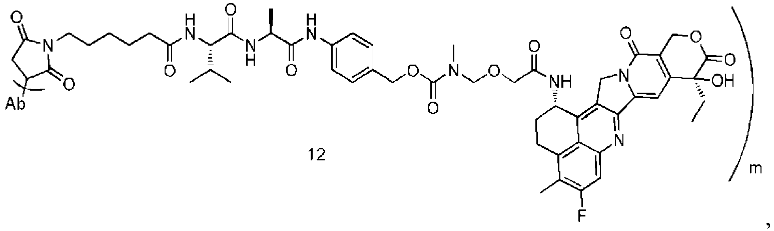 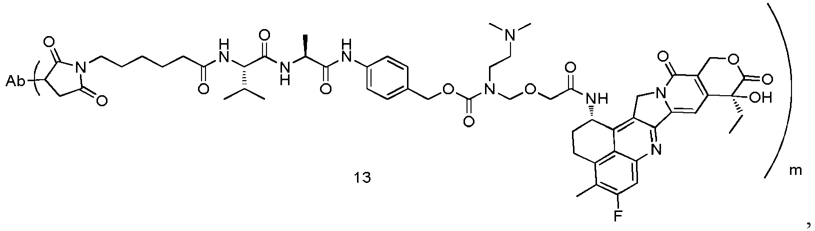 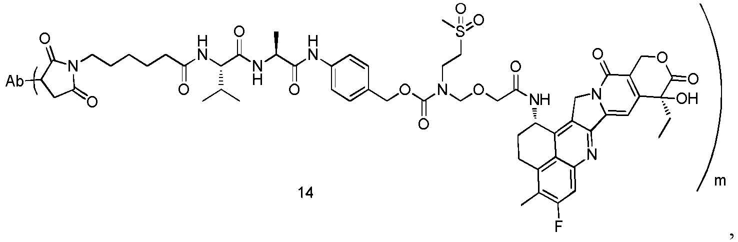 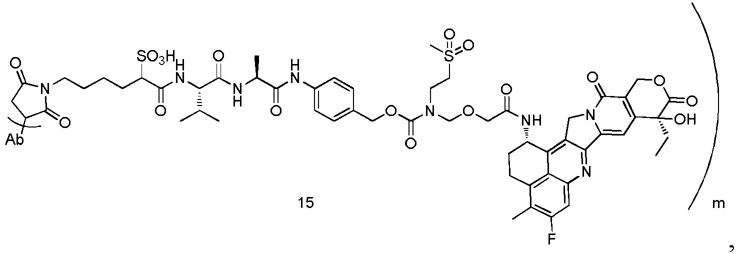 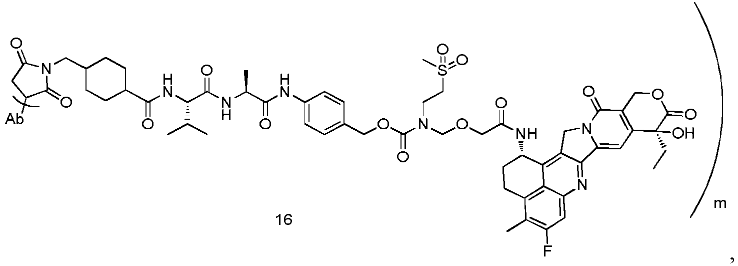 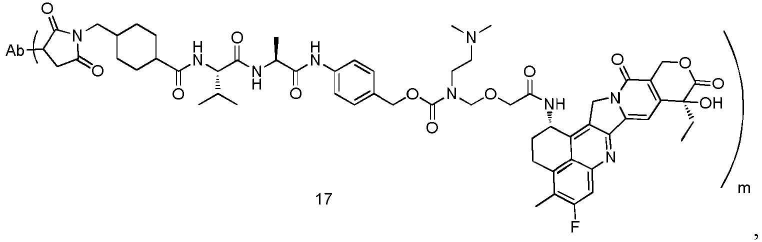 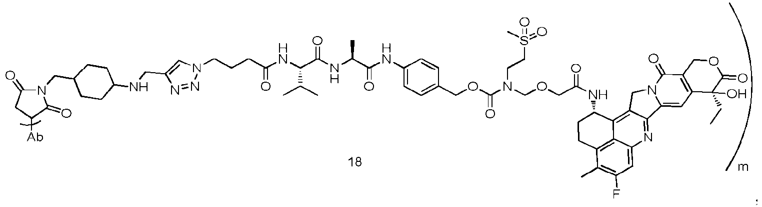 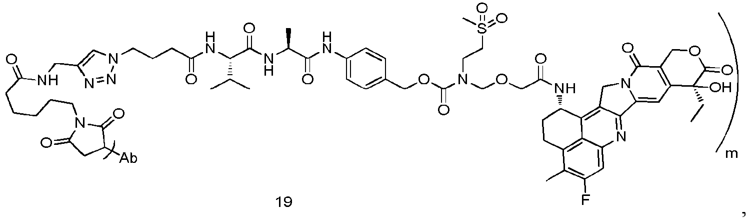 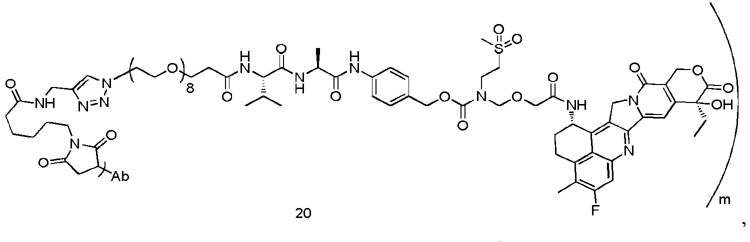 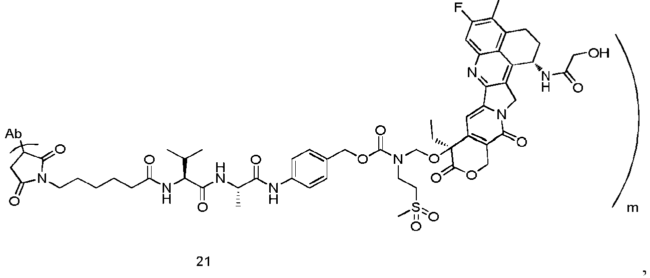 ou 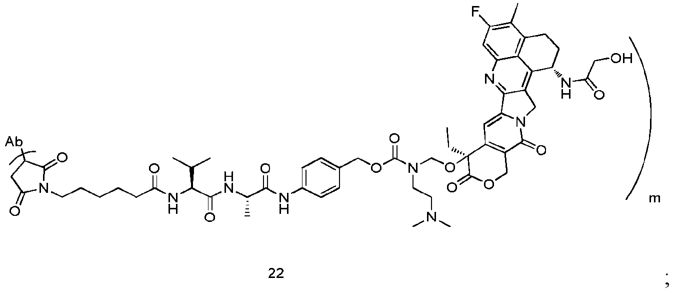 dans lequel Ab est l'anticorps TROP2 ou le variant de l'anticorps TROP2, et m vaut de 3,8 à 4,2, de préférence 3,88, 3,90, 3,96, 3,97, 3,98, 4,00, 4,03, 4,05, 4,10, 4,12 ou 4,13 ; la séquence d'acides aminés de la chaîne légère dans le variant de l'anticorps TROP2 est de préférence montrée dans SEQ ID NO : 1, et la séquence d'acides aminés de la chaîne lourde est de préférence montrée dans SEQ ID NO : 2.

7. Conjugué anticorps-médicament, son sel pharmaceutiquement acceptable, son solvate, ou solvate de son sel pharmaceutiquement acceptable selon la revendication 1, dans lequel le conjugué anticorps-médicament est l'un quelconque des schémas suivants :
schéma A : 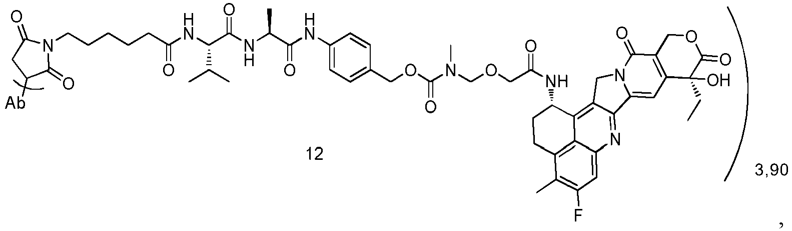 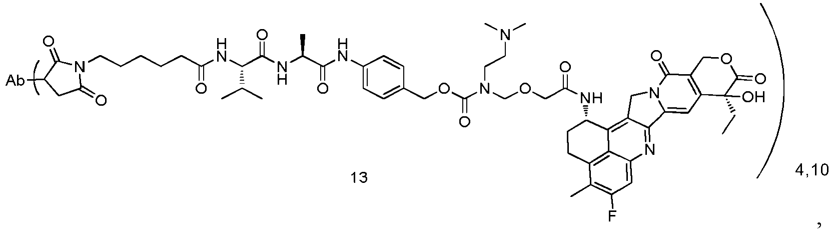 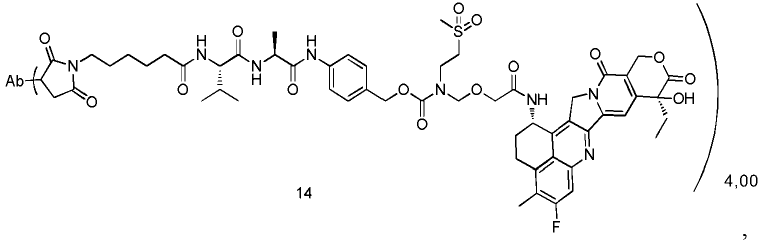 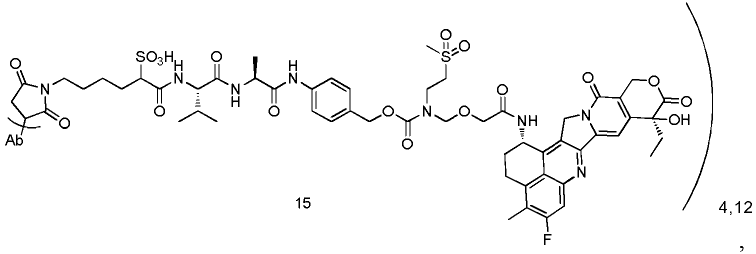 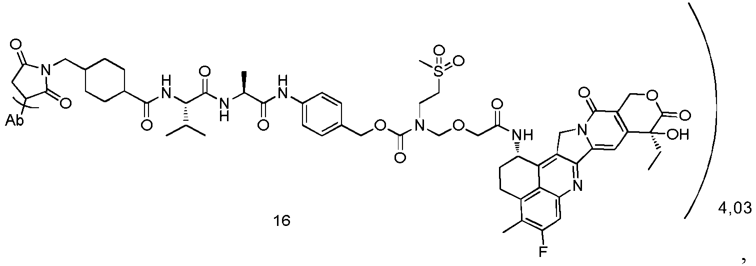 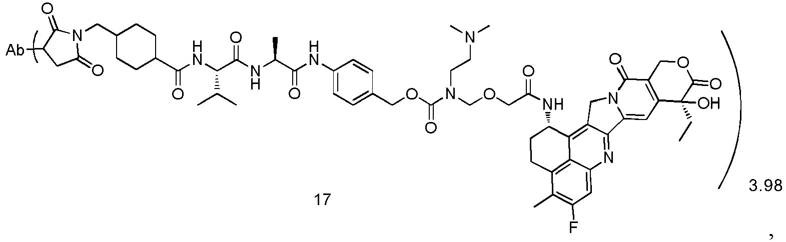 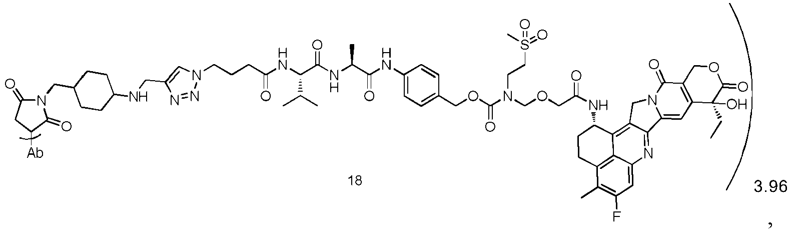 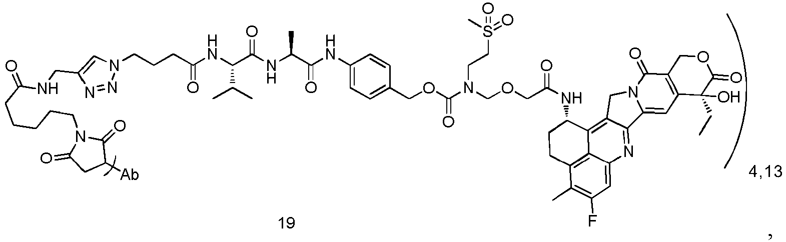 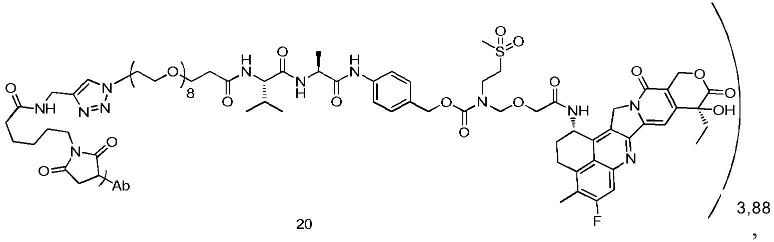 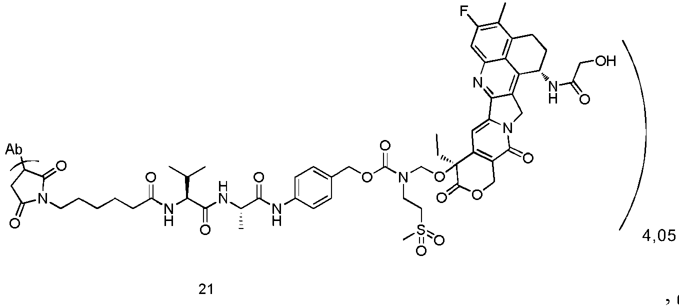 ou 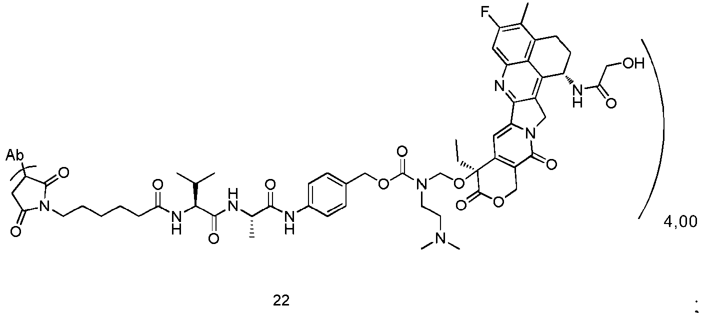
dans lequel Ab est l'anticorps TROP2 ou le variant de l'anticorps TROP2 ; la séquence d'acides aminés de la chaîne légère dans le variant de l'anticorps TROP2 est montrée dans SEQ ID NO : 1, et la séquence d'acides aminés de la chaîne lourde est montrée dans SEQ ID NO : 2 ;
schéma B : 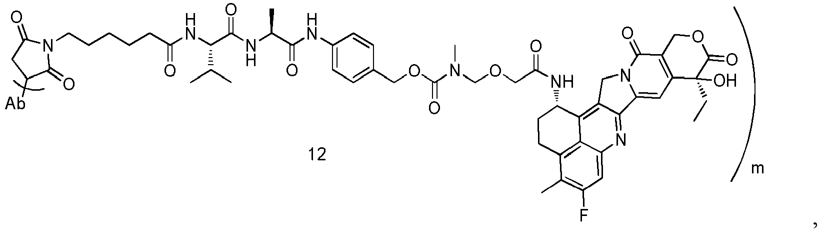 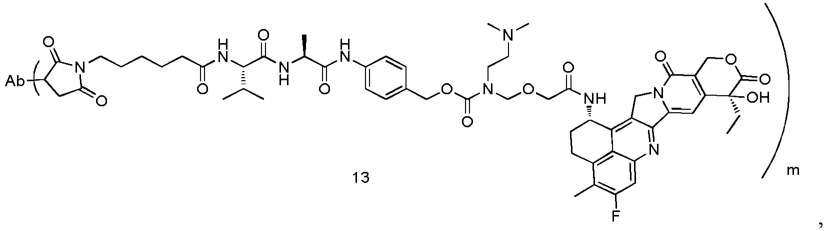 ou 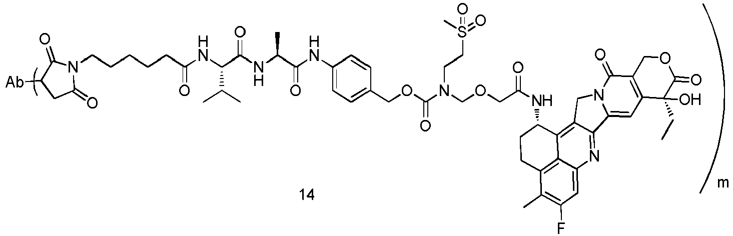
dans lequel Ab est l'anticorps TROP2 ou le variant de l'anticorps TROP2 ; m vaut 3,90, 4,00 ou 4,10 ; la séquence d'acides aminés de la chaîne légère dans le variant de l'anticorps TROP2 est montrée dans SEQ ID NO : 1, et la séquence d'acides aminés de la chaîne lourde est montrée dans SEQ ID NO : 2 ;
schéma C : 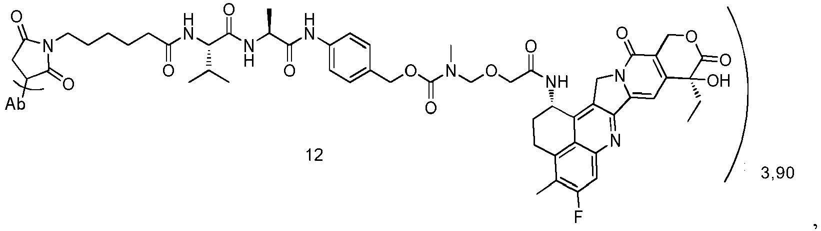 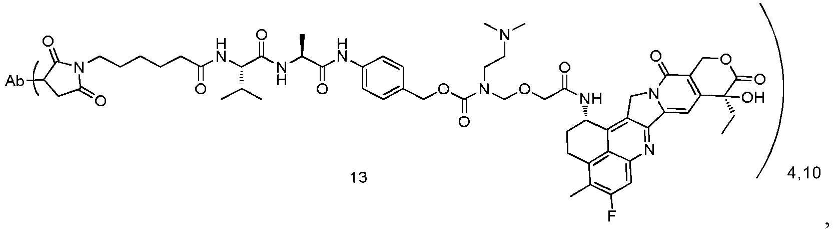 ou 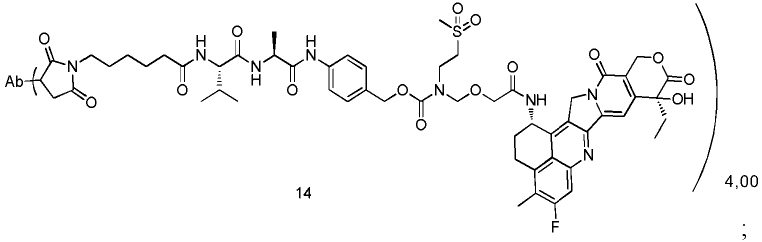
dans lequel Ab est l'anticorps TROP2 ou le variant de l'anticorps TROP2 ;
schéma D : 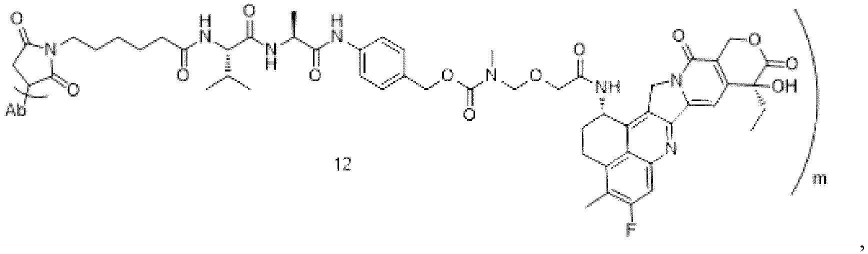
Ab est l'anticorps TROP2, et m vaut 3,90 ; 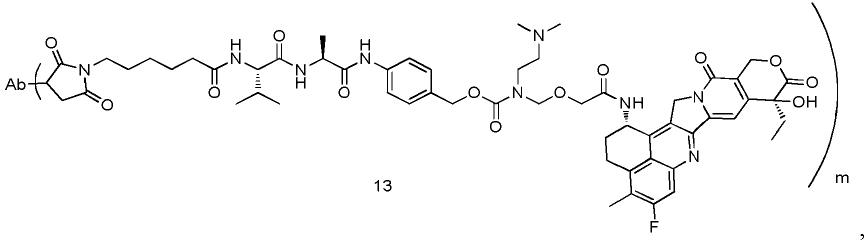
Ab est l'anticorps TROP2, et m vaut 4,10 ; 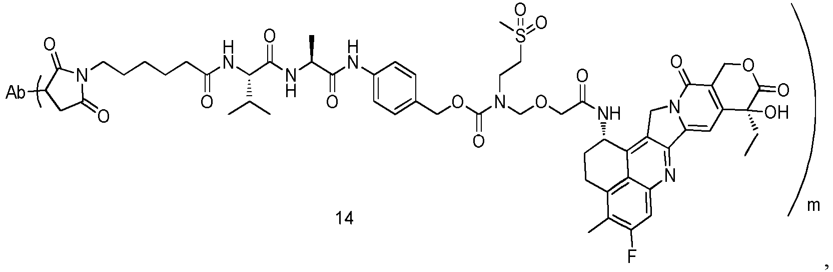
Ab est l'anticorps TROP2, et m vaut 4,00 ; 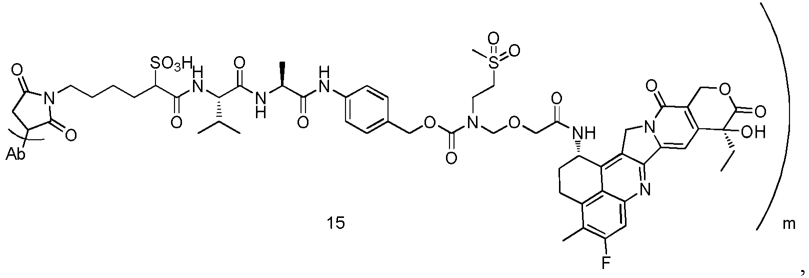
Ab est l'anticorps TROP2, et m vaut 4,12 ; 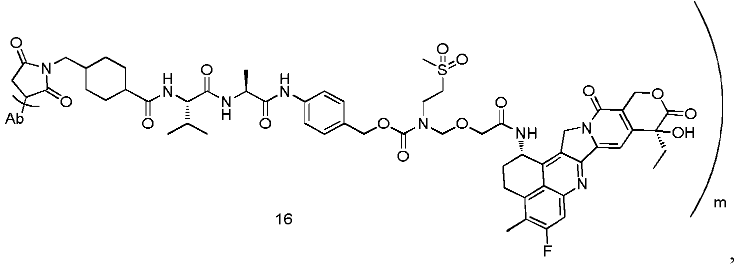
Ab est l'anticorps TROP2, et m vaut 4,03 ; 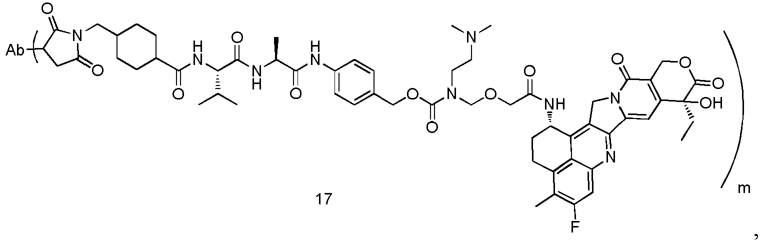
Ab est l'anticorps TROP2, et m vaut 3,98 ; 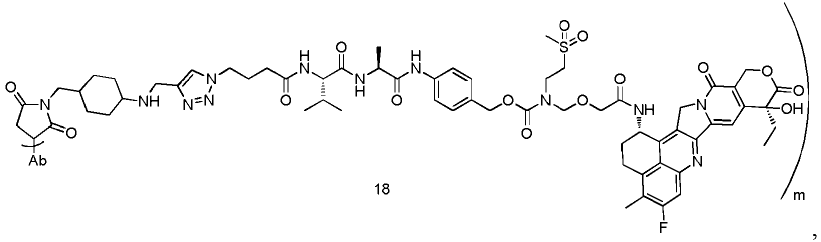
Ab est l'anticorps TROP2, et m vaut 3,96 ; 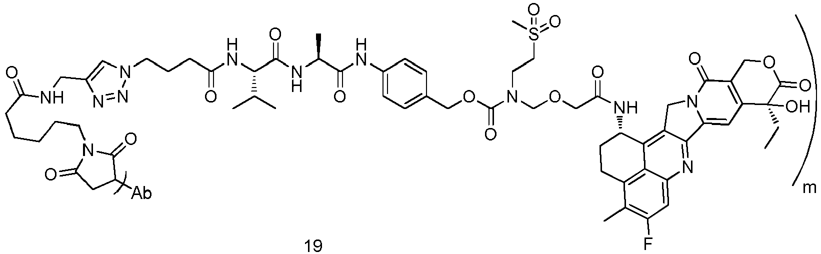
Ab est l'anticorps TROP2, et m vaut 4,13 ; 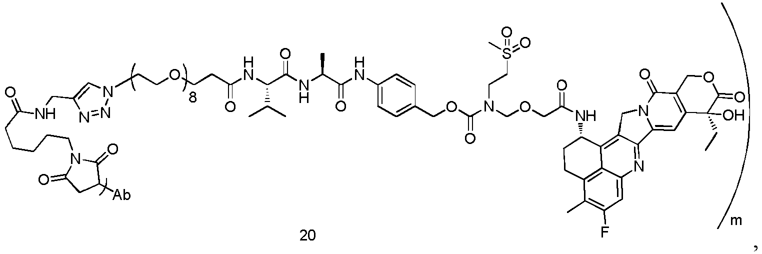
Ab est l'anticorps TROP2, et m vaut 3,88 ; 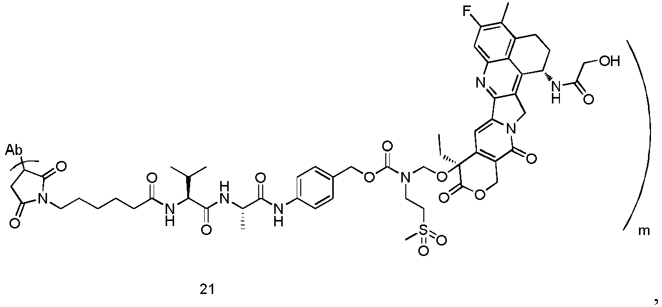
Ab est l'anticorps TROP2, et m vaut 4,05 ; 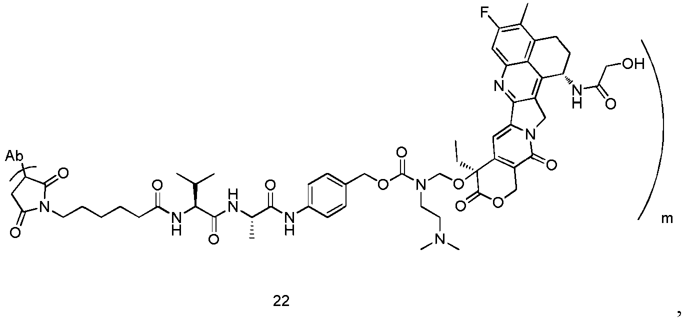
Ab est l'anticorps TROP2, et m vaut 4,00.

8. Procédé de préparation du conjugué anticorps-médicament selon l'une quelconque des revendications 1 à 7, comprenant les étapes suivantes : la réalisation d'une réaction de couplage entre un composé de formule II et Ab-hydrogène comme montré ci-dessous ;

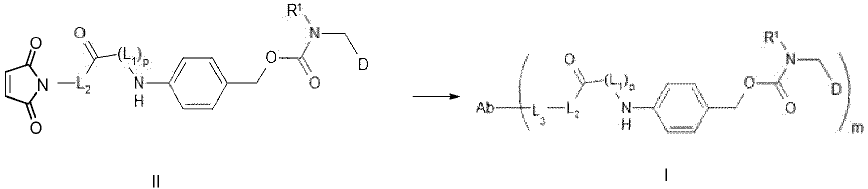

9. Composition pharmaceutique, comprenant une substance X et un excipient pharmaceutiquement acceptable, dans laquelle la substance X est le conjugué anticorps-médicament, le sel pharmaceutiquement acceptable de celui-ci, le solvate de celui-ci ou le solvate du sel pharmaceutiquement acceptable de celui-ci selon l'une quelconque des revendications 1 à 7, la quantité de la substance X est de préférence une quantité thérapeutiquement efficace.

10. Substance X pour utilisation dans l'inhibition de la protéine TROP2, dans laquelle la substance X est le conjugué anticorps-médicament, le sel pharmaceutiquement acceptable de celui-ci, le solvate de celui-ci, ou le solvate du sel pharmaceutiquement acceptable de celui-ci selon l'une quelconque des revendications 1 à 7.

11. Substance X pour utilisation dans le traitement et/ou la prévention d'une tumeur, dans laquelle la substance X est le conjugué anticorps-médicament, le sel pharmaceutiquement acceptable de celui-ci, le solvate de celui-ci, ou le solvate du sel pharmaceutiquement acceptable de celui-ci selon l'une quelconque des revendications 1 à 7.

12. Substance X pour utilisation selon la revendication 11, dans laquelle la tumeur est une tumeur positive pour TROP2 ; et la tumeur positive pour TROP2 est un ou plusieurs parmi le cancer gastrique, le cancer du sein triple négatif et le cancer du pancréas humain positifs pour TROP2.

13. Substance X pour utilisation selon la revendication 12, dans laquelle les cellules de cancer gastrique sont des cellules *NCI-N87 ;*
et/ou, les cellules de cancer du sein triple négatif sont des cellules MDA-MB-468 ;
et/ou, les cellules de cancer du pancréas sont de préférence des cellules BXPC3.
